# EUROPÄISCHE PATENTSCHRIFT

(11) **EP 1 677 911 B1**
(45) Veröffentlichungstag und Bekanntmachung des Hinweises auf die Patenterteilung: **16.06.2010**
(21) Anmeldenummer: 04790777.9
(22) Anmeldetag: 22.10.2004
(51) Int. Cl.: B01J 31/02, C07C 45/50

(54) **STABILISIERUNG VON HYDROFORMYLIERUNGSKATALYSATOREN AUF BASIS VON PHOSPHORAMIDITLIGANDEN**
STABILIZATION OF HYDROFORMYLATION CATALYSTS BASED ON PHOSPHORAMIDE LIGANDS
STABILISATION DE CATALYSEURS D'HYDROFORMYLATION A BASE DE LIGANDS PHOSPHORAMIDITE

(30) Priorität: 23.10.2003 DE 10349343
(43) Veröffentlichungstag der Anmeldung: 12.07.2006
(73) Patentinhaber: BASF SE, 67056 Ludwigshafen (DE)
(72) Erfinder: PAPP, Rainer, 67346 Speyer (DE); AHLERS, Wolfgang, 67549 Worms (DE); MACKEWITZ, Thomas, 68219 Mannheim (DE); PACIELLO, Rocco, 67098 Bad Dürkheim (DE); VOLLAND, Martin, 69115 Heidelberg (DE)
(86) Internationale Anmeldenummer: PCT/EP2004/011986
(87) Internationale Veröffentlichungsnummer: WO 2005/039762

(56) Entgegenhaltungen:
- WO-A-03/018192
- TRZECIAK, ANNA M. ET AL: "Novel rhodium complexes with N-pyrrolylphosphines: attractive precursors of hydroformylation catalysts" JOURNAL OF THE CHEMICAL SOCIETY, DALTON TRANSACTIONS: INORGANIC CHEMISTRY , (11), 1831-1837 CODEN: JCDTBI; ISSN: 0300-9246, 1997, XP002311822

## Beschreibung

Die vorliegende Erfindung betrifft ein Verfahren zur Hydroformylierung ethylenisch ungesättigter Verbindungen durch Umsetzung mit Kohlenmonoxid und Wasserstoff in Gegenwart eines katalytisch aktiven Fluids, das eine gelöste Metallkomplexverbindung eines Metalls der VIII. Nebengruppe des Periodensystems der Elemente mit wenigstens einer Phosphoramiditverbindung als Liganden enthält, wobei man das Fluid mit einer Base in Kontakt bringt.

Die Hydroformylierung oder Oxo-Synthese ist ein wichtiges großtechnisches Verfahren und dient der Herstellung von Aldehyden aus Olefinen, Kohlenmonoxid und Wasserstoff. Diese Aldehyde können gegebenenfalls im gleichen Arbeitsgang mit Wasserstoff zu den entsprechenden Oxo-Alkoholen hydriert werden. Die Reaktion selbst ist stark exotherm und läuft im Allgemeinen unter erhöhtem Druck und bei erhöhten Temperaturen in Gegenwart von Katalysatoren ab. Als Katalysatoren werden Co-, Rh-, Ir-, Ru-, Pd- oder Pt-Verbindungen bzw. -komplexe eingesetzt, die zur Aktivitäts- und/oder Selektivitätsbeeinflussung mit N- oder P-haltigen Liganden modifiziert sein können. Bei der Hydroformylierungsreaktion von Olefinen mit mehr als zwei C-Atomen kann es auf Grund der möglichen CO-Anlagerung an verschiedene C-Atome einer Doppelbindung zur Bildung von Gemischen isomerer Aldehyde kommen. Zusätzlich kann es beim Einsatz von Olefinen mit mindestens vier Kohlenstoffatomen auch zu einer Doppelbindungsisomerisierung kommen, d. h. zu einer Verschiebung interner Doppelbindungen auf eine terminale Position und umgekehrt.

Auf Grund der wesentlich größeren technischen Bedeutung der α-Aldehyde wird eine Optimierung der Hydroformylierungsverfahren zur Erzielung möglichst hoher Umsätze bei gleichzeitig möglichst geringer Neigung zur Bildung von Olefinen mit nicht αständigen Doppelbindungen angestrebt. Zudem besteht ein Bedarf an Hydroformylierungsverfahren, die auch ausgehend von internen linearen Olefinen in guten Ausbeuten zu α-ständigen und insbesondere n-ständigen Aldehyden führen. Hierbei muss der eingesetzte Katalysator sowohl die Einstellung eines Gleichgewichts zwischen internen und terminalen Doppelbindungsisomeren als auch möglichst selektiv die Hydroformylierung der terminalen Olefine ermöglichen.

Die WO 00/56451 beschreibt Hydroformylierungskatalysatoren auf Basis von Phosphinamiditliganden, worin das Phosphoratom gemeinsam mit einem Sauerstoffatom, an das es gebunden ist, für einen 5- bis 8-gliedrigen Heterocyclus steht.

Die WO 02/083695 beschreibt Pnicogenchelatverbindungen, bei denen an jedes der Pnicogenatome wenigstens eine Pyrrolgruppe über das pyrrolische Stickstoffatom gebunden ist. Diese Pnicogenchelatverbindungen eignen sich als Liganden für Hydroformylierungskatalysatoren.

Die WO 03/018192 beschreibt u. a. Pyrrolphosphorverbindungen, bei denen wenigstens eine substituierte und/oder in ein anelliertes Ringsystem integrierte Pyrrolgruppe über ihr pyrrolisches Stickstoffatom kovalent mit dem Phosphoratom verknüpft ist, die sich bei einem Einsatz als Liganden in Hydroformylierungskatalysatoren durch eine sehr gute Stabilität auszeichnen,

A. M. Trzeciak et al. beschreiben in J. Chem. Soc., Dalton Trans., 1997, S. 1831-1837, Rhodiumkomplexe mit N-Pyrrolylphosphinen und deren Verwendung in der homogen katalysierten Hydroformylierung.

Die unveröffentlichte deutsche Patentanmeldung 102 43 138.8 beschreibt Pnicogenverbindungen, die zwei Pnicogenatome aufweisen, wobei an beide Pnicogenatome Pyrrolgruppen über ein pyrrolisches Stickstoffatom gebunden sein können und wobei beide Pnicogenatome über eine Methylengruppe an eine verbrückende Gruppe gebunden sind. Diese Pnicogenverbindungen eignen sich als Liganden für Hydroformylierungskatalysatoren.

Die zuvor genannten Katalysatoren zeichnen sich durch eine hohe Regioselektivität zugunsten terminaler Produktaldehyde sowohl bei der Hydroformylierung von α-Olefinen als auch bei der Hydroformylierung von internen linearen Olefinen aus. Sie verfügen außerdem über eine gute Stabilität unter den Hydroformylierungsbedingungen, was insbesondere für Hydroformylierungskatalysatoren auf Basis von Liganden zutrifft, die eine oder mehrere 3-Alkylindolgruppe(n) an das Phosphoratom gebunden aufweisen. Dennoch ist im Hinblick auf die für einen großtechnischen Einsatz erforderlichen langen Katalysatorstandzeiten eine zusätzliche Stabilisierung erstrebenswert.

Die DE-A-102 06 697 beschreibt ein Hydroformylierungsverfahren, das eine Abtrennung der Wertprodukte und eine Rückführung des Katalysators mit einem möglichst geringen Aktivitätsverlust ermöglicht, Dazu wird ein Hydroformylierungskatalysator auf Basis eines zweizähnigen Phosphinliganden eingesetzt, der durch wenigstens einen einzähnigen Phosphinliganden stabilisiert wird.

Die EP-A-0 149 894 und die US 4,567,306 beschreiben ein kontinuierliches Hydroformylierungsverfahren unter Einsatz eines Hydroformylierungskatalysators, der als Liganden ein cyclisches Phosphit mit einem Phosphoratom als Brückenkopf aufweist. An das Phosphoratom sind drei Sauerstoffatome direkt gebunden, von denen wenigstens zwei gemeinsam mit dem Phosphoratom einen Ring bilden. Geeignete Liganden weisen beispielsweise ein Phosphabicyclo[2.2.2]octan- oder ein Phosphaadamantyl-Gerüst auf. Das Verfahren umfasst die Stabilisierung der Liganden mit einem tertiären Amin.

Die EP-A-0 155 508 und die US 4,599,206 beschreiben Hydroformylierungsverfahren unter Einsatz von Katalysatorkomplexen auf Basis von Diorganophosphit-Liganden, wobei aus der Reaktionszone ein flüssiger Austrag entnommen, mit einem schwach basischen Anionenaustauscher in Kontakt gebracht und anschließend in die Reaktionszone zurückgeführt werden kann. Die US 4,717,775 beschreibt eine Variante des in den zuvor genannten Dokumenten offenbarten Hydroformylierungsverfahrens, nach der die Hydroformylierung in Gegenwart von freiem Diorganophosphit-Ligand erfolgt. Die US 4,774,361 beschreibt ein Verfahren zur Vermeidung oder Minimierung der Ausfällung von Rhodium aus Rhodiumphosphit-Komplexkatalysatoren in einem Hydroformylierungsverfahren mit Flüssigkeitskreislauf, wobei der Aldehyd aus dem Reaktionsaustrag abdestilliert und diese Destillation in Gegenwart eines organischen Polymers, das mindestens drei polare Amidfunktionen enthält, beispielsweise Polyvinylpyrrolidon oder Copolymeren aus Vinylpyrrolidon und Vinylacetat, durchgeführt wird. Die EP-A-0 276 231 hat einen der US 4,774,361 vergleichbaren Offenbarungsgehalt.

Die EP-A-214 622 beschreibt ein Hydroformylierungsverfahren unter Einsatz eines Katalysators auf Basis eines Polyphosphitliganden, der zwei bis sechs Phosphitgruppen aufweist. Es wird beschrieben, im Bedarfsfall zur Stabilisierung der Polyphosphitliganden den flüssigen Austrag aus der Reaktionszone vor oder nach der Abtrennung der Produktaldehyde mit einem schwach basischen Anionenaustauscherharz in Kontakt zu bringen und erst danach den Strom in den Hydroformylierungsreaktor zurückzuführen.

Die WO 97/20794 und die US 5,741,942 beschreiben Verfahren zur Abtrennung saurer Phosphorverbindungen aus einer Reaktionsflüssigkeit, die einen Metallorganophosphitkomplex-Katalysator und gegebenenfalls freien Organophosphitliganden enthält, durch Behandlung der Reaktionsflüssigkeit mit einer wässrigen Pufferlösung, die geeignet ist, wenigstens einen Teil der sauren Phosphorverbindungen zu entfernen. Zusätzlich kann eine organische Stickstoffverbindung eingesetzt werden, die befähigt ist, die sauren Phosphorverbindungen abzufangen, wobei das Reaktionsprodukt aus Stickstoff- und Phosphorverbindung ebenfalls durch Behandlung mit der wässrigen Pufferlösung neutralisiert und entfernt wird. Weiter beschrieben sind Verfahren zur Stabilisierung von Organophosphitliganden gegen hydrolytischen Abbau, zur Stabilisierung von Metallorganophosphitkomplex-Katalysatoren gegen Deaktivierung sowie zur Umsetzung von einem oder mehreren Reaktanten in Gegenwart von Metallorganophosphitkomplex-Katalysatoren, wobei jeweils eine Behandlung mit einer wässrigen Pufferlösung erfolgt. Die US 5,741,944 beschreibt ein analoges Verfahren zur Abtrennung saurer Phosphorverbindungen aus Hydroformylierungsreaktionsausträgen. Die US 5,874,640 beschreibt ein analoges Verfahren zur Entfernung saurer Phosphorverbindungen aus Reaktionsausträgen, die Metallkomplexkatalysatoren mit ganz allgemein Organophosphorliganden enthalten. Ein Einsatz des Verfahrens für Reaktionslösungen, die Phosphoramiditliganden enthalten, ist nicht beschrieben.

Die WO 97/20795, US 5,741,943 und US 5,741,945 beschreiben Verfahren, die die Umsetzung eines oder mehrerer Reaktanten in Anwesenheit eines Metall-Organopolyphosphitligandkomplex-Katalysators und gegebenenfalls von freiem Organopolyphosphitligand und einem davon verschiedenen sterisch gehinderten Organophosphorliganden umfassen. Letzterer hat die Funktion eines Indikatorliganden, der eine Verarmung der Reaktionsmischung an Polyorganophosphitliganden anzeigen und gleichzeitig das Rhodium im Falle einer solcher Verarmung komplex in Lösung halten soll.

Die WO 97/20797, US 5,744,649 und US 5,786,517 beschreiben Verfahren zur Entfernung saurer Phosphorverbindungen aus Organophosphitligand-Metallkomplexkatalysatoren enthaltenden Reaktionsflüssigkeiten durch Behandlung mit Wasser. Die US 5,886,235 beschreibt ein analoges Verfahren zur Behandlung von Reaktionsflüssigkeiten, die ganz allgemein Metallkomplexkatalysatoren auf Basis von Organophosphorliganden enthalten.

Die WO 97/20798 und die US 5,731,472 beschreiben Verfahren zur Stabilisierung von Metall-Organopolyphosphitligand-Komplexkatalysatoren gegen Deaktivierung, bei denen man die katalysierte Reaktion in Gegenwart von wenigstens einer freien, heterocyclischen Stickstoffverbindung, die ausgewählt ist unter Diazolen, Triazolen, Diazinen oder Triazinen, durchführt.

Die WO 97/20799, US 5,763,671 und US 5,789,625 betreffen Verfahren zur Entfernung von sauren Phosphorverbindungen aus Organophosphitligand-Metallkomplexkatalysatoren enthaltenden Reaktionsflüssigkeiten durch deren Extraktion mit Wasser und Behandlung des Wassers mit einer säureentfernenden Substanz. Die US 5,917,095 betrifft ein analoges Verfahren, wobei allgemein Metallkomplexkatalysatoren auf Basis von Organophosphorliganden eingesetzt werden.

Die WO 97/20800, US 5,763,670 und US 5,767,321 betreffen Verfahren, in denen Organopolyphosphitligand-Komplexkatalysatoren in Gegenwart einer ausreichenden Menge an freiem Organopolyphosphitligand eingesetzt werden, um den hydrolytischen Abbau des Liganden bzw. die Deaktivierung des Katalysators zu verhindern oder zu vermindern.

Die WO 97/20796, US 5,763,677 und US 5,763,680 beschreiben Verfahren zur Abtrennung von einer oder mehreren sauren Phosphorverbindungen aus Reaktionsflüssigkeiten, die Organophosphitligand-Metallkomplexkatalysatoren enthalten, durch Extraktion mit Wasser und Behandeln des Wassers mit einem Ionenaustauscher und gegebenenfalls einem Amin. Die US 5,892,119 beschreibt ein analoges Verfahren für Reaktionsflüssigkeiten, die ganz allgemein Metallkomplexkatalysatoren auf Basis von Organophosphorliganden enthalten. Die Behandlung von Reaktionsflüssigkeiten, die Katalysatoren auf Basis von Phosphoramiditliganden enthalten, ist nicht beschrieben.

Der vorliegenden Erfindung liegt die Aufgabe zu Grunde, ein verbessertes Verfahren zur Hydroformylierung von Verbindungen, die wenigstens eine ethylenisch ungesättigte Doppelbindung enthalten, zur Verfügung zu stellen. Dabei sollen Hydroformylierungskatalysatoren zum Einsatz kommen, die die Hydroformylierung längerkettiger, endständiger oder interner Olefine oder von technischen Gemischen aus Olefinen mit endständiger und interner Doppelbindung, z. B. Gemischen aus 1-Buten und 2-Buten, zu Aldehyden mit hoher Linearität (n-Selektivität) bei gutem Umsatz ermöglichen. Eine weitere Forderung, die an die Hydroformylierungskatalysatoren gestellt wird, ist eine gute Stabilität unter den Hydroformylierungsbedingungen und somit eine lange Kataly-Katalysatorstandzeit, da Katalysator- bzw. Ligandverluste sich in besonderem Maße negativ auf die Wirtschaftlichkeit eines Hydroformylierungsverfahrens auswirken.

Überraschenderweise wurde nun gefunden, dass diese Aufgabe durch ein Verfahren zur Hydroformylierung gelöst wird, bei dem zur Katalyse eine im Reaktionsmedium gelöste Metallkomplexverbindung eines Metalls der VIII. Nebengruppe des Periodensystems der Elemente mit wenigstens einer Phosphoramiditverbindung der Formeln I und II, wie im Anspruch 1 definiert, als Liganden eingesetzt wird und wobei die Lösung mit einer Base, wie ebenfalls in Anspruch 1 definiert, in Kontakt gebracht wird.

Gegenstand der Erfindung ist daher ein Verfahren zur Hydroformylierung von Verbindungen, die wenigstens eine ethylenisch ungesättigte Doppelbindung enthalten, durch Umsetzung mit Kohlenmonoxid und Wasserstoff in wenigstens einer Reaktionszone in Gegenwart eines katalytisch aktiven Fluids, das eine gelöste Metallkomplexverbindung eines Metalls der VIII. Nebengruppe des Periodensystems der Elemente mit wenigstens einer Phosphoramiditverbindung als Liganden enthält, bei dem die Phosphoramiditverbindung ausgewählt ist unter Verbindungen der allgemeinen Formeln I oder II 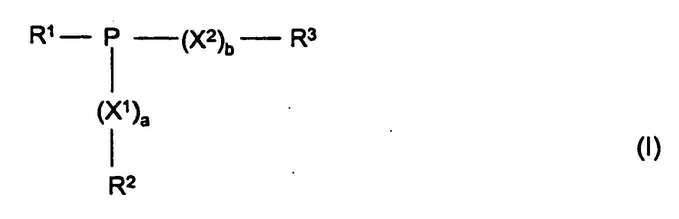 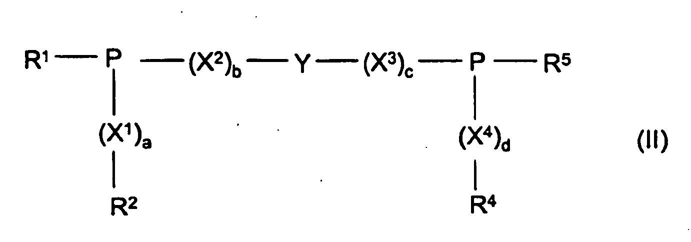 worin
- R¹ und R⁵: unabhängig voneinander für über das Stickstoffatom an das Phosphoratom gebundene Pyrrolgruppen stehen,
- R², R³ und R⁴: unabhängig voneinander für Alkyl, Cycloalkyl, Heterocycloalkyl, Aryl oder Hetaryl stehen,
oder R¹ gemeinsam mit R² und/oder R⁴ gemeinsam mit R⁵ eine zweibindige Gruppe bilden, die mindestens eine über das pyrrolische Stickstoffatom an das Phosphoratom gebundene Pyrrolgruppe enthält,
- Y: für eine zweiwertige verbrückende Gruppe mit 2 bis 20 Brückenatomen zwischen den flankierenden Bindungen steht,
- X¹, X², X³ und X⁴: unabhängig voneinander ausgewählt sind unter O, S, SiR^{α}R^{β} und NR^{γ}, worin R^{α}, R^{β} und R^{γ} unabhängig voneinander für Wasserstoff, Alkyl, Cycloalkyl, Heterocycloalkyl, Aryl oder Hetaryl stehen, und
a, b, c und d unabhängig voneinander für 0 oder 1 stehen, wobei man das Fluid mit einer Base in Kontakt bringt und die Base ausgewählt ist unter in dem katalytisch aktiven Fluid löslichen Basen, ausgewählt unter Trialkylaminen, Dialkylarylaminen, Alkyldiarylaminen, Triarylaminen und stickstoffhaltigen Heterocyclen, an eine Festphase immobilisierten Basen und Kombinationen davon.

Unter einer "Phosphoramiditverbindung" wird im Rahmen der vorliegenden Erfindung eine phosphoratomhaltige Verbindung (der Formel I oder II) verstanden, die wenigstens ein Phosphoratom aufweist, an das eine, zwei oder drei Gruppen kovalent über ein Stickstoffatom, d.h. unter Ausbildung einer P-N-Bindung, gebunden sind. Phosphoramiditverbindungen (der Formel I oder II), insbesondere solche, bei denen eine oder mehrere substituierte Pyrrolgruppen über ihr Stickstoffatom an das Phosphoratom gebunden sind, und die darauf basierenden Hydroformylierungskatalysatoren zeichnen sich bereits durch eine gute Stabilität aus. Die Erfinder der vorliegenden Erfindung haben nun gefunden, dass sich Katalysatoren auf Basis von Phosphoramiditliganden gegenüber einem Abbau der Liganden bzw. einer Deaktivierung der Katalysatoren unter den Hydroformylierungsbedingungen zusätzlich stabilisieren lassen, indem man das katalytisch aktive Fluid mit einer, wie zuvor definierten, Base in Kontakt bringt. Dies ist insofern überraschend, als diese Liganden bereits mehr oder minder basische Stickstoffatom-haltige Gruppen enthalten. Vorteilhafterweise gelingt die Stabilisierung der Hydroformylierungskatalysatoren auf Basis von Phosphoramidit-liganden durch Inkontaktbringen mit einer Base auch in Abwesenheit von Synthesegas. Gegenstand der vorliegenden Erfindung ist somit auch ein Hydroformylierungsverfahren umfassend die Aufarbeitung des Austrags aus der Reaktionszone und die Rückführung des katalytisch aktiven Fluids, wobei wenigstens einer dieser Schritte in Abwesenheit von Kohtenmonoxid und Wasserstoff durchgeführt wird.

Unter "Inkontaktbringen" wird sowohl die Bildung einer einphasigen Mischung als auch ein Inkontaktbringen durch eine Phasengrenzfläche, z. B. flüssig/flüssig oder flüssig/fest, verstanden. Das Inkontaktbringen kann über die gesamte Verfahrensdauer der Hydroformylierung (einschließlich der Aufarbeitung und Rückführung des katalytisch aktiven Fluids), einen Teil davon oder periodisch erfolgen.

Das katalytisch aktive Fluid enthält wenigstens eine gelöste Metallkomplexverbindung eines Metalls der VIII. Nebengruppe des Periodensystems der Elemente mit wenigstens einer Phosphoramiditverbindung als Liganden. Die Metallkomplexverbindung liegt somit im Allgemeinen als homogen einphasige Lösung in einem geeigneten Lösungsmittel vor. Diese Lösung kann zusätzlich Phosphoramiditverbindungen als freien Liganden enthalten. Als Lösungsmittel werden vorzugsweise die bei der Hydroformylierung der jeweiligen ethylenisch ungesättigten Verbindungen entstehenden höhersiedenden Folgereaktionsprodukte, z. B. die Produkte der Aldolkondensation, eingesetzt. Des Weiteren können, bis zu ihrer Abtrennung, auch die Hydroformylierungsprodukte als Lösungsmittel fungieren.

Ebenfalls geeignete Lösungsmittel sind Aromaten, wie Toluol und Xylole, Kohlenwasserstoffe oder Gemische von Kohlenwasserstoffen. Weitere Lösungsmittel sind Ester aliphatischer Carbonsäuren mit Alkanolen, beispielsweise Essigester oder Texanol®, Ether wie tert.-Butylmethylether und Tetrahydrofuran. Bei ausreichend hydrophilisierten Liganden können auch Alkohole, wie Methanol, Ethanol, n-Propanol, Isopropanol, n-Butanol, Isobutanol, Ketone, wie Aceton und Methylethylketon etc., eingesetzt werden. Ferner können als Lösungsmittel auch so genannte "Ionische Flüssigkeiten" verwendet werden. Hierbei handelt es sich um flüssige Salze, beispielsweise um N,N'-Dialkylimidazoliumsalze wie die N-Butyl-N'-methylimidazoliumsalze, Tetraalkylammoniumsalze wie die Tetra-n-butylammoniumsalze, N-Alkylpyridiniumsalze wie die n-Butylpyridiniumsalze, Tetraalkylphosphoniumsalze wie die Trishexyl(tetradecyl)phosphoniumsalze, z. B. die Tetrafluoroborate, Acetate, Tetrachloroaluminate, Hexafluorophosphate, Chloride und Tosylate.

Die Hydroformylierung erfolgt in wenigstens einer Reaktionszone, die einen oder mehrere, gleiche oder verschiedene Reaktoren umfassen kann. Im einfachsten Fall wird die Reaktionszone von einem einzelnen Reaktor gebildet. Sowohl die Reaktoren jeder einzelnen Zone als auch die gegebenenfalls verschiedene Stufen bildenden Reaktoren können jeweils gleiche oder verschiedene Vermischungscharakteristiken aufweisen. Die Reaktoren können gewünschtenfalls durch Einbauten ein- oder mehrfach unterteilt sein. Bilden zwei oder mehrere Reaktoren eine Zone, so können diese untereinander beliebig verschaltet sein, z. B. parallel oder in Reihe.

Geeignete druckfeste Reaktionsapparaturen für die Hydroformylierung sind dem Fachmann bekannt. Dazu zählen die allgemein üblichen Reaktoren für Gas-Flüssig-Reaktionen, wie z. B. Rohrreaktoren, Rührkessel, Gasumlaufreaktoren, Blasensäulen, etc., die gegebenenfalls durch Einbauten unterteilt sein können.

Kohlenmonoxid und Wasserstoff werden üblicherweise in Form eines Gemischs, dem so genannten Synthesegas, eingesetzt. Die Zusammensetzung des im erfindungsgemäßen Verfahren eingesetzten Synthesegases kann in weiten Bereichen variieren. Das molare Verhältnis von Kohlenmonoxid und Wasserstoff beträgt in der Regel 1:1000 bis 1000:1, vorzugsweise 1:100 bis 100:1. Werden mehrere Reaktionszonen eingesetzt, so können diese gleiche oder verschiedene Molverhältnisse von CO zu H₂ aufweisen.

Die Temperatur bei der Hydroformylierungsreaktion liegt im Allgemeinen in einem Bereich von etwa 20 bis 200 °C, vorzugsweise etwa 50 bis 190 °C, insbesondere etwa 60 bis 150 °C. Die Umsetzung wird vorzugsweise bei einem Druck in einem Bereich von etwa 1 bis 700 bar, besonders bevorzugt 3 bis 600 bar, insbesondere 5 bis 50 bar durchgeführt. Der Reaktionsdruck kann in Abhängigkeit von der Aktivität des eingesetzten Hydroformylierungskatalysators variiert werden. So erlauben die im Folgenden näher beschriebenen Hydroformylierungskatalysatoren z. T. eine Umsetzung in einem Bereich niedriger Drücke, wie etwa im Bereich von etwa 1 bis 100 bar. Werden mehrere Reaktionszonen eingesetzt, können diese bei gleichen oder bei verschiedenen Temperaturen und/oder Drücken betrieben werden.

Die Hydroformylierung kann diskontinuierlich oder kontinuierlich erfolgen, bevorzugt ist ein kontinuierliches Verfahren, bei dem man
a) in die Reaktionszone(n) die ethylenisch ungesättigte(n) Verbindung(en) sowie Kohlenmonoxid und Wasserstoff einspeist und in Gegenwart des katalytisch aktiven Fluids umsetzt,
b) aus der Reaktionszone einen Austrag entnimmt und einer Auftrennung unterzieht, wobei eine im Wesentlichen das Hydroformylierungsprodukt enthaltende Fraktion und das katalytisch aktive Fluid, das die höher als das Hydroformylierungsprodukt siedenden Nebenprodukte der Hydroformylierung und die darin gelöste Metallkomplexverbindung enthält, erhalten werden, und
c) das katalytisch aktive Fluid, gegebenenfalls nach Abtrennung wenigstens eines Teils der höher als das Hydroformylierungsprodukt siedenden Nebenprodukte, in die Reaktionszone zurückführt.

Der Austrag aus der Reaktionszone wird einer ein- oder mehrstufigen Trennoperation unterzogen, wobei zumindest ein die Hauptmenge des Hydroformylierungsprodukts enthaltender Strom und ein das katalytisch aktive Fluid enthaltender Strom erhalten werden. In Abhängigkeit von den angewandten Austrags- und Trennverfahren werden im Allgemeinen weitere Ströme erhalten, wie synthesegashaltige Abgase, aus nicht umgesetzter ethylenisch ungesättigter Verbindung und gegebenenfalls gesättigtem Kohlenwasserstoff bestehende Ströme etc. Diese können ganz oder teilweise in die Reaktionszone zurückgeführt oder aus dem Verfahren ausgeschleust werden.

Bevorzugt wird aus der Reaktionszone ein flüssiger Austrag entnommen (Flüssigaustragsverfahren). Dieser flüssige Austrag enthält als wesentliche Bestandteile:
i) das Hydroformylierungsprodukt, d. h. die aus dem eingesetzten Olefin oder Olefingemischen erzeugten Aldehyde,
ii) die hochsiedenden Nebenprodukte der Hydroformylierung, wie sie z. B. aus der Aldolreaktion der gebildeten Aldehyde resultieren,
iii) den homogen gelösten Hydroformylierungskatalysator und gegebenenfalls freien Liganden,
iv) gegebenenfalls nicht umgesetzte Olefine,
v) leichtsiedende Komponenten, wie Alkane, und
vi) gelöstes Synthesegas.

Sofern zur Hydroformylierung ein inertes Lösungsmittel eingesetzt wird, ist auch dieses in dem flüssigen Austrag aus der Reaktionszone enthalten. In der Regel werden jedoch als Lösungsmittel die bei der Hydroformylierung (z. B. durch Aldolkondensation) gebildeten, höher als das Hydroformylierungsprodukt siedenden Nebenprodukte eingesetzt.

Die Auftrennung des flüssigen Austrags aus der Reaktionszone unter Erhalt einer im Wesentlichen das Hydroformylierungsprodukt enthaltenden Fraktion und unter Erhalt des katalytisch aktiven Fluids, das die höher als das Hydroformylierungsprodukt siedenden Nebenprodukte der Hydroformylierung und die darin gelöste Metallkomplexverbindung enthält, erfolgt nach üblichen, dem Fachmann bekannten Verfahren. Dazu zählen Entspannungs- und thermische Auftrennungsschritte (Destillationen). Geeignete Trennapparaturen zur Destillation sind z. B. Destillationskolonnen, wie Bodenkolonnen, die gewünschtenfalls mit Glocken, Siebplatten, Siebböden, Ventilen etc. ausgerüstet sein können, Verdampfer, wie Dünnschichtverdampfer, Fallfilmverdampfer, Wischblattverdampfer etc.

Der flüssige Austrag aus der Reaktionszone kann beispielsweise zur Aufarbeitung zunächst einer ein- oder mehrstufigen Entgasung unterzogen werden.

In einer Ausführungsform mit einstufiger Entgasung wird z.B. der flüssige Austrag aus der Reaktionszone in ein Entspannungsgefäß entspannt, wobei in Folge der Druckerniedrigung der Austrag in eine flüssige, den Hydroformylierungskatalysator und gegebenenfalls freien Liganden, die hochsiedenden Nebenprodukte der Hydroformylierung enthaltende Phase und eine den Hauptteil des gebildeten Hydroformylierungsprodukts, gegebenenfalls nicht umgesetzte Olefine, leicht siedende Komponenten und überschüssiges Synthesegas enthaltende gasförmige Phase aufgetrennt wird. Die das katalytisch aktive Fluid bildende flüssige Phase kann zur Recyclierung des Katalysators als Rückführstrom, gegebenenfalls nach Abtrennung wenigstens eines Teils der hochsiedenden Nebenprodukte, wieder in die Reaktionszone geleitet werden. Die Gasphase kann zur weiteren Aufarbeitung z. B. einem Kondensator zugeführt werden, in welchem das Hydroformylierungsprodukt flüssig abgeschieden wird. Die in dem Kondensator anfallende Gasphase, die im Wesentlichen nicht umgesetztes Synthesegas, nicht umgesetztes Olefin sowie Inertkomponenten enthält, kann, gegebenenfalls nach Abtrennung wenigstens eines Teils der Inertkomponenten, ganz oder teilweise in die Reaktionszone zurückgeführt werden.

In einer weiteren Ausführungsform des Flüssigaustragsverfahrens mit Entgasung wird der flüssige Austrag aus der Reaktionszone zur Aufarbeitung einer zweistufigen Entgasung unterzogen. Dabei kann die erste Entgasungsstufe auch als eine Ruhezone ausgestaltet sein, in der kein Eintrag von Gas in die Flüssigphase erfolgt. Die in dieser Ruhe-/Entspannungsstufe erhaltene Gasphase besteht im Wesentlichen aus Synthesegas. Die aus der Ruhe-/Entspannungsstufe erhaltene Flüssigphase kann in einer zweiten Entspannungsstufe (Entgasungsstufe) wiederum in eine Flüssigphase und eine Gasphase aufgetrennt werden. Die so erhaltene zweite Flüssigphase enthält im Allgemeinen die höher als das Hydroformylierungsprodukt siedenden Nebenprodukte, den homogen gelösten ersten Hydroformylierungskatalysator und gegebenenfalls einen Teil des Hydroformylierungsprodukts. Die zweite Gasphase enthält das nicht umgesetzte Olefin, gesättigte Kohlenwasserstoffe und ebenfalls einen Teil des Hydroformylierungsprodukts. Zur Isolierung des katalytisch aktiven Fluids einerseits und einer die Hauptmenge des Hydroformylierungsprodukts enthaltenden Fraktion andererseits kann sich an die zweite Entspannunsstufe eine thermische Aufarbeitung anschließen. Bei diesem thermischen Abtrennungsschritt kann es sich beispielsweise um eine Destillation handeln. Bevorzugt werden zur Destillation zweite Flüssigphase und zweite Gasphase aus dem zweiten Entspannungsschritt im Gegenstrom geführt und so in besonders innigen Kontakt gebracht (strippen). In einer bevorzugten Ausführungsform ist die zweite Entspannungsstufe als Kombination des Entspannungsschritts (Flash) mit einem thermischen Abtrennungsschritt ausgeführt (Flash/strip-Stufe).

Alternativ zu den zuvor beschriebenen reinen Flüssigaustragsverfahren kann das so genannte Kreisgasverfahren eingesetzt werden, bei dem aus dem Gasraum der Reaktionszone ein weiterer gasförmiger Austrag entnommen wird. Dieser gasförmige Austrag besteht im Wesentlichen aus Synthesegas, unumgesetzten Olefinen und Inertkomponenten, wobei nach Maßgabe des Dampfdrucks des Hydroformylierungsprodukts in der Reaktionszone auch ein Teil der gebildeten Hydroformylierungsprodukte mit ausgeschleust werden. Aus dem Gasstrom kann das mitgeführte Hydroformylierungsprodukt z. B. durch Abkühlen auskondensiert und der vom Flüssiganteil befreite Gasstrom wieder zurück in die Reaktionszone geführt werden.

Erfindungsgemäß sind die in dem erfindungsgemäßen Verfahren eingesetzten Basen ausgewählt unter in dem katalytisch aktiven Fluid löslichen Basen ausgewählt unter Trialkylaminen, Dialkylarylaminen, Alkyldiarylaminen, Triarylaminen und stickstoffhaltigen Heterocyclen, an eine Festphase immobilisierten Basen und Kombinationen davon. Vorzugsweise ist die Base ausgewählt unter basischen Stickstoffverbindungen.

Bevorzugt werden als Basen Stickstoffverbindungen eingesetzt, die keine primären und sekundären Stickstoffatome (d. h. solche, die noch gebundene H-Atome enthalten) aufweisen. Basische Stickstoffverbindungen, die als Verunreinigung Verbindungen mit primären und sekundären Stickstoffatomen enthalten, z. B. tertiäre Amine, die produktionsbedingt mit primären und/oder sekundären Aminen verunreinigt sind, können vor ihrem Einsatz in dem erfindungsgemäßen Verfahren einer Aufarbeitung zur Abtrennung wenigstens eines Teils dieser Verbindungen unterzogen werden.

Geeignete Basen sind z. B. Trialkylamine. Trialkylamine, die einen Siedepunkt unterhalb oder im Bereich der Produktaldehyde aufweisen, wie es in der Regel für Tri-(C₁-C₃-)alkylamine der Fall ist, eignen sich weniger, wenn die Produktaldehyde destillativ vom Reaktionsaustrag abgetrennt werden.

Des Weiteren bevorzugt ist die Base ausgewählt unter Dialkylarylaminen, bevorzugt Di-(C₁-C₄-)alkylarylaminen, wobei die Alkylgruppen und/oder die Arylgruppe zusätzlich substituiert sein können. Die Arylgruppe steht vorzugsweise für Phenyl. Dazu zählen z. B. N,N-Dimethylanilin, N,N-Diethylanilin, N,N,2,4,6-Pentamethylanilin, Bis(4-(N,N-Dimethylamino)phenyl)methylen, 4,4'-Bis(N,N-dimethylamino)benzophenon etc.

Des Weiteren bevorzugt ist die Base ausgewählt unter Alkyldiarylaminen, bevorzugt (C₁-C₄-)Alkyldiarylaminen, wobei die Alkylgruppe und/oder die Arylgruppen gegebenenfalls substituiert sein können. Dazu zählen z. B. Diphenylmethylamin und Diphenylethylamin.

Des Weiteren bevorzugt ist die Base ausgewählt unter Triarylaminen, wobei die Arylgruppen gegebenenfalls substituiert sein können, wie Triphenylamin etc. Des Weiteren bevorzugte Amine sind Tricycloalkylamine, wie Tricyclohexylamin.

Des Weiteren bevorzugt ist die Base ausgewählt unter stickstoffhaltigen Heterocyclen. Vorzugsweise sind die stickstoffhaltigen Heterocyclen ausgewählt aus der Gruppe der Pyrrole, Pyrrolidine, Pyridine, Chinoline, Isochinoline, Purine, Pyrazole, Imidazole, Triazole, Tetrazole, Indolizine, Pyridazine, Pyrimidine, Pyrazine, Triazine, Indole, Isoindole, Oxazole, Oxazolidone, Oxazolidine, Morpholine, Piperazine, Piperidine und deren Derivaten.

Geeignete Derivate der zuvor genannten stickstoffhaltigen Heterocyclen können z. B. einen oder mehrere C₁-C₆-Alkylsubstituenten, wie Methyl, Ethyl, n-Propyl, Isopropyl, n-Butyl, sec.-Butyl, tert.-Butyl etc., aufweisen.

Als Basen bevorzugte Heterocyclen sind Pyrrole, Indole, Pyridine, Chinoline und Triazole, die zusätzlich einen oder mehrere C₁-C₆-Alkylsubstituenten aufweisen können. Dazu zählen beispielsweise 3-Alkylindole, wie 3-Methylindol, 2,6-Dialkylpyridine, wie 2,6-Dimethylpyridin, Chinolin und 1-H-Benztriazol.

Die zuvor genannten Basen können einzeln oder in Form von beliebigen Mischungen eingesetzt werden.

Beim Einsatz wenigstens einer in dem katalytisch aktiven Fluid löslichen Base wird in der Reaktionszone vorzugsweise ein molares Verhältnis von Base zu Phosphoramiditverbindung von 0,01:1 bis 5:1, vorzugsweise von 0,1:1 bis 1,5:1, aufrecht erhalten. Dazu kann beispielsweise der pH-Wert der Reaktionsmischung in regelmäßigen Abständen kontrolliert und im Bedarfsfall Base der Reaktionsmischung zugesetzt werden.

Sofern die Aufarbeitung des Reaktionsaustrags, wie zuvor beschrieben, einen thermischen Trennschritt umfasst, werden vorzugsweise hochsiedende lösliche Basen eingesetzt, die unter den Bedingungen der thermischen Aufarbeitung einen ausreichend höheren Siedepunkt aufweisen als die Hydroformylierungsprodukte. Bevorzugt erfolgt die Auftrennung des Austrags aus der Reaktionszone so, dass die dabei erhaltene, das Hydroformylierungsprodukt enthaltende Fraktion im Wesentlichen frei von der eingesetzten Base ist. Bevorzugt erfolgt die Auftrennung des Austrags aus der Reaktionszone des Weiteren so, dass die Base im Wesentlichen in der das katalytisch aktive Fluid bildenden Fraktion enthalten ist und mit diesem in die Reaktionszone zurückgeführt wird.

In einer Ausführungsform des erfindungsgemäßen Verfahrens wird als Base wenigstens eine an eine Festphase immobilisierte Base eingesetzt. Geeignete immobilisierte Basen sind prinzipiell die dem Fachmann bekannten basischen Ionenaustauscher. Die Festphase dieser basischen Ionenaustauscher umfasst beispielweise eine Polymermatrix. Dazu zählen z. B. Polystyrolmatrices, die neben Styrol wenigstens ein vernetzendes Monomer, z. B. Divinylbenzol, sowie gegebenenfalls weitere Comonomere einpolymerisiert enthalten. Geeignet sind weiterhin Polyacrylmatrices, die durch Polymerisation wenigstens eines (Meth)acrylats, wenigstens eines vernetzenden Monomers sowie gegebenenfalls weiterer Comonomere erhalten werden. Geeignete Polymermatrices sind auch Phenol-Formaldehyd-Harze und Polyalkylamin-Harze, die beispielsweise durch Kondensation von Polyaminen mit Epichlorhydrin erhalten werden.

Die an die Festphase direkt oder über eine Spacergruppe gebundenen so genannten Ankergruppen (deren locker gebundene Gegenionen gegen gleichsinnig geladene Ionen ausgetauscht werden können) sind vorzugsweise ausgewählt unter stickstoffhaltigen Gruppen, vorzugsweise tertiären und quartären Aminogruppen. Bevorzugt sind Ankergruppen, die in der freien Basenform vorliegen.

Geeignete funktionelle Gruppen sind z. B. (geordnet nach abnehmender Basizität):

-CH₂N⁺(CH₃)₃ OH⁻ z. B. Duolite A 101

-CH₂N⁺(CH₃)₂CH₂CH₂OH OH⁻ z. B. Duolite A 102

-CH₂N(CH₃)₂ z. B. Amberlite IRA 67

-CH₂NHCH₃

-CH₂NH₂ z. B. Duolite A 365

Für das erfindungsgemäße Verfahren eignen sich sowohl stark als auch schwach basische Ionenaustauscher, bevorzugt sind schwach basische Ionenaustauscher. Unter den schwach basischen Ionenaustauschern sind solche, die tertiäre Aminogruppen aufweisen bevorzugt. Stark basische Ionenaustauscher weisen in der Regel quaternäre Ammoniumgruppen als Ankergruppen auf. Ein schwach basischer Ionenaustauscher ist in der Regel dadurch charakterisiert, dass er nach der Regenerierung in der freien Basenform vorliegt.

Für das erfindungsgemäße Verfahren geeignete kommerziell erhältliche Ionenaustauscher sind z. B. Amberlite® IRA 67 und Amberlyst A21.

Üblicherweise enthalten Ionenaustauscher eine hydrophile Sphäre von gebundenem Wasser. Bevorzugt werden die an eine Festphase immobilisierten Basen vor ihrem Einsatz in dem erfindungsgemäßen Verfahren mit wenigstens einem wasserfreien Lösungsmittel in Kontakt gebracht, um gebundenes Wasser teilweise oder vollständig zu entfernen. Besonders bevorzugt erfolgt dabei zunächst eine Behandlung mit einem wasserlöslichen bzw. wassermischbaren Lösungsmittel und anschließend mit einem im Wesentlichen wasserunlöslichen Lösungsmittel. Geeignete mit Wasser mischbare Lösungsmittel sind z.B. Alkohole, wie Methanol, Ethanol, n-Propanol, Isopropanol etc. Geeignete im Wesentlichen wasserunlösliche Lösungsmittel sind z. B. Aromaten, wie Toluol und Xylole, Kohlenwasserstoffe und Kohlenwasserstoffgemische sowie hochsiedende Alkohole, z. B. 2-Propylheptanol. Überraschenderweise wurde gefunden, dass die erfindungsgemäß eingesetzten Ionenaustauscher auch in im Wesentlichen wasserfreiem Medium geeignet sind, Phosphoramiditverbindungen gegenüber einem Abbau zu stabilisieren bzw. die entsprechenden Hydroformylierungskatalysatoren vor einer Deaktivierung zu schützen.

Das Inkontaktbringen des katalytisch aktiven Fluids mit einer immobilisierten Base erfolgt vorzugsweise, indem man aus der Reaktionszone einen flüssigen Austrag entnimmt und vor oder nach seiner Auftrennung mit der immobilisierten Base in Kontakt bringt. Bevorzugt wird die aus dem Austrag nach seiner Auftrennung gewonnene, das katalytisch aktive Fluid bildende Fraktion mit der immobilisierten Base in Kontakt gebracht. Die Base kann sowohl in Form einer Aufschlämmung als auch in Form einer Packung, z. B. als Festbett, vorliegen.

Das Regenerieren der immobilisierten Base erfolgt nach üblichen, dem Fachmann bekannten Verfahren, wie z. B. durch Behandlung mit wässriger Base. Geeignete Basen sind beispielsweise Ammoniak, Alkalicarbonate, wie Natriumcarbonat und Kaliumcarbonat, Alkalihydroxide, wie Natriumhydroxid und Kaliumhydroxid. Bevorzugt erfolgt dabei vor dem Regenerieren zunächst eine Behandlung mit einem der zuvor genannten wasserlöslichen bzw. wassermischbaren Lösungsmittel. Vorzugsweise schließt sich an die Regeneration wenigstens ein Spülschritt mit einem trockenen organischen Lösungsmittel, wie zuvor beschrieben, an. Besonders bevorzugt erfolgt auch dabei zunächst eine Behandlung mit einem wasserlöslichen bzw. wassermischbaren Lösungsmittel und anschließend mit einem im Wesentlichen wasserunlöslichen Lösungsmittel.

In einer bevorzugten Ausführungsform des erfindüngsgemäßen Verfahrens wird eine . Kombination aus wenigstens einer in dem katalytischen Fluid löslichen Base und wenigstens einer an eine Festphase immobilisierten Base eingesetzt. Die Basenpaare werden dabei so ausgewählt, dass die immobilisierten Basen befähigt sind; aus den durch Umsetzung der löslichen Basen mit Säuren erhaltenen Säure-Base-Addukten die löslichen Basen zumindest teilweise freizusetzen. Dazu werden die Basen so ausgewählt, dass die Basenstärken der flüssigen Basen unter den Reaktionsbedingungen geringer sind als die Basenstärken der immobilisierten Basen. Diese Basenstärken kann der Fachmann durch einfache Routineexperimente ermitteln. Einen guten Anhaltspunkt geben die für den Einsatz in wässrigen Systemen im Allgemeinen bekannten pK_{b}-Werte der Basen.

Für den Einsatz in dem erfindungsgemäßen Verfahren geeignete Phosphoramiditverbindungen sind in der WO 00/56451, WO 02/083695, WO 03/018192 und der deutschen Patentanmeldung 102 43 138.8 beschrieben.

Bevorzugt handelt es sich bei dem Metall der VIII. Nebengruppe des Periodensystems um Co, Ru, Rh, Pd, Pt, Os oder Ir, speziell um Rh, Co, Ir oder Ru.

Im Folgenden umfasst der Ausdruck "Alkyl" geradkettige und verzweigte Alkylgruppen. Vorzugsweise handelt es sich dabei um geradkettige oder verzweigte C₁-C₂₀-Alkyl, bevorzugterweise C₁-C₁₂-Alkyl-, besonders bevorzugt C₁-C₈-Alkyl- und ganz besonders bevorzugt C₁-C₄-Alkylgruppen. Beispiele für Alkylgruppen sind insbesondere Methyl, Ethyl, Propyl, Isopropyl, n-Butyl, 2-Butyl, sec.-Butyl, tert.-Butyl, n-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl; 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl, n-Heptyl, 2-Heptyl, 3-Heptyl, 2-Ethylpentyl, 1-Propylbutyl, n-Octyl, 2-Ethylhexyl, 2-Propylheptyl, Nonyl, Decyl.

Der Ausdruck "Alkyl" umfasst auch substituierte Alkylgruppen, welche im Allgemeinen 1,2, 3, 4 oder 5, bevorzugt 1, 2 oder 3 und besonders bevorzugt 1 Substituenten, ausgewählt aus den Gruppen Cycloalkyl, Aryl, Hetaryl, Halogen, NE¹E², NE¹E²E³⁺, COOH, Carboxylat, -SO₃H und Sulfonat, tragen können, worin E¹, E² und E³ jeweils gleiche oder verschiedene Reste, ausgewählt unter Wasserstoff, Alkyl, Cycloalkyl und Aryl bedeuten.

Der Ausdruck "Alkylen" im Sinne der vorliegenden Erfindung steht für geradkettige oder verzweigte Alkandiyl-Gruppen mit 1 bis 4 Kohlenstoffatomen.

Der Ausdruck "Cycloalkyl" umfasst im Sinne der vorliegenden Erfindung unsubstituierte als auch substituierte Cycloalkylgruppen, vorzugsweise C₅-C₇-Cycloalkylgruppen, wie Cyclopentyl, Cyclohexyl oder Cycloheptyl, die im Falle einer Substitution, im Allgemeinen 1, 2, 3, 4 oder 5, bevorzugt 1, 2 oder 3 und besonders bevorzugt 1 Substituenten, ausgewählt aus den Gruppen Alkyl, Alkoxy und Halogen, tragen können.

Der Ausdruck "Heterocycloalkyl" im Sinne der vorliegenden Erfindung umfasst gesättigte, cycloaliphatische Gruppen mit im Allgemeinen 4 bis 7, vorzugsweise 5 oder 6 Ringatomen, in denen 1 oder 2 der Ringkohlenstoffatome durch Heteroatome, ausgewählt aus den Elementen Sauerstoff, Stickstoff und Schwefel, ersetzt sind und die gegebenenfalls substituiert sein können, wobei im Falle einer Substitution, diese heterocycloaliphatischen Gruppen 1, 2 oder 3, vorzugsweise 1 oder 2, besonders bevorzugt 1 Substituenten, ausgewählt aus Alkyl, Aryl, COOR^{f} (R^{f} = Wasserstoff, Alkyl, Cycloalkyl und Aryl), COO⁻M⁺ und NE¹E², bevorzugt Alkyl, tragen können. Beispielhaft für solche heterocycloaliphatischen Gruppen seien Pyrrolidinyl, Piperidinyl, 2,2,6,6-Tetramethylpiperidinyl, Imidazolidinyl, Pyrazolidinyl, Oxazolidinyl, Morpholidinyl, Thiazolidinyl, Isothiazolidinyl, Isoxazolidinyl, Piperazinyl-, Tetrahydrothiophenyl, Tetrahydrofuranyl, Tetrahydropyranyl, Dioxanyl genannt.

Der Ausdruck "Aryl" umfasst im Sinne der vorliegenden Erfindung unsubstituierte als auch substituierte Arylgruppen, und steht vorzugsweise für Phenyl, Tolyl, Xylyl, Mesityl, Naphthyl, Fluorenyl, Anthracenyl, Phenanthrenyl oder Naphthacenyl, besonders bevorzugt für Phenyl oder Naphthyl, wobei diese Arylgruppen im Falle einer Substitution im Allgemeinen 1, 2, 3, 4 oder 5, vorzugsweise 1, 2 oder 3 und besonders bevorzugt 1 Substituenten, ausgewählt aus den Gruppen Alkyl, Alkoxy, Carboxyl, Carboxylat, Trifluormethyl, -SO₃H Sulfonat, NE¹E², Alkylen-NE¹E², Nitro, Cyano oder Halogen, tragen können.

Der Ausdruck "Hetaryl" umfasst im Sinne der vorliegenden Erfindung unsubstituierte oder substituierte, heterocycloaromatische Gruppen, vorzugsweise die Gruppen Pyridyl, Chinolinyl, Acridinyl, Pyridazinyl, Pyrimidinyl, Pyrazinyl, sowie die Untergruppe der "Pyrrolgruppe", wobei diese heterocycloaromatischen Gruppen im Falle einer Substitution im Allgemeinen 1, 2 oder 3 Substituenten, ausgewählt aus den Gruppen Alkyl, Alkoxy, Carboxyl, Carboxylat, -SO₃H, Sulfonat, NE¹E², Alkylen-NE¹E², Trifluormethyl oder Halogen, tragen können.

Der Ausdruck "Pyrrolgruppe" steht im Sinne der vorliegenden Erfindung für eine Reihe unsubstituierter oder substituierter, heterocycloaromatischer Gruppen, die strukturell vom Pyrrolgrundgerüst abgeleitet sind und ein pyrrolisches Stickstoffatom im Heterocyclus enthalten, das kovalent mit anderen Atomen, beispielsweise einem Pnicogenatom, verknüpft werden kann. Der Ausdruck "Pyrrolgruppe" umfasst somit die unsubstituierten oder substituierten Gruppen Pyrrolyl, Imidazolyl, Pyrazolyl, Indolyl, Purinyl, Indazolyl, Benzotriazolyl, 1,2,3-Triazolyl, 1,3,4-Triazolyl und Carbazolyl, die im Falle einer Substitution im Allgemeinen 1, 2 oder 3, vorzugsweise 1 oder 2, besonders bevorzugt 1 Substituenten, ausgewählt aus den Gruppen Alkyl, Alkoxy, Acyl, Carboxyl, Carboxylat, -SO₃H, Sulfonat, NE¹E², Alkylen-NE¹E², Trifluormethyl oder Halogen, tragen können. Eine bevorzugte substituierte Indolylgruppe ist die 3-Methyl-indolylgruppe.

Dementsprechend umfasst der Ausdruck "Bispyrrolgruppe" im Sinne der vorliegenden Erfindung zweibindige Gruppen der Formel

Py-I-Py,

die zwei durch direkte chemische Bindung oder Alkylen-, Oxa-, Thio-, Imino-, Silyl oder Alkyliminogruppen vermittelte Verknüpfung, verbundene Pyrrolgruppen enthalten, wie die Bisindoldiyl-Gruppe der Formel 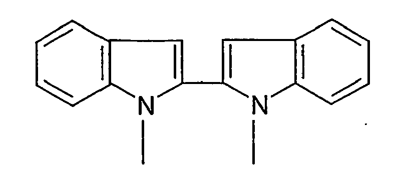 als Beispiel für eine Bispyrrolgruppe, die zwei direkt verknüpfte Pyrrolgruppen, in diesem Falle Indolyl, enthält, oder die Bispyrroldiylmethan-Gruppe der Formel 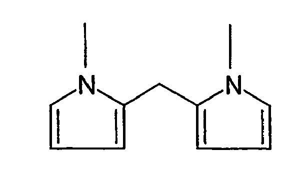 als Beispiel für eine Bispyrrolgruppe, die zwei über eine Methylengruppe verknüpfte Pyrrolgruppen, in diesem Falle Pyrrolyl, enthält. Wie die Pyrrolgruppen können auch die Bispyrrolgruppen unsubstituiert oder substituiert sein und im Falle einer Substitution pro Pyrrolgruppeneinheit im Allgemeinen 1, 2 oder 3, vorzugsweise 1 oder 2, insbesondere 1 Substituenten, ausgewählt aus Alkyl, Alkoxy, Carboxyl, Carboxylat, -SO₃H, Sulfonat, NE¹E², Alkylen-NE¹E², Trifluormethyl oder Halogen, tragen, wobei bei diesen Angaben zur Anzahl möglicher Substituenten die Verknüpfung der Pyrrolgruppeneinheiten durch direkte chemische Bindung oder durch die mittels der vorstehend genannten Gruppen vermittelte Verknüpfung nicht als Substitution betrachtet wird.

Carboxylat und Sulfonat stehen im Rahmen dieser Erfindung vorzugsweise für ein Derivat einer Carbonsäurefunktion bzw. einer Sulfonsäurefunktion, insbesondere für ein Metallcarboxylat oder -sulfonat, eine Carbonsäure- oder Sulfonsäureesterfunktion oder eine Carbonsäure- oder Sulfonsäureamidfunktion. Dazu zählen z. B. die Ester mit C₁-C₄-Alkanolen, wie Methanol, Ethanol, n-Propanol, Isopropanol, n-Butanol, sec.-Butanol und tert.-Butanol. Dazu zählen weiterhin die primären Amide und deren N-Alkyl- und N,N-Dialkylderivate.

Die obigen Erläuterungen zu den Ausdrücken "Alkyl", "Cycloalkyl", "Aryl", "Heterocycloalkyl" und "Hetaryl" gelten entsprechend für die Ausdrücke "Alkoxy", "Cycloalkoxy", "Aryloxy", "Heterocycloalkoxy" und "Hetaryloxy".

Der Ausdruck "Acyl" steht im Sinne der vorliegenden Erfindung für Alkanoyl- oder Aroylgruppen mit im Allgemeinen 2 bis 11, vorzugsweise 2 bis 8 Kohlenstoffatomen, beispielsweise für die Acetyl-, Propanoyl-, Butanoyl-, Pentanoyl-, Hexanoyl-, Heptanoyl-, 2-Ethylhexanoyl-, 2-Propylheptanoyl-, Benzoyl- oder Naphthoyl-Gruppe.

Die Reste E¹ bis E¹² sind unabhängig voneinander ausgewählt unter Wasserstoff, Alkyl, Cycloalkyl und Aryl. Die Gruppen NE¹E², NE⁴E⁵, NE⁷E⁸ und NE¹⁰E¹¹ stehen vorzugsweise für N,N-Dimethylamino, N,N-Diethylamino, N,N-Dipropylamino, N,N-Diisopropylamino, N,N-Di-n-butylamino, N,N-Di-t.-butylamino, N,N-Dicyclohexylamino oder N,N-Diphenylamino.

Halogen steht für Fluor, Chlor, Brom und Iod, bevorzugt für Fluor, Chlor und Brom.

M⁺ steht für ein Kationäquivalent, d. h. für ein einwertiges Kation oder den einer positiven Einfachladung entsprechenden Anteil eines mehrwertigen Kations. Das Kation M⁺ dient lediglich als Gegenion zur Neutralisation negativ geladener Substituentengruppen, wie der COO- oder der Sulfonat-Gruppe und kann im Prinzip beliebig gewählt werden. Vorzugsweise werden deshalb Alkalimetall-, insbesondere Na⁺, K⁺-, Li⁺-Ionen oder Onium-Ionen, wie Ammonium-, Mono-, Di-, Tri-, Tetraalkylammonium-, Phosphonium-, Tetraalkylphosphonium- oder Tetraarylphosphonium-Ionen verwendet.

Entsprechendes gilt für das Anionäquivalent X⁻, das lediglich als Gegenion positiv geladener Substituentengruppen, wie den Ammoniumgruppen, dient und beliebig gewählt werden kann unter einwertigen Anionen und den einer negativen Einfachladung entsprechenden Anteilen eines mehrwertigen Anions. Geeignete Anionen sind z.B. Halogenid-Ionen X', wie Chlorid und Bromid. Bevorzugte Anionen sind Sulfat und Sulfonat, z.B. SO₄²⁻, Tosylat, Trifluormethansulfonat und Methylsulfonat.

Die Werte für x und y stehen für eine ganze Zahl von 1 bis 240, vorzugsweise für eine ganze Zahl von 3 bis 120.

Kondensierte Ringsysteme können durch Anellierung verknüpfte (ankondensierte) aromatische, hydroaromatische und cyclische Verbindungen sein. Kondensierte Ringsysteme bestehen aus zwei, drei oder mehr als drei Ringen. Je nach der Verknüpfungsart unterscheidet man bei kondensierten Ringsystemen zwischen einer ortho-Anellierung, d. h. jeder Ring hat mit jedem Nachbarring jeweils eine Kante, bzw. zwei Atome gemeinsam, und einer peri-Anellierung, bei der ein Kohlenstoffatom mehr als zwei Ringen angehört. Bevorzugt unter den kondensierten Ringsystemen sind ortho-kondensierte Ringsysteme.

Erfindungsgemäß ist die in dem erfindungsgemäßen Verfahren eingesetzte Phosphoramiditverbindung ausgewählt unter Verbindungen der allgemeinen Formeln I oder II 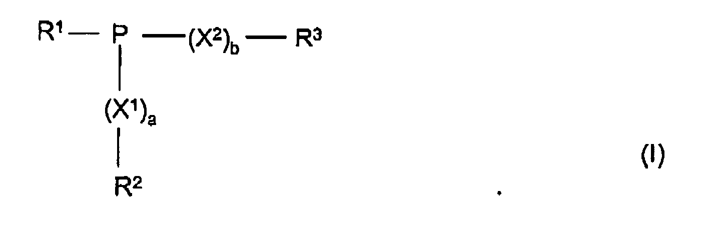 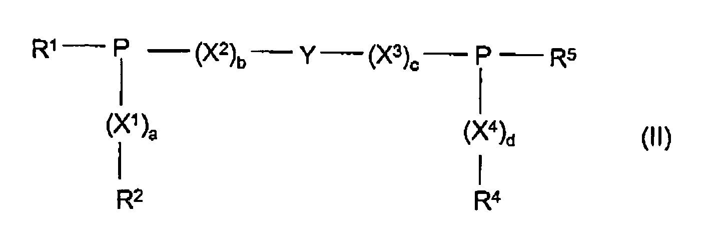 worin
- R¹ und R⁵: unabhängig voneinander für über das Stickstoffatom an das Phosphoratom gebundene Pyrrolgruppen stehen,
- R², R³ und R⁴: unabhängig voneinander für Alkyl, Cycloalkyl, Heterocycloalkyl, Aryl oder Hetaryl stehen,
oder R¹ gemeinsam mit R² und/oder R⁴ gemeinsam mit R⁵ eine zweibindige Gruppe bilden, die mindestens eine über das pyrrolische Stickstoffatom an das Phosphoratom gebundene Pyrrolgruppe enthält,
- Y: für eine zweiwertige verbrückende Gruppe mit 2 bis 20 Brückenatomen zwischen den flankierenden Bindungen steht,
- X¹, X², X³ und X⁴: unabhängig voneinander ausgewählt sind unter O, S, SiR^{α}R^{β} und NR^{γ}, worin R^{α}, R^{β} und R^{γ} unabhängig voneinander für Wasserstoff, Alkyl, Cycloalkyl, Heterocycloalkyl, Aryl oder Hetaryl stehen, und
- a, b, c und d: unabhängig voneinander für 0 oder 1 stehen.

Die Reste R², R³ und R⁴ in den Formeln (I) und (II) können unabhängig voneinander für Alkyl, Cycloalkyl, Heterocycloalkyl, Aryl oder Hetaryl stehen, wobei die Alkylreste 1, 2, 3, 4 oder 5 Substituenten, ausgewählt unter Cycloalkyl, Heterocycloalkyl, Aryl, Hetaryl, Alkoxy, Cycloalkoxy, Heterocycloalkoxy, Aryloxy, Hetaryloxy, Hydroxy, Thiol, Polyalkylenoxid, Polyalkylenimin, COOH, Carboxylat, SO₃H, Sulfonat, NE⁷E⁸, NE⁷E⁸E⁹⁺X⁻, Halogen, Nitro, Acyl oder Cyano aufweisen können, worin E⁷, E⁸ und E⁹ jeweils gleiche oder verschiedene Reste, ausgewählt unter Wasserstoff, Alkyl, Cycloalkyl, oder Aryl bedeuten und X⁻ für ein Anionäquivalent steht, und wobei die Cycloalkyl-, Heterocycloalkyl-, Aryl- und Hetarylreste R², R³ und R⁴ je 1, 2, 3, 4 oder 5 Substituenten aufweisen können, die ausgewählt sind unter Alkyl und den zuvor für die Alkylreste R², R³ und R⁴ genannten Substituenten.

Vorteilhaft stehen auch die Substituenten R², R³ und/oder R⁴ für über das pyrrolische Stickstoffatom an das Phosphoratom gebundene Pyrrolgruppen.

Besonders bevorzugt sind die in dem erfindungsgemäßen Verfahren eingesetzten Phosphoramiditverbindungen ausgewählt unter Chelatphosphoramiditen. Besonders bevorzugte Chelatphosphoramidite sind die Phosphoramiditverbindungen der allgemeinen Formel II.1 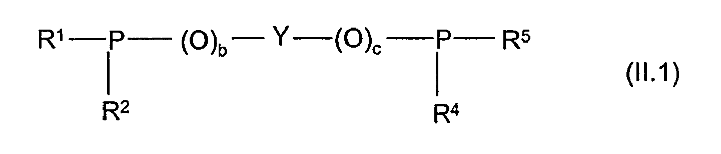 worin
R¹, R², R⁴, R⁵, Y, b und c die zuvor angegebenen Bedeutungen besitzen.

In einer ersten bevorzugten Ausführungsform stehen die Substituenten R¹, R², R⁴ und R⁵ für über das pyrrolische Stickstoffatom an das Phosphoratom gebundene Pyrrolgruppen, wobei R¹ und R² bzw. R⁴ und R⁵ nicht miteinander verbunden sind. Die Bedeutung des Begriffs Pyrrolgruppe entspricht dabei der zuvor gegebenen Definition.

Bevorzugt sind Phosphorchelatverbindungen, in denen die Reste R¹, R², R⁴ und R⁵ unabhängig voneinander ausgewählt sind unter Gruppen der Formeln III.a bis III.k 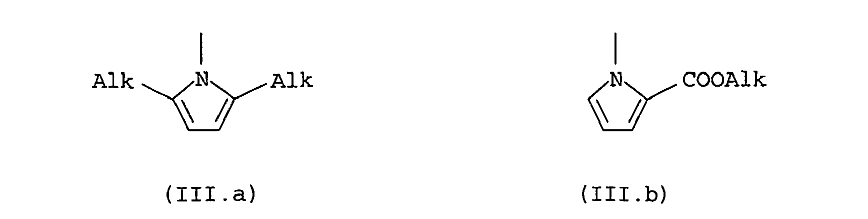 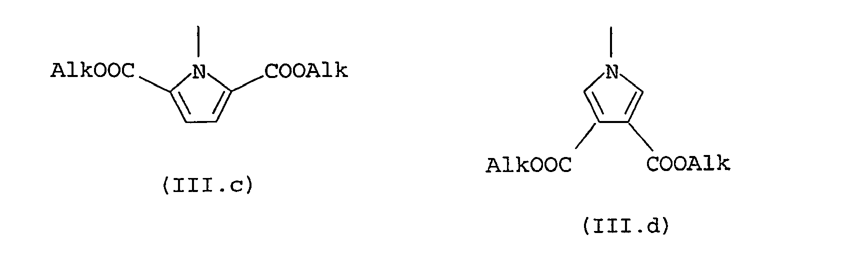 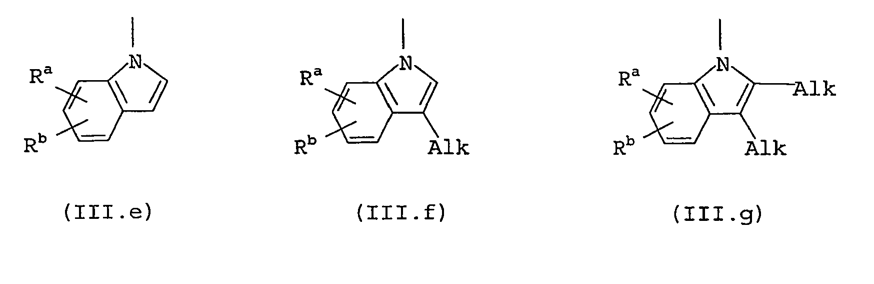 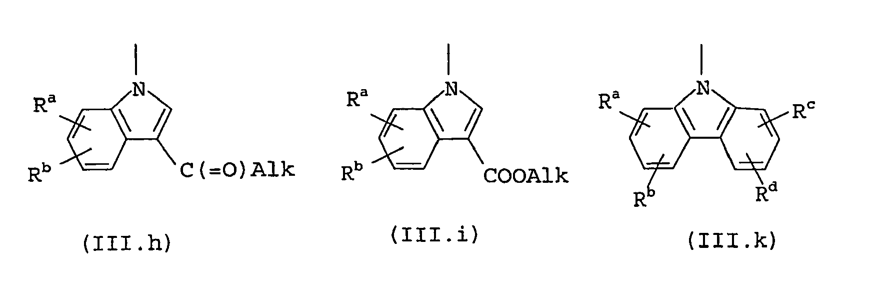 worin
Alk eine C₁-C₁₂-Alkylgruppe ist und
- R^{a}, R^{b}, R^{c} und R^{d}: unabhängig voneinander für Wasserstoff, C₁-C₄-Alkyl, C₁-C₄-Alkoxy, Acyl, Halogen, C₁-C₄-Alkoxycarbonyl oder Carboxyl stehen.

Zur Veranschaulichung werden im Folgenden einige vorteilhafte Pyrrolgruppen aufgelistet:

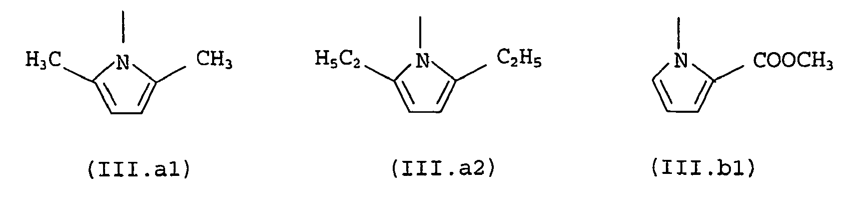

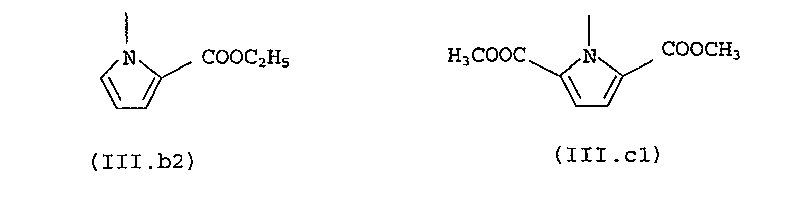

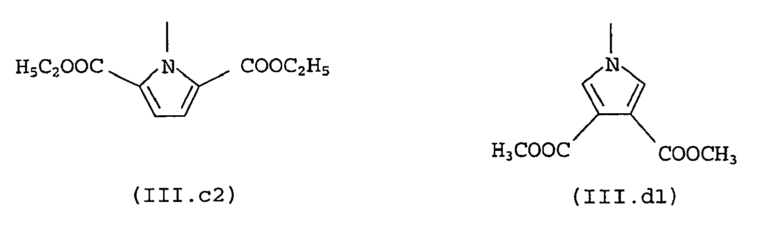

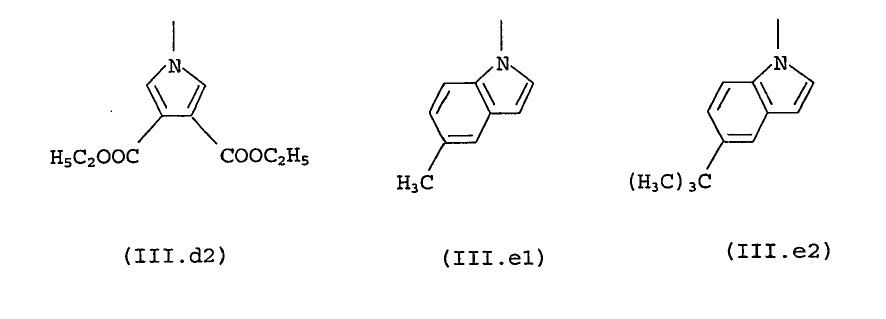

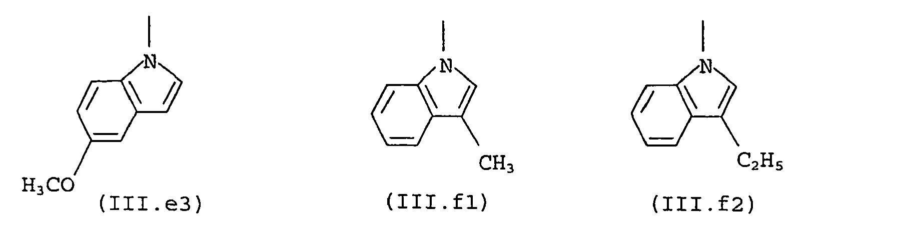

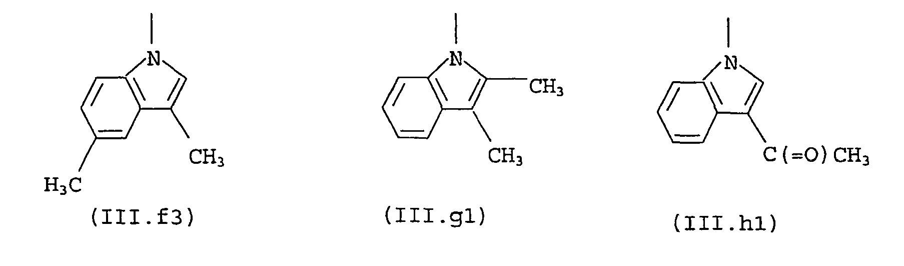

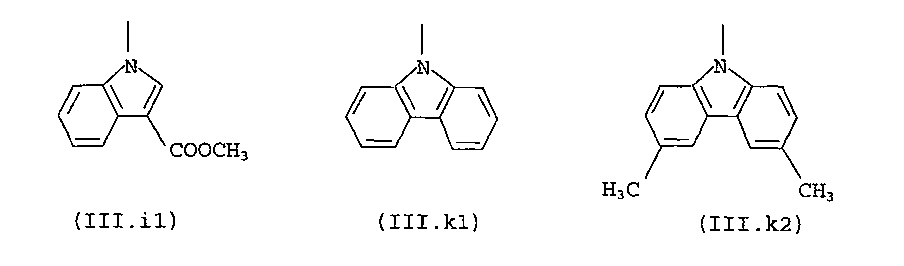

Besonders vorteilhaft ist die 3-Methylindolylgruppe (Skatolylgruppe) der Formel III.f1. Hydroformylierungskatalysatoren auf Basis von Liganden, die eine oder mehrere 3-Methylindolylgruppe(n) an das Phosphoratom gebunden aufweisen, zeichnen sich bereits ohne Stabilisierung durch eine Base durch eine besonders hohe Stabilität und somit besonders lange Katalysatorstandzeiten aus.

In einer weiteren vorteilhaften Ausgestaltung der vorliegenden Erfindung kann in den Formeln I, II und II.1 der Substituent R¹ gemeinsam mit dem Substituenten R² und/oder der Substituent R⁴ gemeinsam mit dem Substituenten R⁵ eine über das pyrrolische Stickstoffatom an das Phosphoratom gebundene Pyrrolgruppe enthaltende zweibindige Gruppe der Formel

Py-I-W

bilden,
worin
- Py: eine Pyrrolgruppe ist,
- I: für eine chemische Bindung oder für O, S, SiR^{π}R^{χ}, NR^{ω} oder gegebenenfalls substituiertes C₁-C₁₀-Alkylen, bevorzugt CR^{λ}R^{µ}, steht,
- W: für Cycloalkyloxy oder -amino, Aryloxy oder -amino, Hetaryloxy oder -amino steht
und
R^{π}, R^{χ}, R^{ω}, R^{λ} und R^{µ} unabhängig voneinander für Wasserstoff, Alkyl, Cycloalkyl, Heterocycloalkyl, Aryl oder Hetaryl stehen,
wobei die hierbei verwendeten Bezeichnungen die eingangs erläuterte Bedeutung haben.

Bevorzugte zweibindige Gruppen der Formel

Py-I-W

sind z. B.

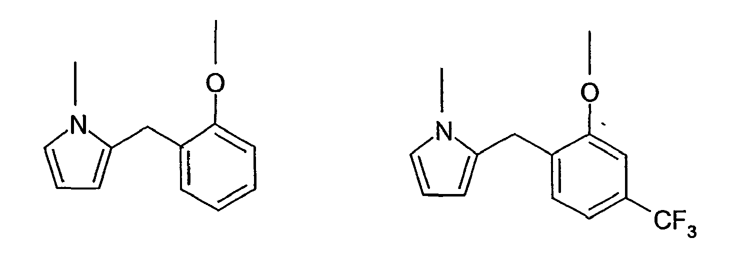

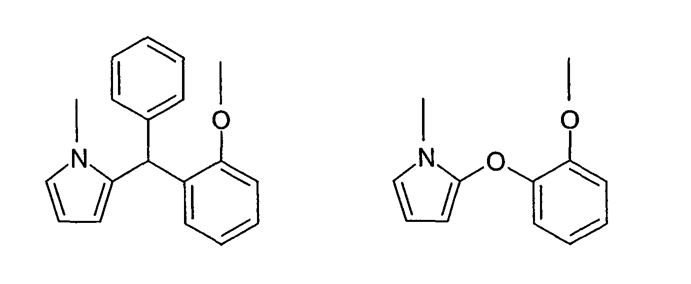

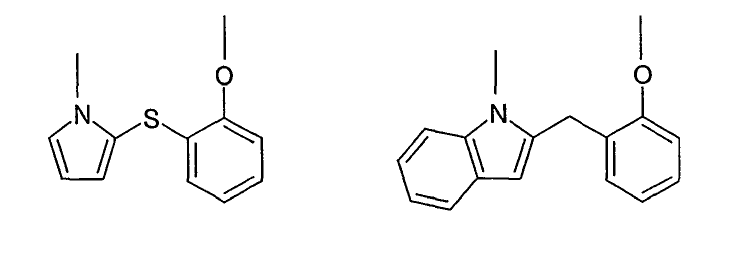

Bevorzugt sind Phosphoramidite, worin der Substituent R¹ gemeinsam mit dem Substituenten R² und/oder der Substituent R⁴ gemeinsam mit dem Substituenten R⁵ eine Bispyrrolgruppe der Formel 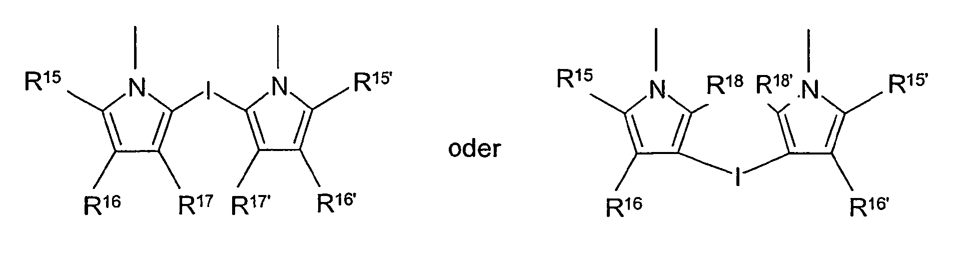 bildet, worin
- I: für eine chemische Bindung oder für O, S, SiR^{π}R^{χ},NR^{ω} oder gegebenenfalls substituiertes C₁-C₁₀-Alkylen, bevorzugt CR^{λ}R^{µ}, steht, worin R^{π}, R^{χ}, R^{ω}, R^{λ} und R^{µ} unabhängig voneinander für Wasserstoff, Alkyl, Cycloalkyl, Heterocycloalkyl, Aryl oder Hetaryl stehen,
- R¹⁵, R¹⁵, R¹⁶, R^{16'}, R¹⁷, R^{17'}, R¹⁸ und R^{18'}: unabhängig voneinander für Wasserstoff, Alkyl, Cycloalkyl, Heterocycloalkyl, Aryl, Hetaryl, W'COOR^{f}, W'COO⁻M⁺, W'(SO₃)R^{f}, W'(SO₃)⁻M⁺, W'PO₃(R^{f})(R^{g}), W'(PO₃)²⁻(M⁺)₂, W'NE¹⁰E¹¹, W'(NE¹⁰E¹¹E¹²)⁺X⁻, W'OR^{f}, W'SR^{f}, (CHR^{g}CH₂O)ₓR^{f}, (CH₂NE¹⁰)ₓR^{f}, (CH₂CH₂NE¹⁰)ₓR^{f}, Halogen, Trifluormethyl, Nitro, Acyl oder Cyano stehen,
worin
W' für eine Einfachbindung, ein Heteroatom, eine Heteroatom-haltige Gruppe oder eine zweiwertige verbrückende Gruppe mit 1 bis 20 Brückenatomen steht,
R^{f}, E¹⁰, E¹¹, E¹² jeweils gleiche oder verschiedene Reste, ausgewählt unter Wasserstoff, Alkyl, Cycloalkyl oder Aryl bedeuten,
R⁹ für Wasserstoff, Methyl oder Ethyl steht,
M⁺ für ein Kationäquivalent steht,
X⁻ für ein Anionäquivalent steht und
x für eine ganze Zahl von 1 bis 240 steht,
wobei jeweils zwei benachbarte Reste R¹⁵ und R¹⁶ und/oder R^{15'} und R^{16'} zusammen mit den Kohlenstoffatomen des Pyrrolrings, an die sie gebunden sind, auch für ein kondensiertes Ringsystem mit 1, 2 oder 3 weiteren Ringen stehen können.

Vorzugsweise steht I für eine chemische Bindung oder eine C₁-C₄-Alkylengruppe, besonders bevorzugt eine Methylengruppe.

Zur Veranschaulichung werden im Folgenden einige vorteilhafte "Bispyrrolylgruppen" aufgelistet:

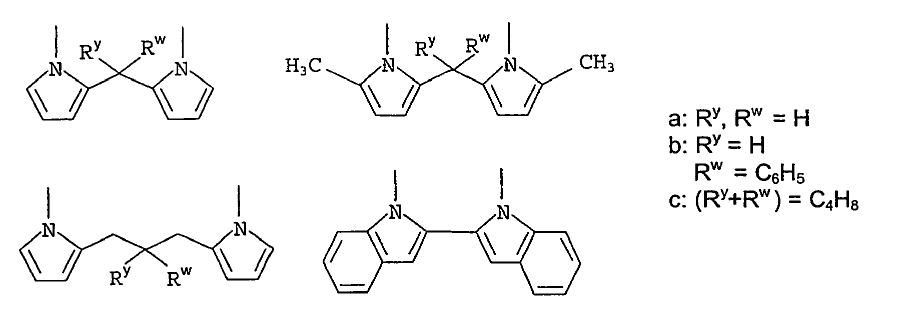

Nach einer bevorzugten Ausführungsform ist die verbrückende Gruppe Y in den Formel (I), (II) und (II.1) ausgewählt unter Gruppen der Formeln IV.a bis IV.u 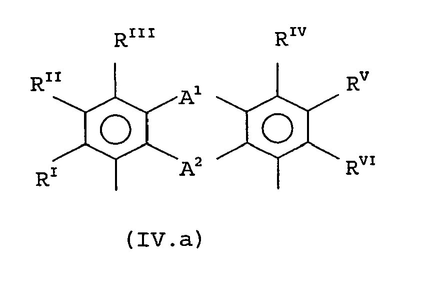 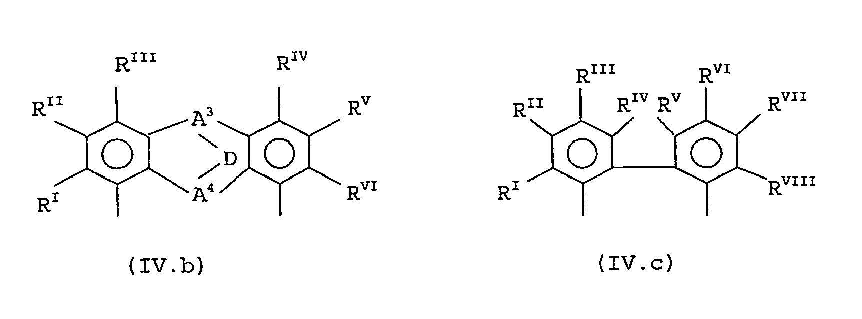 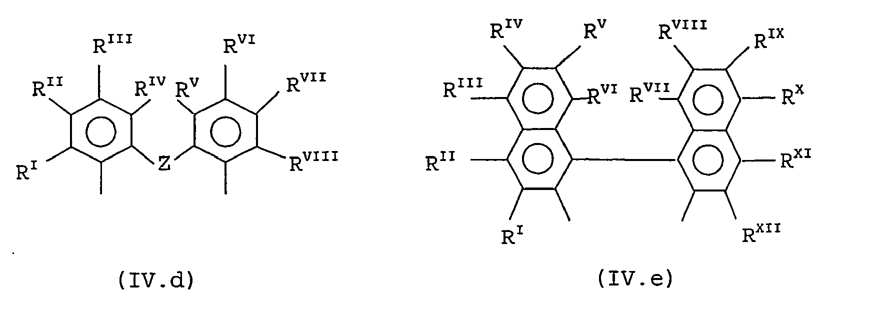 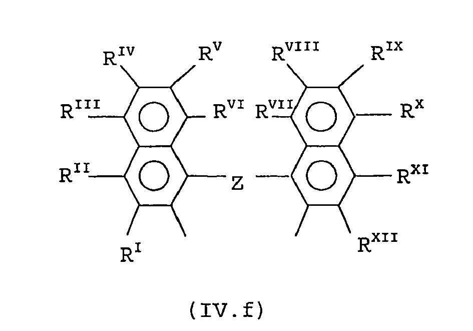 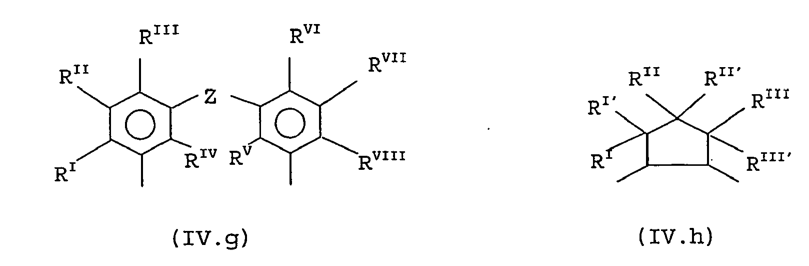 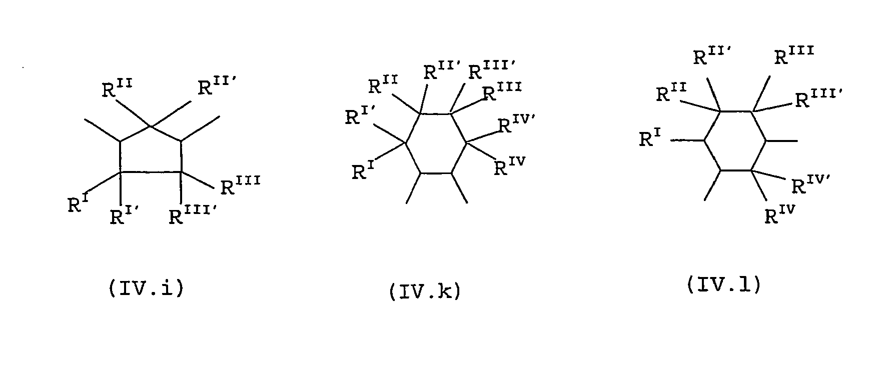 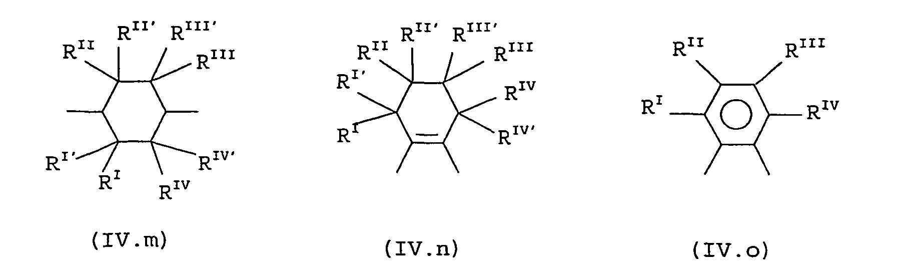 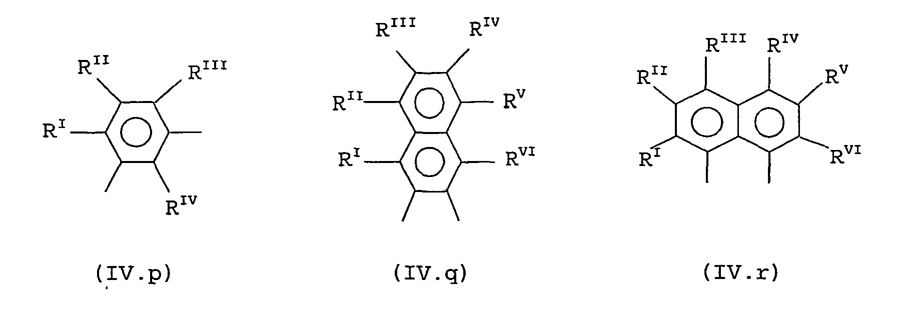 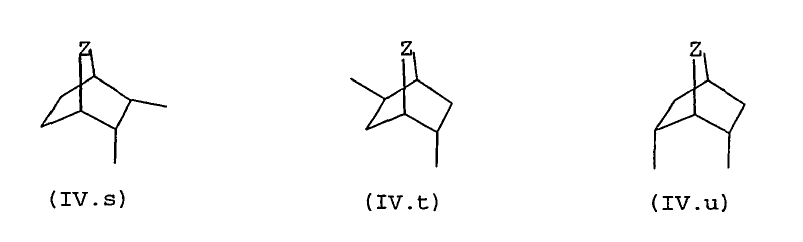 worin
- R^{I}, R^{I'}, R^{II}, R^{II'}, R^{III}, R^{III'}, R^{IV}, R^{IV'}, R^{V},:
- R^{VI}, R^{VII}, R^{VIII}, R^{IX}, R^{X}, R^{XI} und R^{XII}: unabhängig voneinander für Wasserstoff, Alkyl, Cycloalkyl, Heterocycloalkyl, Aryl, Hetaryl; Hydroxy, Thiol, Polyalkylenoxid, Polyalkylenimin, Alkoxy, Halogen, SO₃H, Sulfonat, NE¹E², Alkylen-NE¹E², Nitro, Alkoxycarbonyl, Carboxyl, Acyl oder Cyano stehen, worin E¹ und E² jeweils gleiche oder verschiedene Reste, ausgewählt unter Wasserstoff, Alkyl, Cycloalkyl und Aryl bedeuten,
- Z: für O, S, NR^{δ} oder SiR^{δ}R^{ε} steht, wobei
R^{δ} und R^{ε} unabhängig voneinander für Wasserstoff, Alkyl, Cycloalkyl, Heterocycloalkyl, Aryl oder Hetaryl stehen,
oder Z für eine C₁-C₄-Alkylenbrücke steht, die eine Doppelbindung und/oder einen Alkyl-, Cycloalkyl-, Heterocycloalkyl-, Aryl- oder Hetaryl-Substituenten aufweisen kann,
oder Z für eine C₂-C₄-Alkylenbrücke steht, die durch O, S oder NR^{δ} oder SiR^{δ}R^{ε} unterbrochen ist,
wobei in den Gruppen der Formel IV.a und IV.b zwei benachbarte Reste R^{I} bis R^{VI} gemeinsam mit den Kohlenstoffatomen des Benzolkerns, an die sie gebunden sind, auch für ein kondensiertes Ringsystem mit 1, 2 oder 3 weiteren Ringen stehen können,
wobei in den Gruppen der Formeln IV.h bis IV.n zwei geminale Reste R^{I}, R^{I'}; R^{II}, R^{II"}; R^{III}, R^{III'} und/oder R^{IV}, R^{IV"} auch für Oxo oder ein Ketal davon stehen können,
A¹ und A² unabhängig voneinander für O, S, SiR^{φ}R^{γ}, NR^{η} oder CR^{τ}R^{κ} stehen, wobei R^{φ}, R^{γ}, R^{η}, R^{τ} und R^{κ} unabhängig voneinander für Wasserstoff, Alkyl, Cycloalkyl, Heterocycloalkyl, Aryl oder Hetaryl stehen,
- A³ und A⁴: unabhängig voneinander für SiR^{φ}, N oder CR^{τ} stehen,
- D: für eine zweibindige Brückengruppe der allgemeinen Formel 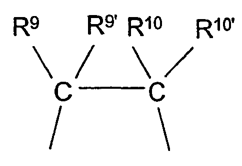 steht, in der
- R⁹, R^{9'}, R¹⁰ und R^{10'}: unabhängig voneinander für Wasserstoff, Alkyl, Cycloalkyl, Aryl, Halogen, Trifluormethyl, Carboxyl, Carboxylat oder Cyano stehen,
wobei R^{9'} auch gemeinsam mit R^{10'} für den Bindungsanteil einer Doppelbindung zwischen den beiden Kohlenstoffatomen, an die R^{9'} und R^{10'} gebunden sind, stehen kann, und/oder R⁹ und R¹⁰ gemeinsam mit den Kohlenstoffatomen, an die sie gebunden sind, auch für einen 4- bis 8-gliedrigen Carbo- oder Heterocyclus stehen können, der gegebenenfalls zusätzlich ein-, zwei- oder dreifach mit Cycloalkyl, Heterocycloalkyl, Aryl oder Hetaryl anelliert ist, wobei der Heterocyclus und, falls vorhanden, die anellierten Gruppen unabhängig voneinander je einen, zwei, drei oder vier Substituenten tragen können, die ausgewählt sind unter Alkyl, Cycloalkyl, Heterocycloalkyl, Aryl, Hetaryl, COOR^{f}, COO⁻M⁺, SO₃R^{f}, SO⁻₃M⁺, NE⁴E⁵, Alkylen-NE⁴E⁵, NE⁴E⁵E⁶⁺X⁻, Alkylen-NE⁴E⁵E⁶⁺X⁻, OR^{f}, SR^{f}, (CHR^{e}CH₂O)_{y}R^{f}, (CH₂N(E⁴))_{y}R^{f}, (CH₂CH₂N(E ⁴))_{y}R^{f}, Halogen, Trifluormethyl, Nitro, Acyl oder Cyano, stehen, worin
- R^{f}, E⁴, E⁵ und E⁶: jeweils gleiche oder verschiedene Reste, ausgewählt unter Wasserstoff, Alkyl, Cycloalkyl oder Aryl bedeuten,
- R^{e}: für Wasserstoff, Methyl oder Ethyl steht,
- M⁺: für ein Kation steht,
- X⁻: für ein Anion steht, und
- y: für eine ganze Zahl von 1 bis 240 steht.

Bevorzugt steht die Brückengruppe Y für eine Gruppe der Formel IV.a, worin die Gruppen A¹ und A² ausgewählt sind aus den Gruppen O, S und CRⁱR^{k}, insbesondere unter O, S, der Methylengruppe (Rⁱ = R^{k} = H), der Dimethylmethylengruppe (Rⁱ = R^{k} = CH₃), der Diethylmethylengruppe (Rⁱ = R^{k} = C₂H₅), der Di-n-propyl-methylengruppe (Rⁱ = R^{k} = n-Propyl) oder der Di-n-butylmethylengruppe (R^{d} = R^{e} = n-Butyl). Insbesondere sind solche Brückengruppen Y bevorzugt, in denen A¹ von A² verschieden ist, wobei A¹ bevorzugt eine CRⁱR^{k}-Gruppe und A² bevorzugt eine O- oder S-Gruppe, besonders bevorzugt eine Oxagruppe O ist.

Bevorzugt steht die Brückengruppe Y für eine Gruppe der Formel IV.b, worin D für eine zweibindige Brückengruppe steht, die ausgewählt ist aus den Gruppen 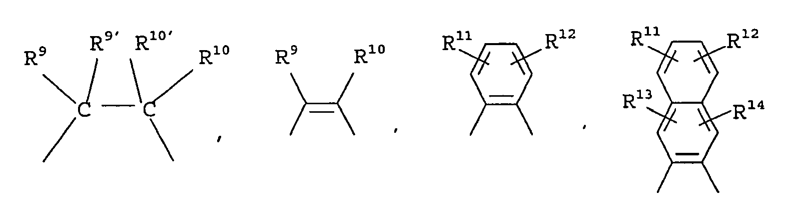 in denen R⁹, R^{9'}, R¹⁰ und R^{10'} unabhängig voneinander für Wasserstoff, Alkyl, Cycloalkyl, Aryl, Halogen, Trifluormethyl, Carboxyl, Carboxylat oder Cyano stehen oder miteinander zu einer C₃-C₄-Alkylengruppe verbunden sind und R¹¹, R¹², R¹³ und R¹⁴ unabhängig voneinander für Wasserstoff, Alkyl, Cycloalkyl, Aryl, Halogen, Trifluormethyl, COOH, Carboxylat, Cyano, Alkoxy, SO₃H, Sulfonat, NE¹E², Alkylen-NE¹E²E³⁺X⁻, Aryl oder Nitro stehen können. Vorzugsweise stehen die Gruppen R⁹, R^{9'}, R¹⁰ und R^{10'} für Wasserstoff, C₁-C₁₀-Alkyl oder Carboxylat und die Gruppen R¹¹, R¹², R¹³ und R¹⁴ für Wasserstoff, C₁-C₁₀-Alkyl, Halogen, insbesondere Fluor, Chlor oder Brom, Trifluormethyl, C₁-C₄-Alkoxy, Carboxylat, Sulfonat oder C₁-C₈-Aryl. Besonders bevorzugt stehen R⁹, R^{9'}, R¹⁰, R^{10'}, R¹¹, R¹², R¹³ und R¹⁴ für Wasserstoff. Für den Einsatz in einem wässrigen Reaktionsmedium sind solche Pnicogenchelatverbindungen bevorzugt, in denen 1, 2 oder 3, vorzugsweise 1 oder 2, insbesondere 1 der Gruppen R¹¹, R¹², R¹³ und/oder R¹⁴ für eine COO-M⁺, eine SO₃⁻M⁺ oder eine (NE¹E²E³)⁺X⁻-Gruppe stehen, wobei M⁺ und X⁻ die vorstehend genannte Bedeutung haben.

Besonders bevorzugte Brückengruppen D sind die Ethylengruppe 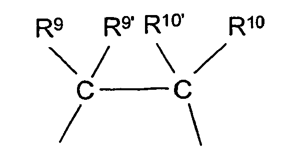 und die 1,2-Phenylengruppe

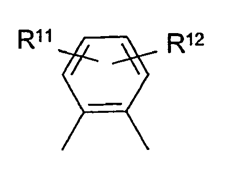

In den Brückengruppen Y der Formel IV.a und IV.b sind die Substituenten R^{I}, R^{II}, R^{III}, R^{IV}, R^{V} und R^{VI} vorzugsweise ausgewählt unter Wasserstoff, Alkyl, Alkoxy, Cycloalkyl, Heterocycloalkyl, Aryl und Hetaryl. Nach einer ersten bevorzugten Ausführungsform stehen R^{I}, R^{II}, R^{III}, R^{IV}, R^{V} und R^{VI} für Wasserstoff. Nach einer weiteren bevorzugten Ausführungsform stehen R^{I} und R^{VI} unabhängig voneinander für C₁-C₄-Alkyl oder C₁-C₄-Alkoxy. Vorzugsweise sind R¹ und R^{VI} ausgewählt unter Methyl, Ethyl, Isopropyl, tert.-Butyl und Methoxy. Bevorzugt stehen in diesen Verbindungen R^{II}, R^{III}, R^{IV} und R^{V} für Wasserstoff. Nach einer weiteren bevorzugten Ausführungsform stehen R^{II} und R^{V} unabhängig voneinander für C₁-C₄-Alkyl oder C₁-C₄-Alkoxy. Vorzugsweise sind R^{II} und R^{V} ausgewählt unter Methyl, Ethyl, Isopropyl und tert.-Butyl. Bevorzugt stehen in diesen Verbindungen R^{I}, R^{III}, R^{IV} und R^{VI} für Wasserstoff.

Wenn in den Brückengruppen Y der Formel IV.a und IV.b zwei benachbarte Reste, ausgewählt unter R^{I}, R^{II}, R^{III}, R^{IV}, R^{V} und R^{VI} für ein ankondensiertes, also anelliertes, Ringsystem stehen, so handelt es sich bevorzugt um Benzol- oder Naphthalinringe. Anellierte Benzolringe sind vorzugsweise unsubstituiert oder weisen 1, 2 oder 3, insbesondere 1 oder 2 Substituenten auf, die ausgewählt sind unter Alkyl, Alkoxy, Halogen, SO₃H Sulfonat, NE¹E², Alkylen-NE¹E², Trifluormethyl, Nitro, COOR^{f}, Alkoxycarbonyl, Acyl und Cyano. Anellierte Naphthalinringe sind vorzugsweise unsubstituiert oder weisen im nicht anellierten Ring und/oder im anellierten Ring insgesamt 1, 2 oder 3, insbesondere 1 oder 2 der zuvor bei den anellierten Benzolringen genannten Substituenten auf.

Bevorzugt steht Y für eine Gruppe der Formel IV.c, worin R^{IV} und R^{V} unabhängig voneinander für C₁-C₄-Alkyl oder C₁-C₄-Alkoxy stehen. Vorzugsweise sind R^{IV} und R^{V} ausgewählt unter Methyl, Ethyl, Isopropyl, tert.-Butyl und Methoxy. Bevorzugt stehen in diesen Verbindungen R^{I}, R^{II}, R^{III}, R^{VI}, R^{VII} und R^{VIII} für Wasserstoff.

Des Weiteren bevorzugt steht Y für eine Gruppe der Formel IV.c, worin R^{I} und R^{VIII} unabhängig voneinander für C₁-C₄-Alkyl oder C₁-C₄-Alkoxy stehen. Besonders bevorzugt stehen R¹ und R^{VIII} für tert.-Butyl. Besonders bevorzugt stehen in diesen Verbindungen R^{II}, R^{III}, R^{IV}, R^{V}, R^{VI}, RV^{II} für Wasserstoff. Des Weiteren bevorzugt stehen in diesen Verbindungen R^{III} und R^{VI} unabhängig voneinander für C₁-C₄-Alkyl oder C₁-C₄-Alkoxy. Besonders bevorzugt sind R^{III} und R^{VI} unabhängig voneinander ausgewählt unter Methyl, Ethyl, Isopropyl, tert.-Butyl und Methoxy.

Des Weiteren bevorzugt steht Y für eine Gruppe der Formel IV.c, worin R^{II} und R^{VII} für Wasserstoff stehen. Bevorzugt stehen in diesen Verbindungen R^{I}, R^{III}, R^{IV}, R^{V}, R^{VI} und R^{VIII} unabhängig voneinander für C₁-C₄-Alkyl oder C₁-C₄-Alkoxy. Besonders bevorzugt sind R^{I}, R^{III}, R^{IV}, R^{V}, R^{VI} und R^{VIII} unabhängig voneinander ausgewählt unter Methyl, Ethyl, Isopropyl, tert.-Butyl und Methoxy.

Weiterhin bevorzugt steht Y für eine Gruppe der Formel IV.d, worin Z für eine C₁-C₄-Alkylengruppe, insbesondere Methylen, steht. Bevorzugt stehen in diesen Verbindungen R^{IV} und R^{V} unabhängig voneinander für C₁-C₄-Alkyl oder C₁-C₄-Alkoxy. Besonders bevorzugt sind R^{IV} und R^{V} unabhängig voneinander ausgewählt unter Methyl, Ethyl, Isopropyl, tert.-Butyl und Methoxy. Die Reste R^{I}, R^{II}, R^{III}, R^{VI}, R^{VII} und R^{VIII} stehen vorzugsweise für Wasserstoff.

Des Weiteren bevorzugt steht Y für eine Gruppe der Formel IV.d, worin Z für eine C₁-C₄-Alkylenbrücke steht, die wenigstens einen Alkyl-, Cycloalkyl- oder Arylrest aufweist. Besonders bevorzugt steht Z für eine Methylenbrücke, die zwei C₁-C₄-Alkylreste, insbesondere zwei Methylreste, aufweist. Vorzugsweise stehen in diesen Verbindungen die Reste R¹ und R^{VIII} unabhängig voneinander für C₁-C₄-Alkyl oder C₁-C₄-Alkoxy. Besonders bevorzugt sind R^{I} und R^{VIII} unabhängig voneinander ausgewählt unter Methyl, Ethyl, Isopropyl, tert.-Butyl und Methoxy.

Weiterhin bevorzugt steht Y für eine Gruppe der Formel IV.e, worin R^{I} und R^{XII} unabhängig voneinander für C₁-C₄-Alkyl oder C₁-C₄-Alkoxy stehen. Insbesondere sind R^{I} und R^{XII} unabhängig voneinander ausgewählt unter Methyl, Ethyl, Isopropyl, tert.-Butyl, Methoxy und Alkoxycarbonyl, bevorzugt Methoxycarbonyl. Besonders bevorzugt stehen in diesen Verbindungen die Reste R^{II} bis R^{XI} für Wasserstoff.

Des Weiteren bevorzugt steht Y für eine Gruppe der Formel IV.f, worin R^{I} und R^{XII} unabhängig voneinander für C₁-C₄-Alkyl oder C₁-C₄-Alkoxy stehen. Insbesondere sind R^{I} und R^{XII} unabhängig voneinander ausgewählt unter Methyl, Ethyl, Isopropyl, tert.-Butyl und Methoxy. Besonders bevorzugt stehen in diesen Verbindungen die Reste R^{II} bis R^{XI} für Wasserstoff.

Weiterhin bevorzugt steht Y für eine Gruppe der Formel IV.g, worin Z für eine C₁-C₄-Alkylengruppe steht, die wenigstens einen Alkyl-, Cycloalkyl- oder Arylsubstituenten aufweist. Besonders bevorzugt steht Z für eine Methylengruppe, die zwei C₁-C₄-Alkylreste, speziell zwei Methylreste, aufweist. Besonders bevorzugt stehen in diesen Verbindungen die Reste R¹ und R^{VIII} unabhängig voneinander für C₁-C₄-Alkyl oder C₁-C₄-Alkoxy. Insbesondere sind R¹ und R^{VIII} unabhängig voneinander ausgewählt unter Methyl, Ethyl, Isopropyl, tert.-Butyl und Methoxy. Die Reste R^{II}, R^{III}, R^{IV}, R^{V}, R^{VI} und R^{VII} stehen vorzugsweise für Wasserstoff.

Des Weiteren bevorzugt steht Y für eine Gruppe der Formel IV.h, worin R^{I}, R^{I'}, R^{II}, R^{II'}, R^{III} und R^{III'} für Wasserstoff stehen.

Des Weiteren bevorzugt steht Yfür eine Gruppe der Formel IV.h worin R^{II} und R^{II} gemeinsam für eine Oxo-Gruppe oder ein Ketal davon stehen und die übrigen Reste für Wasserstoff stehen.

Des Weiteren bevorzugt steht Y für eine Gruppe der Formel IV.i, worin R^{I}, R^{I'}, R^{II}, R^{II'}, R^{III} und R^{III'} für Wasserstoff stehen.

Des Weiteren bevorzugt steht Y für eine Gruppe der Formel IV.i, worin R^{II} und R^{II'} gemeinsam für eine Oxo-Gruppe oder ein Ketal davon stehen und die übrigen Reste für Wasserstoff stehen.

Des Weiteren bevorzugt steht Y für eine Gruppe der Formel IV.k, worin R^{I}, R^{I'}, R^{II}, R^{II'}, R^{III}, R^{III'}, R^{IV} und R^{IV'} für Wasserstoff stehen.

Des Weiteren bevorzugt steht Y für eine Gruppe der Formel IV.I, worin R^{I}, R^{I'}, R^{II}, R^{II'}, R^{III}, R^{III'}, R^{IV} und R^{IV'} für Wasserstoff stehen.

Des Weiteren bevorzugt steht Y für eine Gruppe der Formel IV.m, worin R^{I}, R^{I'}, R^{II}, R^{II'}, R^{III}, R^{III'}, R^{IV} und R^{IV'} für Wasserstoff stehen.

Des Weiteren bevorzugt steht Y für eine Gruppe der Formel IV.n, worin R^{I}, R^{I'}, R^{II}, R^{II'}, R^{III}, R^{III'}, R^{IV} und R^{IV'} für Wasserstoff stehen.

Des Weiteren bevorzugt steht Y für eine Gruppe der Formel IV.o, worin R^{I}, R^{I'}, R^{II}, R^{II'}, R^{III}, R^{III'}, R^{IV} und R^{IV'} für Wasserstoff stehen.

Des Weiteren bevorzugt steht Y für eine Gruppe der Formel IV.o, worin einer der Reste R^{I} bis R^{IV} für C₁-C₄-Alkyl oder C₁-C₄-Alkoxy steht. Besonders bevorzugt steht dann wenigstens einer der Reste R^{I} bis R^{IV} für Methyl, Ethyl, Isopropyl, tert.-Butyl oder Methoxy.

Des Weiteren bevorzugt steht Y für eine Gruppe der Formel IV.p, worin R^{I}, R^{II}, R^{III} und R^{IV} für Wasserstoff stehen.

Des Weiteren bevorzugt steht Y für eine Gruppe der Formel IV.p, worin einer der Reste R^{I}, R^{II}, R^{III} oder R^{IV} für C₁-C₄-Alkyl oder C₁-C₄-Alkoxy steht. Besonders bevorzugt steht dann einer der Reste R^{I} bis R^{IV} für Methyl, Ethyl, tert.-Butyl oder Methoxy.

Des Weiteren bevorzugt steht Y für eine Gruppe der Formel IV.q, worin R^{I} und R^{VI} unabhängig voneinander für C₁-C₄-Alkyl oder C₁-C₄-Alkoxy stehen. Besonders bevorzugt sind R^{I} und R^{VI} unabhängig voneinander ausgewählt unter Methyl, Ethyl, Isopropyl, tert.-Butyl und Methoxy. Besonders bevorzugt stehen in diesen Verbindungen R^{II}, R^{III}, R^{IV} und R^{V} für Wasserstoff. Des Weiteren bevorzugt stehen in den Verbindungen IV.q R^{I}, R^{III}, R^{IV} und R^{VI} unabhängig voneinander für C₁-C₄-Alkyl oder C₁-C₄-Alkoxy. Besonders bevorzugt sind R^{I}, R^{III}, R^{IV} und K^{VI} dann unabhängig voneinander ausgewählt unter Methyl, Ethyl, Isopropyl, tert.-Butyl und Methoxy.

Des Weiteren bevorzugt steht Y für eine Gruppe der Formel IV.r, worin R^{I} und R^{VI} unabhängig voneinander für C₁-C₄-Alkyl oder C₁-C₄-Alkoxy stehen. Besonders bevorzugt sind R^{I} und R^{VI} unabhängig voneinander ausgewählt unter Methyl, Ethyl, Isopropyl, tert.-Butyl und Methoxy. Besonders bevorzugt stehen in diesen Verbindungen R^{II}, R^{III}, R^{IV} und R^{V} für Wasserstoff. Des Weiteren bevorzugt stehen in diesen Verbindungen R^{III} und R^{IV} unabhängig voneinander für C₁-C₄-Alkyl oder C₁-C₄-Alkoxy. Besonders bevorzugt sind R^{III} und R^{IV} dann unabhängig voneinander ausgewählt unter Methyl, Ethyl, Isopropyl, tert.-Butyl und Methoxy.

Des Weiteren bevorzugt steht Y für eine Gruppe der Formel IV.s, IV.t oder IV.u, worin Z für CH₂, C₂H₂ oder C₂H₄ steht.

In den Verbindungen der Formeln IV.s, IV.t und IV.u sind gleichermaßen für die dargestellten Bindungen zu den verbrückten Gruppen endo- und exo-Stellung möglich.

Die erfindungsgemäß eingesetzten Katalysatoren können noch wenigstens einen weiteren Liganden, der vorzugsweise ausgewählt ist unter Halogeniden, Aminen, Carboxylaten, Acetylacetonat, Aryl- oder Alkylsulfonaten, Hydrid, CO, Olefinen, Dienen, Cycloolefinen, Nitrilen, N-haltigen Heterocyclen, Aromaten und Heteroaromaten, Ethern, PF₃, Phospholen, Phosphabenzolen, ein-, zwei- und mehrzähnigen Phosphin-, Phosphinit-, Phosphonit-, Phosphitliganden und Mischungen davon, aufweisen.

Im Allgemeinen werden unter Hydroformylierungsbedingungen aus den jeweils eingesetzten Katalysatoren oder Katalysatorvorstufen katalytisch aktive Spezies der allgemeinen Formel HₜMᵤ(CO)ᵥL_{w} gebildet, worin M für ein Metall der VIII. Nebengruppe, L für eine Phosphoramiditverbindung und t, u, v, w für ganze Zahlen, abhängig von der Wertigkeit und Art des Metalls sowie der Bindigkeit des Liganden L, stehen. Vorzugsweise stehen v und w unabhängig voneinander mindestens für einen Wert von 1, wie z. B. 1, 2 oder 3. Die Summe aus v und w steht bevorzugt für einen Wert von 1 bis 5. Dabei können die Komplexe gewünschtenfalls zusätzlich noch mindestens einen der zuvor beschriebenen weiteren Liganden aufweisen.

Nach einer bevorzugten Ausführungsform werden die Hydroformylierungskatalysatoren in situ, in dem für die Hydroformylierungsreaktion eingesetzten Reaktor, hergestellt. Gewünschtenfalls können die erfindungsgemäßen Katalysatoren jedoch auch separat hergestellt und nach üblichen Verfahren isoliert werden. Zur in situ-Herstellung der erfindungsgemäßen Katalysatoren kann man z. B. wenigstens eine Phosphoramiditverbindung, eine Verbindung oder einen Komplex eines Metalls der VIII. Nebengruppe, gegebenenfalls wenigstens einen weiteren zusätzlichen Liganden und gegebenenfalls ein Aktivierungsmittel in einem inerten Lösungsmittel unter den Hydroformylierungsbedingungen umsetzen.

Geeignete Rhodiumverbindungen oder -komplexe sind z. B. Rhodium(II)- und Rhodium(III)-salze, wie Rhodium(III)-chlorid, Rhodium(III)-nitrat, Rhodium(III)-sulfat, Kalium-Rhodiumsulfat, Rhodium(II)- bzw. Rhodium(III)-carboxylat, Rhodium(II)- und Rhodium(III)-acetat, Rhodium(II)- und Rhodium(III)-ethylhexanoat, Rhodium(III)-oxid, Salze der Rhodium(III)-säure, Trisammonium-hexachlororhodat(III) etc. Weiterhin eignen sich Rhodiumkomplexe, wie Rhodiumbiscarbonylacetylacetonat, Acetylacetonatobisethylenrhodium(I) etc. Vorzugsweise werden Rhodiumbiscarbonylacetylacetonat oder Rhodiumacetat eingesetzt.

Ebenfalls geeignet sind Rutheniumsalze oder -verbindungen. Geeignete Rutheniumsalze sind beispielsweise Ruthenium(III)chlorid, Ruthenium(IV)-, Ruthenium(VI)- oder Ruthenium(VIII)oxid, Alkalisalze der Rutheniumsauerstoffsäuren wie K₂RuO₄ oder KRuO₄ oder Komplexverbindungen, wie z. B. RuHCl(CO)(PPh₃)₃. Auch können die Metallcarbonyle des Rutheniums wie Trisrutheniumdodecacarbonyl oder Hexarutheniumoctadecacarbonyl, oder Mischformen, in denen CO teilweise durch Liganden der Formel PR₃ ersetzt sind, wie Ru(CO)₃(PPh₃)₂, im erfindungsgemäßen Verfahren verwendet werden.

Geeignete Cobaltverbindungen sind beispielsweise Cobalt(II)chlorid, Cobalt(II)sulfat, Cobalt(II)carbonat, Cobalt(II)nitrat, deren Amin- oder Hydratkomplexe, Cobaltcarboxylate, wie Cobaltacetat, Cobaltethylhexanoat, Cobaltnaphthanoat, sowie der Cobalt-Caproat-Komplex. Auch hier können die Carbonylkomplexe des Cobalts wie Dicobaltoctacarbonyl, Tetracobaltdodecacarbonyl und Hexacobalthexadecacarbonyl eingesetzt werden.

Die genannten und weitere geeignete Verbindungen des Cobalts, Rhodiums, Rutheniums und Iridiums sind im Prinzip bekannt und in der Literatur hinreichend beschrieben oder sie können vom Fachmann analog zu den bereits bekannten Verbindungen hergestellt werden.

Geeignete Aktivierungsmittel sind z. B. Brönsted-Säuren, Lewis-Säuren, wie z.B. BF₃, AlCl₃, ZnCl₂, SnCl₂ und Lewis-Basen.

Geeignete Olefin-Einsatzmaterialien für das erfindungsgemäße Verfahren sind prinzipiell alle Verbindungen, welche eine oder mehrere ethylenisch ungesättigte Doppelbindungen enthalten. Dazu zählen Olefine mit endständigen und mit innenständigen Doppelbindungen, geradkettige und verzweigte Olefine, cyclische Olefine sowie Olefine, die unter den Hydroformylierungsbedingungen im Wesentlichen inerte Substituenten aufweisen. Bevorzugt sind Olefin-Einsatzmaterialien, die Olefine mit 4 bis 12, besonders bevorzugt mit 4 bis 6 Kohlenstoffatomen enthalten. Bevorzugt sind die zur Hydroformylierung eingesetzten Olefine ausgewählt unter linearen (geradkettigen) Olefinen und Olefingemischen, die wenigstens ein lineares Olefin enthalten. Mit dem erfindungsgemäßen Verfahren können insbesondere lineare α-Olefine, lineare interne Olefine und Gemische aus linearen α-Olefinen und linearen internen Olefinen hydroformyliert werden.

Als Substrate für das erfindungsgemäße Hydroformylierungsverfahren geeignete α-Olefine sind vorzugsweise C₄-C₂₀-α-Olefine, z. B. 1-Buten, Isobuten, 1-Penten, 2-Methyl-1-buten, 1-Hexen, 1-Hepten, 1-Octen, 1-Nonen, 1-Decen, 1-Undecen, 1-Dodecen, Allylalkohole etc.. Bevorzugt sind lineare α-Olefine und Olefingemische, die wenigstens ein lineares α-Olefin enthalten.

Bevorzugt ist die zur Hydroformylierung eingesetzte ungesättigte Verbindung ausgewählt unter internen linearen Olefinen und Olefingemischen, die wenigstens ein internes lineares Olefin enthalten. Geeignete lineare interne Olefine sind vorzugsweise C₄-C₂₀-Olefine, wie 2-Buten, 2-Penten, 2-Hexen, 3-Hexen, 2-Hepten, 3-Hepten, 2-Octen, 3-Octen, 4-Octen etc. und Mischungen davon.

Geeignete verzweigte, interne Olefine sind vorzugsweise C₄-C₂₀-Olefine, wie 2-Methyl-2-buten, 2-Methyl-2-penten, 3-Methyl-2-penten, verzweigte, interne Hepten-Gemische, verzweigte, interne Octen-Gemische, verzweigte, interne Nonen-Gemische, verzweigte, interne Decen-Gemische, verzweigte, interne Undecen-Gemische, verzweigte, interne Dodecen-Gemische etc..

Geeignete zu hydroformylierende Olefine sind weiterhin C₅-C₈-Cycloalkene, wie Cyclopenten, Cyclohexen, Cyclohepten, Cycloocten und deren Derivate, wie z. B. deren C₁-C₂₀-Alkylderivate mit 1 bis 5 Alkylsubstituenten. Geeignete zu hydroformylierende Olefine sind weiterhin Vinylaromaten, wie Styrol, α-Methylstyrol, 4-Isobutylstyrol etc. Geeignete zu hydroformylierende Olefine sind weiterhin α,β-ethylenisch ungesättigte Mono- und/oder Dicarbonsäuren, deren Ester, Halbester und Amide, wie Acrylsäure, Methacrylsäure, Maleinsäure, Fumarsäure, Crotonsäure, Itaconsäure, 3-Pentensäuremethylester, 4-Pentensäuremethylester, Ölsäuremethylester, Acrylsäuremethylester und Methacrylsäuremethylester. Geeignete zu hydroformylierende Olefine sind weiterhin ungesättigte Nitrile, wie 3-Pentennitril, 4-Pentennitril und Acrylnitril. Weiterhin geeignet sind Vinylether, wie Vinylmethylether, Vinylethylether, Vinylpropylether etc.. Geeignete zu hydroformylierende Olefine sind weiterhin Alkenole, Alkendiole und Alkadienole, wie 2,7-Octadienol-1. Geeignete zu hydroformylierende Olefine sind weiterhin Di- oder Polyene mit isolierten oder konjugierten Doppelbindungen. Dazu zählen z. B. 1,3-Butadien, 1,4-Pentadien, 1,5-Hexadien, 1,6-Heptadien, 1,7-Octadien, 1,9-Decadien, Vinylcyclohexen, Dicyclopentadien, 1,5,9-Cyclooctatrien, Butadienhomo- und -copolymere sowie Olefine mit terminalen und internen Doppelbindungen, wie z. B. 1,4-Octadien.

Vorzugsweise wird in dem erfindungsgemäßen Hydroformylierungsverfahren ein technisch zur Verfügung stehendes olefinhaltiges Kohlenwasserstoffgemisch eingesetzt.

Bevorzugte großtechnisch zur Verfügung stehende Olefingemische resultieren aus der Kohlenwasserstoff-Spaltung bei der Erdölverarbeitung, beispielsweise durch Katcracken, wie Fluid Catalytic Cracking (FCC), Thermocracken oder Hydrocracken mit anschließender Dehydrierung. Ein geeignetes technisches Olefingemisch ist der C₄-Schnitt. C₄-Schnitte sind beispielsweise durch Fluid Catalytic Cracking oder Steamcracken von Gasöl bzw. durch Steamcracken von Naphtha erhältlich. Je nach Zusammensetzung des C₄-Schnitts unterscheidet man den Gesamt-C₄-Schnitt (Roh-C₄-Schnitt), das nach der Abtrennung von 1,3-Butadien erhaltene so genannte Raffinat I sowie das nach der Isobutenabtrennung erhaltene Raffinat II. Ein weiteres geeignetes technisches Olefingemisch ist der bei der Naphtha-Spaltung erhältliche C₅-Schnitt. Für den Einsatz in Schritt a) geeignete olefinhaltige Kohlenwasserstoffgemische mit 4 bis 6 Kohlenstoffatomen lassen sich weiterhin durch katalytische Dehydrierung geeigneter großtechnisch zur Verfügung stehender Paraffingemische erhalten. So gelingt beispielsweise die Herstellung von C₄-Olefin-Gemischen aus Flüssiggasen (liquified petroleum gas, LPG) und verflüssigbaren Erdgasen (liquified natural gas, LNG). Letztere umfassen neben der LPG-Fraktion auch zusätzlich größere Mengen höhermolekularer Kohlenwasserstoffe (leichtes Naphtha) und eignen sich somit auch zur Herstellung von C₅- und C₆-Olefin-Gemischen. Die Herstellung von olefinhaltigen Kohlenwasserstoffgemischen, die Monoolefine mit 4 bis 6 Kohlenstoffatomen enthalten, aus LPG- oder LNG-Strömen gelingt nach üblichen, dem Fachmann bekannten Verfahren, die neben der Dehydrierung in der Regel noch einen oder mehrere Aufarbeitungsschritte umfassen. Dazu zählt beispielsweise die Abtrennung wenigstens eines Teils der in den zuvor genannten Olefin-Einsatzgemischen enthaltenen gesättigten Kohlenwasserstoffe. Diese können beispielsweise erneut zur Herstellung von Olefin-Einsatzmaterialien durch Crackung und/oder Dehydrierung eingesetzt werden. Die in dem erfindungsgemäßen Verfahren eingesetzten Olefine können jedoch auch einen Anteil gesättigter Kohlenwasserstoffe enthalten, die sich gegenüber den erfindungsgemäßen Hydroformylierungsbedingungen inert verhalten. Der Anteil dieser gesättigten Komponenten beträgt im Allgemeinen höchstens 60 Gew.-%, bevorzugt höchstens 40 Gew.-%, besonders bevorzugt höchstens 20 Gew.-%, bezogen auf die Gesamtmenge der in dem Kohlenwasserstoff-Einsatzmaterial enthaltenen Olefine und gesättigten Kohlenwasserstoffe.

Ein zum Einsatz in dem erfindungsgemäßen Verfahren geeignetes Raffinat II hat beispielsweise die folgende Zusammensetzung:
0,5 bis 5 Gew.-% Isobutan,
5 bis 20 Gew.-% n-Butan,
20 bis 40 Gew.-% trans-2-Buten,
10 bis 20 Gew.-% cis-2-Buten,
25 bis 55 Gew.-% 1-Buten,
0,5 bis 5 Gew.-% Isobuten
sowie Spurengase, wie 1,3-Butadien, Propen, Propan, Cyclopropan, Propadien, Methylcyclopropan, Vinylacetylen, Pentene, Pentane etc. im Bereich von jeweils maximal 1 Gew.%.

Überraschenderweise wurde gefunden, dass sich katalytisch aktive Fluids auf Basis von Metallkomplexen von Phosphoramiditverbindungen durch Inkontaktbringen mit einer Base zusätzlich stabilisieren lassen. In dem erfindungsgemäßen Verfahren werden somit längere Katalysatorstandzeiten erzielt als mit aus dem Stand der Technik bekannten Hydroformylierungsverfahren unter Einsatz von Katalysatoren sowohl auf Basis herkömmlicher ein- und mehrzähniger Liganden als insbesondere auf Basis von Phosphoramiditliganden. Dabei wird die katalytische Aktivität durch das Inkontaktbringen mit der Base im Allgemeinen nicht beeinträchtigt.

Die Erfindung wird anhand der folgenden nicht einschränkenden Beispiele näher erläutert.

### Beispiele

### 1. Herstellung der Verbindung (1)

28,5 g (218 mmol) 3-Methylindol (Skatol) wurden in ca. 50 ml getrocknetem Toluol vorgelegt und das Lösungsmittel im Vakuum abdestilliert, um Wasserspuren azeotrop zu entfernen. Dieser Vorgang wurde noch einmal wiederholt. Der Rückstand wurde anschließend in 700 ml getrocknetem Toluol unter Argon aufgenommen und auf -65 °C abgekühlt. Dann wurden 14,9 g (109 mmol) PCl₃ und danach 40 g (396 mmol) Triethylamin bei -65 °C langsam zugegeben. Das Reaktionsgemisch wurde innerhalb von 16 h auf Raumtemperatur gebracht und danach 16 h unter Rückfluss erhitzt. Zum Reaktionsgemisch wurden 19,3 g (58 mmol) 4,5-Dihydroxy-2,7-di-tert-butyl-9,9-dimethylxanthen in 300 ml getrocknetem Toluol gegeben, danach 16 Stunden unter Rückfluss erhitzt und nach Abkühlen auf Raumtemperatur der ausgefallene farblose Feststoff (Triethylamin-Hydrochlorid) abgesaugt, das Lösungsmittel abdestilliert und der Rückstand zweimal aus heißem Ethanol umkristallisiert. Nach Trocknung im Vakuum wurden 36,3 g (71 % der Theorie) eines farblosen Feststoffes erhalten. ³¹P-NMR (298K): δ = 105 ppm.

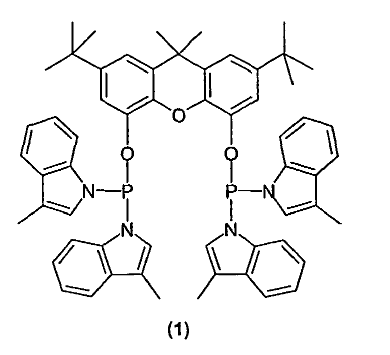

### 2. Hydroformylierung von trans 2-Buten ohne Zusatz (Vergleichsbeispiel)

0,005 g Rh(CO)₂(acac) und 0,181 g der Verbindung (1) wurden unter Schutzgasatmosphäre in 10,17 g Xylol gelöst und der Ansatz in einen 100 ml Stahlautoklaven überführt. Der Autoklav wurde mit 10 bar Synthesegas (CO/H₂ = 1:2) beaufschlagt und dann innerhalb von einer Stunde auf 90 °C aufgeheizt. Anschließend wurde der Autoklav bei 90 °C vorsichtig auf 7 bar entspannt, und über eine Schleuse wurden mittels Synthesegas der zuvor genannten Zusammensetzung (p = 12 bar) 10,81 g einer Flüssiggasmischung (30 vol% trans 2-Buten und 70 vol-% Isobutan) zugepresst. Dann wurde der Druck mit dem Synthesegas auf 16 bar (gesamt) eingestellt. Während der Reaktionszeit von 4 h wurde die Temperatur bei 90 °C und der Druck durch Zugabe von CO/H₂ (1:1) bei 16 bar (gesamt) gehalten. Nach dem Ende der Reaktion wurde der Autoklav über eine Kühlfalle entspannt und der Inhalt des Autoklaven und der Kühlfalle gaschromatographisch analysiert, um den Umsatz, die Ausbeute an Pentanalen und den Anteil des n-Valeraldehyds an den Pentanalen zu bestimmen.

Ergebnisse der gaschromatographischen Analyse:

| | |
|---|---|
| Umsatz | 32 % |
| Ausbeute | 31% |
| n-Anteil | 93 % |

### 3. Abbauversuch unter Zusatz von N,N-Dimethylanilin

0,005 g (Rh(CO)₂(acac), 0,181 g Verbindung (1) und 0,26 g N,N-Dimethylanilin wurden unter Schutzgasatmosphäre in 8,12 g Texanol® (2,2,4-Trimethyl-1,3-pentandiolmonobutyrat, Fa. Eastman) gelöst und der Ansatz in einen 60 ml Stahlautoklaven überführt. Auf den Autoklaven wurden bei 25 °C 20 bar CO/H₂ (1:1) aufgepresst und dieser in 60 min auf 120 °C erwärmt. Anschließend wurde der Autoklav bei 120 °C vorsichtig auf 7 bar entspannt und über eine Schleuse 11,23 g einer Flüssiggasmischung (2,9 vol-% Isobutan; 14,6 vol% n-Butan; 27,4 vol% trans 2-Buten; 37,4 vol-% 1-Buten; 2,6 vol% Isobuten; 15,3 % cis 2-Buten) mit 12 bar CO/H₂ (1:1) zugepresst. Der Druck wurde mit CO/H₂ (1:1) auf 28 bar (gesamt) erhöht und der Autoklav bei 120 °C 24 h betrieben. Nach dem Ende der Reaktionszeit wurde der Autoklav abgekühlt, entspannt und unter Schutzgasatmosphäre eine Probe für eine ³¹P-NMR Analyse genommen, um festzustellen, wie stark der Ligand abgebaut wurde.

Die Auswertung der ³¹P-NMR Analyse über Integrale ergab, dass 18 % der Verbindung (1) abgebaut wurden.

Anschließend wurde der Ansatz wieder in den Autoklaven gefüllt, dreimal mit Stickstoff gespült und der Autoklav zur Simulierung einer Dauerbelastung des Katalysators, wie sie bei längerem kontinuierlichen Betrieb auftritt, 24 h bei 120 °C und 3 bar Stickstoffdruck betrieben. Nach dem Ende der Reaktionszeit wurde der Autoklav abgekühlt, entspannt und unter Schutzgasatmosphäre eine Probe für eine ³¹P-NMR Analyse genommen, um festzustellen, wie stark der Ligand abgebaut wurde.

Die Auswertung der ³¹P-NMR Analyse über Integrale ergab, dass insgesamt nur 42 % der Verbindung (1) abgebaut wurden.

### 4. Hydroformylierung von trans 2-Buten unter Zusatz von N,N-Dimethylanilin

0,005 g Rh(CO)₂(acac), 0,181 g der Verbindung (1) und 0,025 g N,N-Dimethylanilin wurden unter Schutzgasatmosphäre in 10,17 g Xylol gelöst und der Ansatz in einen 100 ml Stahlautoklaven überführt. Auf den Autoklav wurden 10 bar CO/H₂ (1:2) aufgepresst und dieser dann innerhalb 1 h auf 90 °C aufgeheizt. Anschließend wurde der Autoklav bei 90 °C vorsichtig auf 7 bar entspannt und aus einer Schleuse 10,81 g einer Flüssiggasmischung (30 vol% trans 2-Buten und 70 vol-% Isobutan) mit 12 bar CO/H₂ (1:2) zugepresst und der Druck mit CO/H₂ (1:2) auf 16 bar (gesamt) eingestellt. Während der Reaktionszeit von 4 h wurde die Temperatur bei 90 °C und der Druck mit CO/H₂ (1:1) bei 16 bar (gesamt) gehalten. Nach dem Ende der Reaktion wurde der Autoklav über eine Kühlfalle entspannt und der Inhalt des Autoklaven und der Kühlfalle gaschromatographisch analysiert, um den Umsatz, die Ausbeute an Pentanalen und den Anteil von n-Valeraldehyd an den Pentanalen zu bestimmen.

Ergebnisse der gaschromatographischen Analyse:

| | |
|---|---|
| Umsatz | 30 % |
| Ausbeute | 28 % |
| n-Anteil | 94 % |

Umsatz, Ausbeute und n-Anteil wurden durch den Zusatz der Base gegenüber Vergleichsbeispiel 2 nicht signifikant verringert.

### 5. Abbauversuch unter Zusatz von N,N,2,4,6-Pentamethylanilin

0,005 g (Rh(CO)₂(acac), 0,181 g Verbindung (1) und 0,35 g N,N,2,4,6-Pentamethylanilin wurden unter Schutzgasatmosphäre in 8,11 g Texanol gelöst und der Ansatz in einen 60 ml Stahlautoklaven überführt. Auf den Autoklaven wurden bei 25 °C 20 bar CO/H₂ (1:1) aufgepresst und dieser dann innerhalb von 60 min auf 120 °C erwärmt. Anschließend wurde der Autoklav bei 120 °C vorsichtig auf 7 bar entspannt und aus einer Schleuse 11,23 g einer Flüssiggasmischung (2,9 vol% Isobutan; 14,6 vol% n-Butan; 27,4 vol-% trans 2-Buten; 37,4 vol% 1-Buten; 2,6 vol% Isobuten; 15,3 % cis 2-Buten) mit 12 bar CO/H₂ (1:1 ) zugepresst. Der Druck wurde mit CO/H₂ (1:1) auf 28 bar (gesamt) erhöht und der Autoklav bei 120 °C 24 h betrieben.

Nach dem Ende der Reaktionszeit wurde der Autoklav abgekühlt, entspannt und unter Schutzgasatmosphäre eine Probe für eine ³¹P-NMR Analyse genommen, um festzustellen, wie stark der Ligand abgebaut wurde.

Die Auswertung der ³¹P-NMR Analyse über Integrale ergab, dass 4 % der Verbindung (1) abgebaut wurden.

Anschließend wurde der Ansatz wieder in den Autoklaven gefüllt, dreimal mit Stickstoff gespült und der Autoklav 24 h bei 120 °C und 3 bar Stickstoffdruck betrieben. Nach dem Ende der Reaktionszeit wurde der Autoklav abgekühlt, entspannt und unter Schutzgasatmosphäre eine Probe für eine ³¹P-NMR Analyse genommen, um festzustellen, wie stark der Ligand abgebaut wurde.

Die Auswertung der ³¹P-NMR Analyse über Integrale ergab, dass insgesamt 23 % der Verbindung (1) abgebaut wurden.

### 6. Hydroformylierung von trans 2-Buten unter Zusatz von N,N,2,4,6-Pentamethylanilin

0,005 g Rh(CO)₂(acac), 0,181 g der Verbindung (1) und 0,035 g N,N,2,4,6-Pentamethylanilin wurden unter Schutzgasatmosphäre in 10,26 g Xylol gelöst und der Ansatz in einen 100 ml Stahlautoklaven überführt. Auf den Autoklav wurden 10 bar CO/H₂ (1:2) aufgepresst und dieser dann innerhalb von 1 h auf 90 °C aufgeheizt. Anschließend wurde der Autoklav bei 90 °C vorsichtig auf 7 bar entspannt und aus einer Schleuse 10,81 g einer Flüssiggasmischung (30 vol-% trans 2-Buten und 70 vol-% Isobutan) mit 12 bar CO/H₂ (1:2) zugepresst und der Druck mit CO/H₂ (1:2) auf 16 bar (gesamt) eingestellt. Während der Reaktionszeit von 4 h wurde die Temperatur bei 90 °C und der Druck mit CO/H₂ (1:1) bei 16 bar (gesamt) gehalten. Nach dem Ende der Reaktion wurde der Autoklav über eine Kühlfalle entspannt und der Inhalt des Autoklaven und der Kühlfalle gaschromatographisch analysiert, um den Umsatz, die Ausbeute an Pentanalen und den Anteil von n-Valeraldehyd an den Pentanalen zu bestimmen.

Ergebnisse der gaschromatographischen Analyse:

| | |
|---|---|
| Umsatz | 29 % |
| Ausbeute | 28 % |
| n-Anteil | 93 % |

### 7. Hydroformylierung von trans 2-Buten unter Zusatz von 3-Methylindol

0,005 g Rh(CO)₂(acac), 0,180 g der Verbindung (1) und 0,10 g 3-Methylindol wurden unter Schutzgasatmosphäre in 10,14 g Xylol gelöst und der Ansatz in einen 100 ml Stahlautoklaven überführt. Auf den Autoklav wurden 10 bar CO/H₂ (1:2) aufgepresst und dieser dann innerhalb von 1 h auf 90 °C aufgeheizt. Anschließend wurde der Autoklav bei 90 °C vorsichtig auf 7 bar entspannt und aus einer Schleuse 10,81 g einer Flüssiggasmischung (30 vol% trans 2-Buten und 70 vol-% Isobutan) mit 12 bar CO/H₂ (1:2) zugepresst und der Druck mit CO/H₂ (1:2) auf 16 bar (gesamt) eingestellt. Während der Reaktionszeit von 4 h wurde die Temperatur bei 90 °C und der Druck mit CO/H₂ (1:1) bei 16 bar (gesamt) gehalten. Nach dem Ende der Reaktion wurde der Autoklav über eine Kühlfalle entspannt und der Inhalt des Autoklaven und der Kühlfalle gaschromatographisch analysiert, um den Umsatz, die Ausbeute an Pentanalen und den Anteil von n-Valeraldehyd an den Pentanalen zu bestimmen.

Ergebnisse der gaschromatographischen Analyse:

| | |
|---|---|
| Umsatz | 33 % |
| Ausbeute | 32 % |
| n-Anteil | 94 % |

### 8. Hydroformylierung von trans 2-Buten unter Zusatz von Chinolin

0,005 g Rh(CO)₂(acac), 0,181 g der Verbindung (1) und 0,029 g Chinolin wurden unter Schutzgasatmosphäre in 10,16 g Xylol gelöst und der Ansatz in einen 100 ml Stahlautoklaven überführt. Auf den Autoklav wurden 10 bar CO/H₂ (1:2) aufgepresst und dieser dann 1 h auf 90 °C aufgeheizt. Anschließend wurde der Autoklav bei 90 °C vorsichtig auf 7 bar entspannt und über eine Schleuse 10,81 g einer Flüssiggasmischung (30 vol% trans 2-Buten und 70 vol-% Isobutan) mit 12 bar CO/H₂ (1:2) zugepresst und der Druck durch Zugabe von CO/H₂ (1:2) auf 16 bar (gesamt) eingestellt. Während der Reaktionszeit von 4 h wurde die Temperatur bei 90 °C und der Druck durch Zugabe von CO/H₂ (1:1) bei 16 bar (gesamt) gehalten. Nach dem Ende der Reaktion wurde der Autoklav über eine Kühlfalle entspannt und der Inhalt des Autoklaven und der Kühlfalle gaschromatographisch analysiert, um den Umsatz, die Ausbeute an Pentanalen und den Anteil von n-Valeraldehyd an den Pentanalen zu bestimmen.

Ergebnisse der gaschromatographischen Analyse:

| | |
|---|---|
| Umsatz | 29 % |
| Ausbeute | 27 % |
| n-Anteil | 90 % |

### 9. Hydroformylierung von Raffinat II ohne Zusatz (Vergleichsbeispiel)

0,006 g Rh(CO)₂(acac) und 0,217 g der Verbindung (1) wurden unter Schutzgasatmosphäre in 10,0 g Toluol gelöst und der Ansatz in einen 100 ml Stahlautoklaven überführt. Auf den Autoklav wurden 10 bar CO/H₂ (1:2) aufgepresst und dieser in 0,5 h auf 90 °C aufgeheizt. Anschließend wurde der Autoklav bei 90 °C vorsichtig auf 7 bar entspannt und über eine Schleuse 10,2 g einer Flüssiggasmischung (1,7 % iso-Butan, 12,4 % n-Butan, 31,7 % trans-2-Buten, 35,1 % 1-Buten, 2,4 % iso-Buten, 16,8 % cis-2-Buten) mit 12 bar CO/H₂ (1:2) zugepresst und der Druck mit CO/H₂ (1:2) auf 17 bar (gesamt) eingestellt. Während der Reaktionszeit von 4 h wurde die Temperatur bei 90 °C und der Druck mit CO/H₂ (1:1) bei 17 bar (gesamt) gehalten. Nach dem Ende der Reaktion wurde der Autoklav über eine Kühlfalle entspannt und der Inhalt des Autoklaven und der Kühlfalle gaschromatographisch analysiert, um den Umsatz, die Ausbeute an Pentanalen und den Anteil von n-Valeraldehyd an den Pentanalen zu bestimmen.

Ergebnisse der gaschromatographischen Analyse:

| | |
|---|---|
| Umsatz | 89 % |
| Ausbeute | 88 % |
| n-Anteil | 95 % |

### 10. Hydroformylierung von Raffinat II mit Zusatz von 1-H-Benztriazol

0,006 g Rh(CO)₂(acac) und 0,212 g der Verbindung (1) und 0,014 g 1-H-Benztriazol wurden unter Schutzgasatmosphäre in 10,1 g Toluol gelöst und der Ansatz in einen 100 ml Stahlautoklaven überführt. Auf den Autoklav wurden 10 bar CO/H₂ (1:2) aufgepresst und dieser in 0,5 h auf 90 °C aufgeheizt. Anschließend wurde der Autoklav bei 90 °C vorsichtig auf 7 bar entspannt und aus einer Schleuse 10,4 g einer Flüssiggasmischung (1,7 % iso-Butan, 12,4 % n-Butan, 31,7 % trans-2-Buten, 35,1 % 1-Buten, 2,4 % iso-Buten, 16,8 % cis-2-Buten) mit 12 bar CO/H₂ (1:2) zugepresst und der Druck mit CO/H₂ (1:2) auf 17 bar (gesamt) eingestellt. Während der Reaktionszeit von 4 h wurde die Temperatur bei 90 °C und der Druck mit CO/H₂ (1:1) bei 17 bar (gesamt) gehalten. Nach dem Ende der Reaktion wurde der Autoklav über eine Kühlfalle entspannt und der Inhalt des Autoklaven und der Kühlfalle gaschromatographisch analysiert, um den Umsatz, die Ausbeute an Pentanalen und den Anteil von n-Valeraldehyd an den Pentanalen zu bestimmen.

Ergebnisse der gaschromatographischen Analyse:

| | |
|---|---|
| Umsatz | 88 % |
| Ausbeute | 87 % |
| n-Anteil | 95 % |

(keine signifikante Veränderung gegenüber Vergleichsbeispiel 9)

### 11. Abbauversuch unter Zusatz von 1-H-Benztriazol

0,005 g (Rh(CO)₂(acac), 0,181 g Verbindung (1) und 0,024 g 1-H-Benztriazol wurden unter Schutzgasatmosphäre in 8,02 g Texanol® gelöst und der Ansatz in einen 60 ml Stahlautoklaven überführt. Auf den Autoklaven wurden bei 25 °C 20 bar CO/H₂ (1:1) aufgepresst und in dieser dann 60 min auf 120 °C erwärmt. Anschließend wurde der Autoklav bei 120 °C vorsichtig auf 7 bar entspannt und aus einer Schleuse 11,23 g einer Flüssiggasmischung (2,9 vol% Isobutan; 14,6 vol% n-Butan; 27,4 vol-% trans 2-Buten; 37,4 vol-% 1-Buten; 2,6 vol-% Isobuten; 15,3 % cis 2-Buten) mit 12 bar CO/H₂ (1:1) zugepresst. Der Druck wurde mit CO/H₂ (1:1) auf 28 bar (gesamt) erhöht und der Autoklav 24 h bei 120 °C gehalten. Nach dem Ende der Reaktionszeit wurde der Autoklav abgekühlt, entspannt und unter Schutzgasatmosphäre eine Probe für eine ³¹P-NMR Analyse genommen, um festzustellen, wie stark der Ligand abgebaut wurde.

Die Auswertung der ³¹P-NMR Analyse über Integrale ergab, dass 3 % der Verbindung (1) abgebaut wurden.

Anschließend wurde der Ansatz wieder in den Autoklaven gefüllt, dreimal mit Stickstoff gespült und der Autoklav 24 h bei 120 °C und 3 bar Stickstoffdruck betrieben. Nach dem Ende der Reaktionszeit wurde der Autoklav abgekühlt, entspannt und unter Schutzgasatmosphäre eine Probe für eine ³¹P-NMR Analyse genommen, um festzustellen, wie stark der Ligand abgebaut wurde.

Die Auswertung der ³¹P-NMR Analyse über Integrale ergab, dass insgesamt 29 % der Verbindung (1) abgebaut wurden.

### 12. Hydroformylierung von Raffinat II und Behandlung des Reaktionsaustrags mit einem Ionenaustauscher

0,0051 g Rh(CO)₂(acac) (acac = Acetylacetonat) und 0,1806 g Ligand (1) wurden unter N₂ in 8,05 g Toluol gelöst. Diese Lösung wurde mit ³¹P-NMR analysiert (siehe Tabelle 1; Nullprobe) und der Ansatz in einen 100 ml Stahlautoklaven überführt. Auf den Autoklaven wurden bei 25 °C 20 bar CO/H₂ (1:1) aufgepresst, der Autoklav auf 120 °C erwärmt und 30 min bei dieser Temperatur gehalten. Anschließend wurde der Autoklav auf 7 bar entspannt und über eine Schleuse mit 12 bar CO/H₂ (1:1) 11,37 g Flüssiggasmischung zugepresst.

Die Flüssiggasmischung hatte folgende Zusammensetzung (in Gew.-%):

| | |
|---|---|
| iso-Butan | 2,9 % |
| n-Butan | 14,6 % |
| trans 2-Buten | 27,4 % |
| 1-Buten | 37,4% |
| iso-Buten | 2,6 % |
| cis 2-Buten | 15,3% |

Der Druck im Autoklaven wurde mit CO/H₂ (1:1) auf 28 bar Gesamtdruck eingestellt und der Autoklav 24 h bei diesen Bedingungen betrieben. Anschließend wurde der Autoklav abgekühlt, entspannt und eine Probe des Reaktorinhaltes mit ³¹P-NMR analysiert (siehe Tabelle 1). Es wurden 21,8 g gelbe, homogene Lösung erhalten.

Der Austrag wurde bei 25 °C mit 2 g Amberlite® IRA 67 30 min unter N₂ gerührt.

Anschließend wurde eine Probe des flüssigen Reaktionsgemisches mit ³¹P-NMR analysiert (siehe Tabelle 1).

**Tabelle 1:**

| Ergebnisse der ³¹P-NMR Analyse: | | | | |
|---|---|---|---|---|
| quantitative ³¹P-NMR Analyse | | | | |
| Auswertung der Integrale in Flächen-% | | | | |
| Probe | Ligand (1) | Oxid (2) | Oxid (3) | Abbauprodukte |
| Nullprobe | 98,6 | | 1,4 | |
| nach Hydroformylierung | 25,3 | 7,1 | 1,5 | 66,1 |
| nach lonentauscherbehandlung | 37,3 | 10,2 | 3,1 | 49,4 |

Die Oxidation ist bedingt durch die Probenentnahme. Die Oxide sind dem Liganden zuzurechnen:

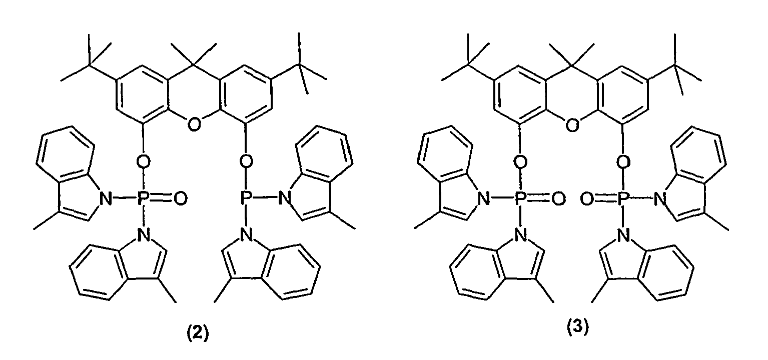

### 13. Kontinuierliche Hydroformylierung ohne Stabilisierung (Vergleichsbeispiel)

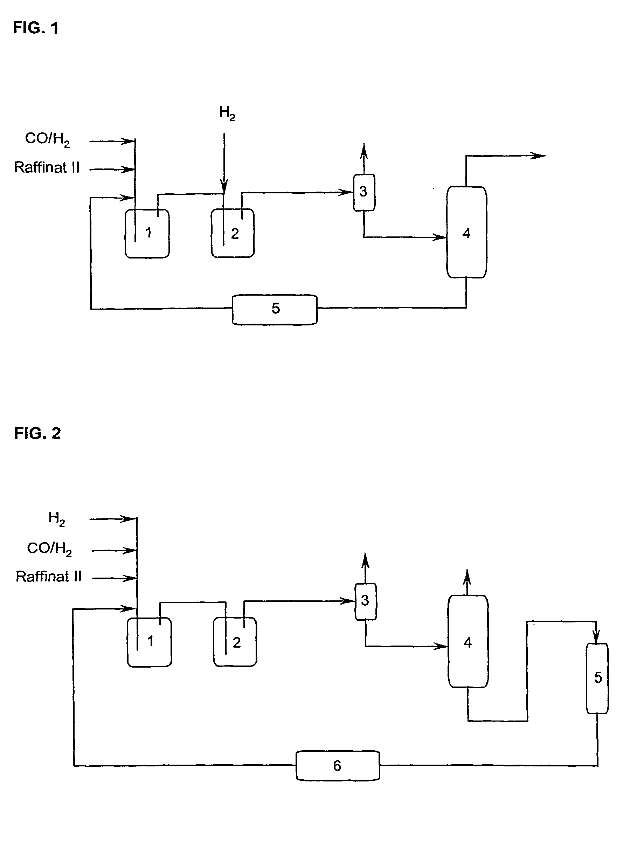

Fig. 1 zeigt eine so genannte Miniplant zur Durchführung kontinuierlicher Hydroformylierungen. Diese besteht aus zwei in Reihe geschalteten Hubrührautoklaven (1 und 2) mit 0,4 l (Reaktor 1) bzw. 1,9 l (Reaktor 2) Flüssigvolumen, einem Druckabscheider (3), einer mit Stickstoff als Stripgas betriebenen Flash-Strip-Kolonne (4) zur Abtrennung der katalysatorhaltigen Schwersiederphase von der Produktphase und nicht umgesetzten C₄-Kohlenwasserstoffen sowie einem lonentauscherbett (5). In dieser Anlage wurde Raffinat II (iso-Butan 2,4 %, n-Butan 12,6 %, trans-2-Buten 31,5 %, 1-Buten 36,8 %, iso-Buten 1,8 %, cis-2-Buten 14,9 %) mit Rhodium und dem Ligand aus Beispiel 1 als Katalysator hydroformyliert. Der Katalysatorrückstrom aus dem Flasher (4) betrug etwa 200 g/h, die Raffinat II-Zufuhr etwa 180 g/h. Die Temperatur der beiden Reaktoren betrug 90 °C. Der erste Reaktor wurde mit Synthesegas mit einem CO:H₂-Molverhältnis von 4:6 und bei einem Gesamtdruck von ca. 17 bar betrieben. In den zweiten Reaktor wurde zusätzlich Wasserstoff geführt und der Reaktor wurde bei einem Gesamtdruck von 16 bar betrieben. Der CO-Gehalt im Abgas wurde auf 10 % eingestellt. In einem repräsentativen Zeitraum von acht Tagen wurde die Anlage bei einer Aldehydausbeute von 55 % stationär betrieben. Dabei war der Ionentauscher (5) nicht aktiv. Die Rhodium-Konzentration im Katalysatorrücklaufstrom aus dem Flasher (4) betrug ca. 320 ppm. Laut HPLC-Analyse waren am Anfang des betrachteten Zeitraums im Katalysatorrückstrom 15000 ppm SkatOX-Ligand (1) enthalten. Nach sechs Tagen konnten noch 3100 ppm und nach 8 Tagen kein SkatOX-Ligand (1) mit der HPLC-Analyse nachgewiesen werden.

### 14. Kontinuierliche Hydroformylierung mit Stabilisierung durch einen Ionenaustauscher

Fig. 2 zeigt eine Miniplant zur Durchführung kontinuierlicher Hydroformylierungen. Diese besteht aus zwei in Reihe geschalteten Hubrührautoklaven (1 und 2) mit je 1,9 l Flüssigvolumen, einem Druckabscheider (3), einem beheizten Entspannungsbehälter zur Abtrennung von C₄-Kohlenwasserstoffen (4), einem Wischblattverdampfer (5) zur Abtrennung der katalysatorhaltigen Schwersiederphase von der Produktphase sowie einem lonentauscherbett (6). In dieser Anlage wurde Raffinat II (iso-Butan 3,6 %, n-Butan 13,8 %, trans-2-Buten 30,9 %, 1-Buten 32,0 %, iso-Buten 2,2 %, cis-2-Buten 17,5 %) mit Rhodium und dem Ligand (1) als Katalysator hydroformyliert. Der Katalysatorrückstrom aus der Destillation (5) betrug etwa 250 g/h, die Raffinat II-Zufuhr etwa 180 g/h. Die Temperatur der beiden Reaktoren betrug 90 °C. Die Reaktoren wurden mit Synthesegas mit einem CO:H₂-Molverhältnis von 4:6 versorgt und bei einem Gesamtdruck von ca. 17 bar betrieben. Zusätzlich wurde Wasserstoff in den ersten Reaktor geführt, um den CO-Gehalt im Abgas auf 10 % einzustellen. In einem repräsentativen Zeitraum von 40 Tagen wurde die Anlage bei einer Aldehydausbeute von 65 % stationär betrieben. Die Rhodium-Konzentration im Strom vom Abscheider (4) zur Destillation (5) betrug ca. 110 ppm. Laut HPLC-Analyse waren am Anfang des betrachteten Zeitraumes im Strom vom Abscheider (4) zur Destillation (5) 7740 ppm SkatOX-Ligand (1) enthalten. Nach 40 Tagen konnten noch 2150 ppm (1) nachgewiesen werden.

### 15. Hydroformylierung von Raffinat II unter Zusatz eines Anlagensumpfes

0,004 g (Rh(CO)₂(acac), 0,141 g Verbindung (1) und 3,71 g eines Katalysatorsumpfes aus einer kontinuierlich betriebenen Miniplant (wie in Beispiel 13 beschrieben) wurden unter Schutzgasatmosphäre in 5,8 g Toluol gelöst und der Ansatz in einen 100 ml Stahlautoklaven überführt.

Der Sumpf stammt aus der bereits in den Beispielen 13 und 14 beschriebenen Miniplantanlage und enthält 480 ppm Rhodium und 3800 ppm Phosphor. Auf den Autoklaven wurden bei 25 °C 10 bar CO/H₂ (1:2) aufgepresst und dieser dann in 30 min auf 90°C erwärmt. Anschließend wurde der Autoklav bei 90 °C vorsichtig entspannt und über eine Schleuse 11,6 g einer Flüssiggasmischung (2,9 vol-% Isobutan; 14,6 vol-% n-Butan; 27,4 vol-% trans 2-Buten; 37,4 vol-% 1-Buten; 2,6 vol-% Isobuten; 15,3 % cis 2-Buten) mit 8 bar CO/H₂ (1:1) zugepresst. Der Druck wurde mit CO/H₂ (1:1) auf 17 bar (gesamt) erhöht und der Autoklav 6 h bei 90 °C gehalten. Nach dem Ende der Reaktion wurde der Autoklav über eine Kühlfalle entspannt und der Inhalt des Autoklaven und der Kühlfalle gaschromatographisch analysiert, um den Umsatz, die Ausbeute an Pentanalen und den Anteil von n-Valeraldehyd an den Pentanalen zu bestimmen.

Ergebnisse der gaschromatographischen Analyse:

| | |
|---|---|
| Umsatz | 65% |
| Ausbeute | 59 % |
| n-Anteil | 90,7 % |

### 16. Hydroformylierung von Raffinat II unter Zusatz eines Anlagensumpfes Wäsche mit Wasser

0,004 g (Rh(CO)₂(acac), 0,130 g Verbindung (1) und 5,37 g eines Katalysatorsumpfes aus einer kontinuierlich betriebenen Miniplant (wie in Beispiel 13 beschrieben) wurden unter Schutzgasatmosphäre in 5,37 g Toluol gelöst und der Ansatz in einen 100 ml Stahlautoklaven überführt.

Der Sumpf stammt aus der bereits in den Beispielen 13 und 14 beschriebenen Miniplantanlage und enthält 480 ppm Rhodium und 3800 ppm Phosphor. Der Sumpf wurde vor dem Einsatz im Experiment unter Schutzgasatmosphäre mit Wasser ausgeschüttelt. Auf den Autoklaven wurden bei 25 °C 10 bar CO/H₂ (1:2) aufgepresst und dieser dann in 30 min auf 90 °C erwärmt. Anschließend wurde der Autoklav bei 90 °C vorsichtig entspannt und über eine Schleuse 9,8 g einer Flüssiggasmischung (2,9 vol-% Isobutan; 14,6 vol-% n-Butan; 27,4 vol-% trans 2-Buten; 37,4 vol-% 1-Buten; 2,6 vol-% Isobuten; 15,3 % cis 2-Buten) mit 8 bar CO/H₂ (1:1) zugepresst. Der Druck wurde mit CO/H₂ (1:1) auf 17 bar (gesamt) erhöht und der Autoklav 6 h bei 90 °C gehalten. Nach dem Ende der Reaktion wurde der Autoklav über eine Kühlfalle entspannt und der Inhalt des Autoklaven und der Kühlfalle gaschromatographisch analysiert, um den Umsatz, die Ausbeute an Pentanalen und den Anteil von n-Valeraldehyd an den Pentanalen zu bestimmen.

Ergebnisse der gaschromatographischen Analyse:

| | |
|---|---|
| Umsatz | 66 % |
| Ausbeute | 60 % |
| n-Anteil | 90,8 % |

### 17. Hydroformylierung von Raffinat II unter Zusatz eines Anlagensumpfes Wäsche mit wässriger NaHCO₃-Lösung

0,003 g (Rh(CO)₂(acac), 0,104 g Verbindung (1) und 2,73 g eines Katalysatorsumpfes aus einer kontinuierlich betriebenen Miniplant (wie in Beispiel 13 beschrieben) wurden unter Schutzgasatmosphäre in 4,27 g Toluol gelöst und der Ansatz in einen 100 ml Stahlautoklaven überführt.

Der Sumpf stammt aus der bereits in den Beispielen 13 und 14 beschriebenen Miniplantanlage und enthält 480 ppm Rhodium und 3800 ppm Phosphor. Der Sumpf wurde vor dem Einsatz im Experiment unter Schutzgasatmosphäre mit wässriger NaHCO₃-Lösun ausgeschüttelt. Auf den Autoklaven wurden bei 25 °C 10 bar CO/H₂ (1:2) aufgepresst und dieser dann in 30 min auf 90 °C erwärmt. Anschließend wurde der Autoklav bei 90 °C vorsichtig entspannt und über eine Schleuse 10,6 g einer Flüssiggasmischung (2,9 vol-% Isobutan; 14,6 vol-% n-Butan; 27,4 vol-% trans 2-Buten; 37,4 vol-% 1-Buten; 2,6 vol-% Isobuten; 15,3 % cis 2-Buten) mit 8 bar CO/H₂ (1:1) zugepresst. Der Druck wurde mit CO/H₂ (1:1) auf 17 bar (gesamt) erhöht und der Autoklav 6 h bei 90 °C gehalten. Nach dem Ende der Reaktion wurde der Autoklav über eine Kühlfalle entspannt und der Inhalt des Autoklaven und der Kühlfalle gaschromatographisch analysiert, um den Umsatz, die Ausbeute an Pentanalen und den Anteil von n-Valeraldehyd an den Pentanalen zu bestimmen.

Ergebnisse der gaschromatographischen Analyse:

| | |
|---|---|
| Umsatz | 68 % |
| Ausbeute | 62 % |
| n-Anteil | 91,7 % |

## Patentansprüche

1. Verfahren zur Hydroformylierung von Verbindungen, die wenigstens eine ethylenisch ungesättigte Doppelbindung enthalten, durch Umsetzung mit Kohlenmonoxid und Wasserstoff in wenigstens einer Reaktionszone in Gegenwart eines katalytisch aktiven Fluids, das eine gelöste Metallkomplexverbindung eines Metalls der VIII. Nebengruppe des Periodensystems der Elemente mit wenigstens einer Phosphoramiditverbindung als Liganden enthält bei dem die Phosphoramiditverbindung ausgewählt ist unter Verbindungen der allgemeinen Formeln I oder II 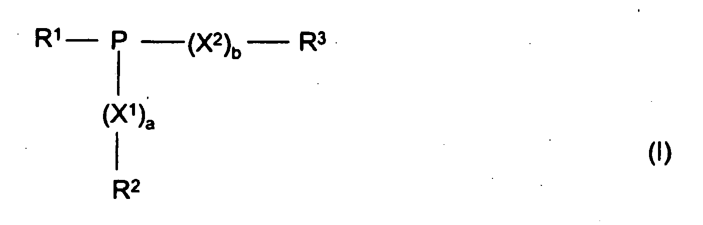 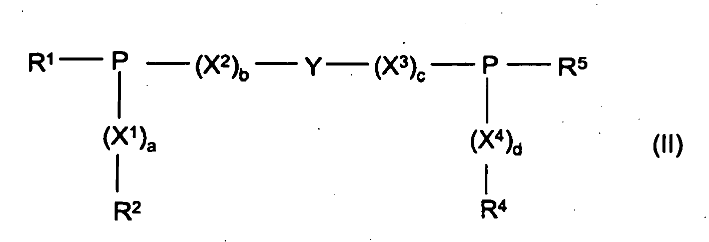 worin
R¹ und R⁵ unabhängig voneinander für über das Stickstoffatom an das Phosphoratom gebundene Pyrrolgruppen stehen,
R², R³ und R⁴ unabhängig voneinander für Alkyl, Cycloalkyl, Heterocycloalkyl, Aryl oder Hetaryl, stehen,
oder R¹ gemeinsam mit R² und/oder R⁴ gemeinsam mit R⁵ eine zweibindige Gruppe bilden, die mindestens eine über das pyrrolische Stickstoffatom an das Phosphoratom gebundene Pyrrolgruppe enthält,
Y für eine zweiwertige verbrückende Gruppe mit 2 bis 20 Brückenatomen zwischen den flankierenden Bindungen steht,
X¹, X², X³ und X⁴ unabhängig voneinander ausgewählt sind unter O, S, SiR^{α}R^{β} und NR^{γ}, worin R^{α}, R^{β} und R^{γ} unabhängig voneinander für Wasserstoff, Alkyl, Cycloalkyl, Heterocycloalkyl, Aryl oder Hetaryl stehen, und
a, b, c und d unabhängig voneinander für 0 oder 1 stehen,
wobei man das Fluid mit einer Base in Kontakt bringt und die Base ausgewählt ist unter in dem katalytisch aktiven Fluid löslichen Basen ausgewählt unter Trialkylaminen, Dialkylarylaminen, Alkyldiarylaminen, Triarylaminen und stickstoffhaltigen Heterocyclen, an eine Festphase immobilisierten Basen und Kombinationen davon.

2. Verfahren nach Anspruch 1, bei dem man
a) in die Reaktionszone(n) die ethylenisch ungesättigte(n) Verbindunge(n) sowie Kohlenmonoxid und Wasserstoff einspeist und in Gegenwart des katalytisch aktiven Fluids umsetzt,
b) aus der Reaktionszone einen Austrag entnimmt und einer Auftrennung unterzieht, wobei eine im Wesentlichen das Hydroformylierungsprodukt enthaltende Fraktion und das katalytische aktive Fluid, das die höher als das Hydroformylierungsprodukt siedenden Nebenprodukte der Hydroformylierung und die darin gelöste Metallkomplexverbindung enthält, erhalten werden, und
c) das katalytisch aktive Fluid, gegebenenfalls nach Abtrennung wenigstens eines Teils der höher als das Hydroformylierungsprodukt siedenden Nebenprodukte, in die Reaktionszone zurückführt.

3. Verfahren nach einem der vorhergehenden Ansprüche, wobei die Base ausgewählt ist unter basischen Stickstoffverbindungen.

4. Verfahren nach einem der vorhergehenden Ansprüche, bei dem man wenigstens eine in dem katalytischen Fluid lösliche Base einsetzt und in der Reaktionszone ein molares Verhältnis von Base zu Phosphoramiditverbindung von 0,01:1 bis 5:1, vorzugsweise von 0,1:1 bis 1,5:1, aufrecht erhalten wird.

5. Verfahren nach einem der vorhergehenden Ansprüche, bei dem man eine Kombination aus wenigstens einer in dem katalytischen Fluid löslichen Base und wenigstens einer an eine Festphase immobilisierten Base einsetzt, wobei die immobilisierten Basen befähigt sind, aus den durch Umsetzung der löslichen Basen mit Säuren erhaltenen Säure-Base-Addukten die löslichen Basen zumindest teilweise freizusetzen.

6. Verfahren nach einem der Ansprüche 2 bis 5, bei dem die Auftrennung des Reaktionsaustrags in Schritt b) einen thermischen Trennschritt umfasst und wobei wenigstens eine hochsiedende lösliche Base eingesetzt wird, die nach der Auftrennung in dem katalytisch aktiven Fluid enthalten ist.

7. Verfahren nach einem der Ansprüche 2 bis 6, bei dem wenigstens eine an eine Festphase immobilisierte Base eingesetzt und das in Schritt b) erhaltene katalytisch aktive Fluid vor der Rückführung in die Reaktionszone mit der immobilisierten Base in Kontakt gebracht wird.

8. Verfahren nach einem der vorhergehenden Ansprüche, bei dem die Phosphoramiditverbindung ausgewählt ist unter Verbindungen der allgemeinen Formel II.1 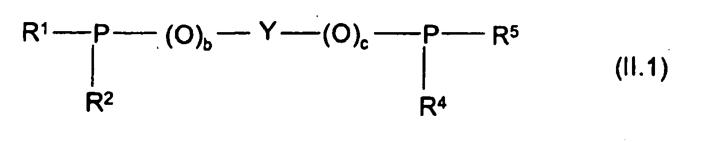 worin
R¹, R², R⁴, R⁵, Y, b und c die in Anspruch 1 angegebenen Bedeutungen besitzen.

9. Verfahren nach einem der Ansprüche 1 bis 8, worin R¹, R², R⁴ und R⁵ unabhängig voneinander ausgewählt sind unter Gruppen der Formeln III.a bis III.k 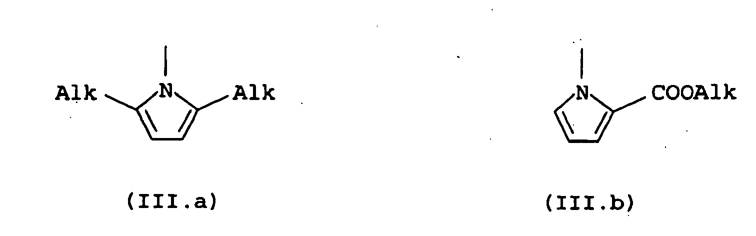 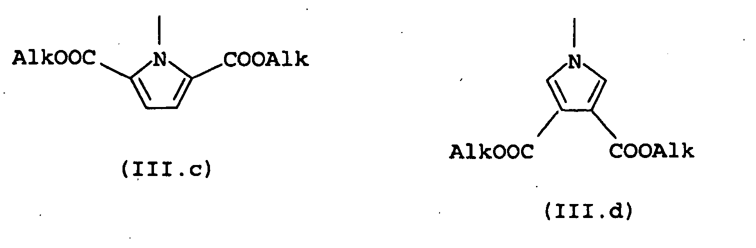 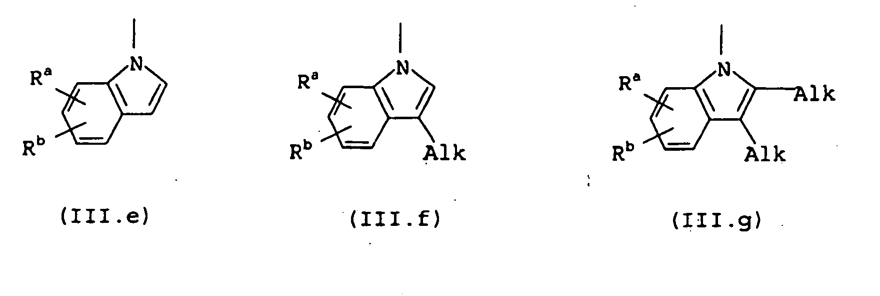 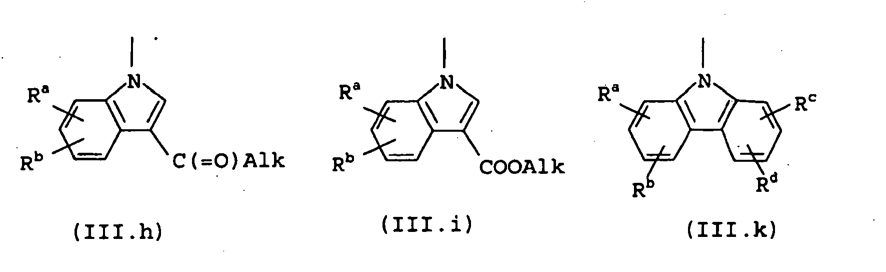 worin
Alk eine C₁-C₁₂-Alkylgruppe ist und
R^{a}, R^{b}, R^{c} und R^{d} unabhängig voneinander für Wasserstoff, C₁-C₄-Alkyl, C₁-C₄-Alkoxy, Acyl, Halogen, C₁-C₄-Alkoxycarbonyl oder Carboxyl stehen.

10. Verfahren nach einem der vorhergehenden Ansprüche, wobei die verbrückende Gruppe Y ausgewählt ist unter Gruppen der Formeln IV.a bis IV.u 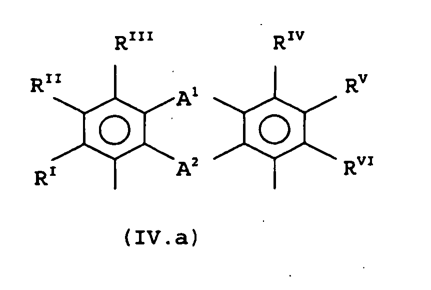 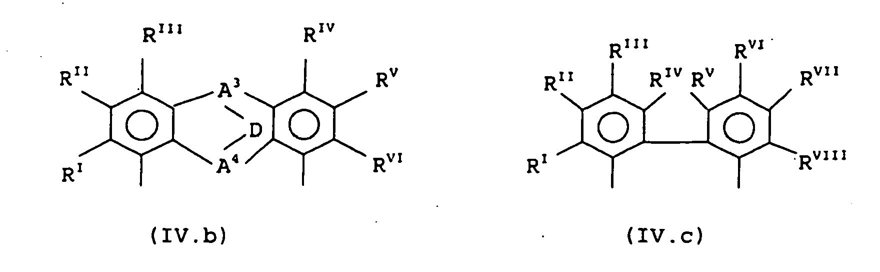 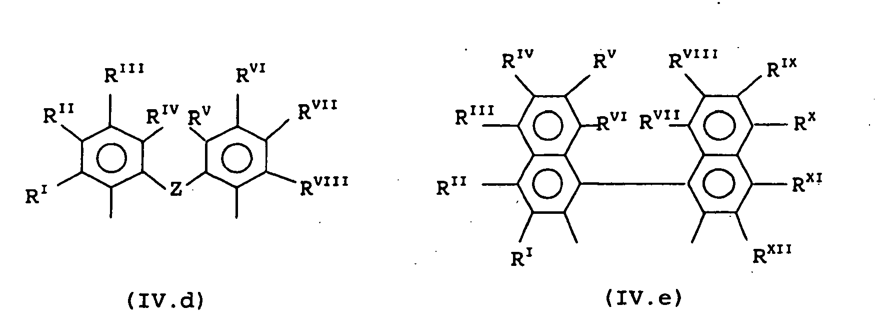 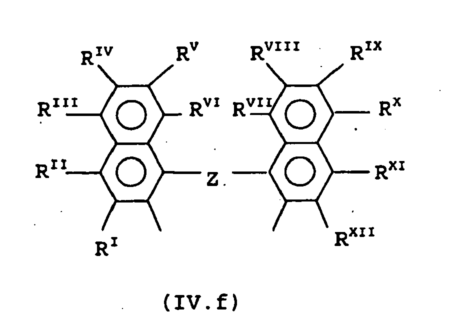 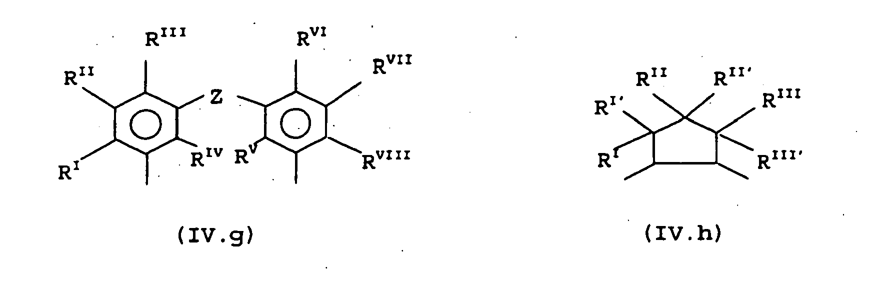 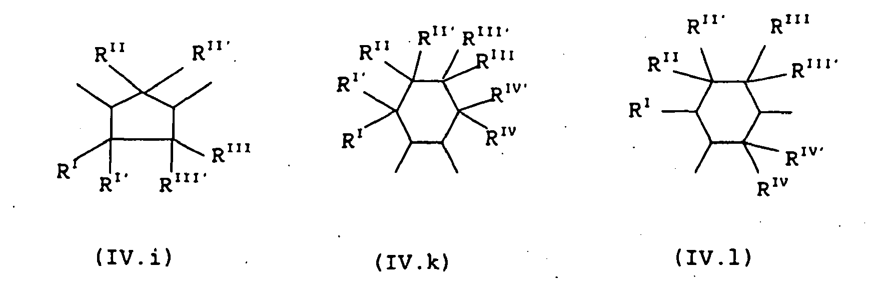 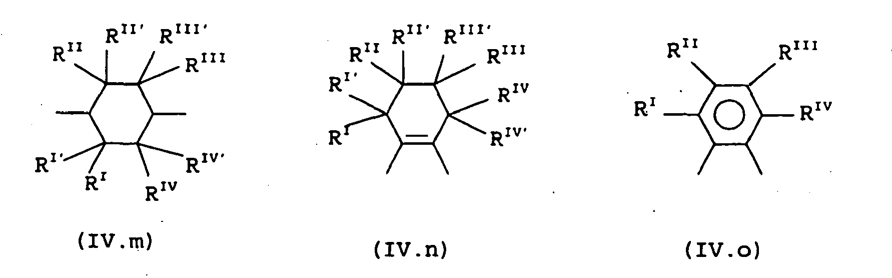 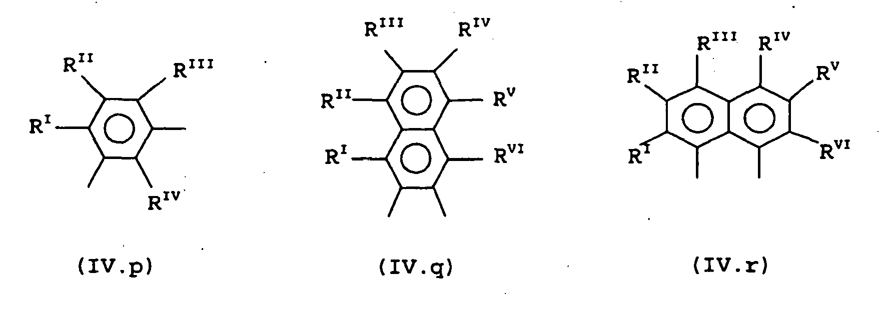 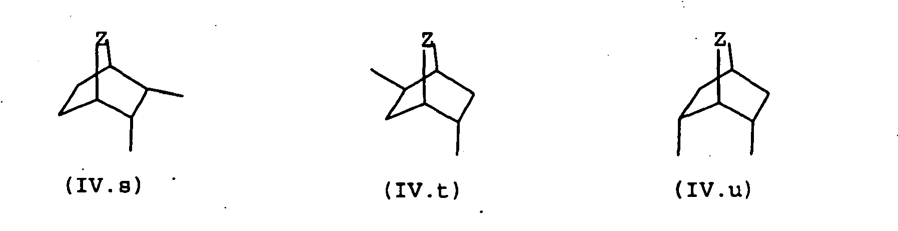 worin
R^{I}, R^{I'}, R^{II}, R^{II'}, R^{III}, R^{III}, R^{IV}, R^{IV'}, R^{V}, R^{VI}, R^{VII}, R^{VIII}, R^{IX}, R^{X}, R^{XI} und R^{XII} unabhängig voneinander für Wasserstoff, Alkyl, Cycloalkyl, Heterocycloalkyl, Aryl, Hetaryl, Hydroxy, Thiol, Polyalkylenoxid, Polyalkylenimin, Alkoxy, Halogen, SO₃H, Sulfonat, NE¹E², Alkylen-NE¹E², Nitro, Alkoxycarbonyl, Carboxyl, Acyl oder Cyano stehen, worin E¹ und E² jeweils gleiche oder verschiedene Reste, ausgewählt unter Wasserstoff, Alkyl, Cycloalkyl und Aryl bedeuten,
Z für O, S, NR^{δ} oder SiR^{δ}R^{c} steht, wobei
R^{δ} und R^{ε} unabhängig voneinander für Wasserstoff, Alkyl, Cycloalkyl, Heterocycloalkyl, Aryl oder Hetaryl stehen,
oder Z für eine C₁-C₄-Alkylenbrücke steht, die eine Doppelbindung und/oder einen Alkyl-, Cycloalkyl-, Heterocycloalkyl-, Aryl- oder Hetaryl- Substituenten aufweisen kann,
oder Z für eine C₂-C₄-Alkylenbrücke steht, die durch O, S oder NR^{δ} oder SiR^{δ}R^{ε} unterbrochen ist,
wobei in den Gruppen der Formel IV.a und IV.b zwei benachbarte Reste R^{I} bis R^{VI} gemeinsam mit den Kohlenstoffatomen des Benzolkerns, an die sie gebunden sind, auch für ein kondensiertes Ringsystem mit 1, 2 oder 3 weiteren Ringen stehen können,
wobei in den Gruppen der Formeln IV.h bis IV.n zwei geminale Reste R¹, R^{I'}; R^{II}, R^{II'}; R^{III}, R^{III'} und/oder R^{IV}, R^{IV"} auch für Oxo oder ein Ketal davon stehen können,
A¹ und A² unabhängig voneinander für O, S, SiR^{φ}R^{γ}, NR^{η} oder CR^{l}R^{κ} stehen, wobei R^{φ}, R^{γ}, R^{η}, R^{l} und R^{κ} unabhängig voneinander für Wasserstoff, Alkyl, Cycloalkyl, Heterocycloalkyl, Aryl oder Hetaryl stehen,
A³ und A⁴ unabhängig voneinander für SiR^{φ}, N oder CR' stehen,
D für eine zweibindige Brückengruppe der allgemeinen Formel 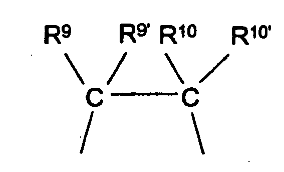 steht, in der
R⁹, R^{9'}, R¹⁰ und R^{10'} unabhängig voneinander für Wasserstoff, Alkyl, Cycloalkyl, Aryl, Halogen, Trifluormethyl, Carboxyl, Carboxylat oder Cyano stehen,
wobei R^{9'} auch gemeinsam mit R^{10'} für den Bindungsanteil einer Doppelbindung zwischen den beiden Kohlenstoffatomen, an die R^{9'} und R^{10'} gebunden sind, stehen kann, und/oder R⁹ und R¹⁰ gemeinsam mit den Kohlenstoffatomen, an die sie gebunden sind, auch für einen 4- bis 8-gliedrigen Carbo- oder Heterocyclus stehen können, der gegebenenfalls zusätzlich ein-, zwei- oder dreifach mit Cycloalkyl, Heterocycloalkyl, Aryl oder Hetaryl anelliert ist, wobei der Heterocyclus und, falls vorhanden, die anellierten Gruppen unabhängig voneinander je einen, zwei, drei oder vier Substituenten tragen können, die ausgewählt sind unter Alkyl, Cycloalkyl, Heterocycloalkyl, Aryl, Hetaryl, COOR^{f}, COO⁻M⁺, SO₃R^{f}, SO⁻₃M⁺, NE⁴E⁵, Alkylen-NE⁴E⁵, NE⁴E⁵E⁶⁺X⁻, Alkylen-NE⁴E⁵E⁶⁺X⁻, OR^{f}, SR^{f}, (CHR^{e}CH₂O)_{γ}R^{f}, (CH₂N(E⁴))_{γ}R^{f}, (CH₂CH₂N(E⁴))_{γ}R^{f}, Halogen, Trifluormethyl, Nitro, Acyl oder Cyano, stehen, worin
R^{f}, E⁴, E⁵ und E⁶ jeweils gleiche oder verschiedene Reste, ausgewählt unter Wasserstoff, Alkyl, Cycloalkyl oder Aryl bedeuten,
R^{e} für Wasserstoff, Methyl oder Ethyl steht,
M⁺ für ein Kation steht,
X⁻ für ein Anion steht, und
y für eine ganze Zahl von 1 bis 240 steht.

## Claims

1. A process for the hydroformylation of compounds comprising at least one ethylenically unsaturated double bond by reaction with carbon monoxide and hydrogen in at least one reaction zone in the presence of a catalytically active fluid which comprises a dissolved metal complex of a metal of transition group VIII of the Periodic Table of the Elements with at least one phosphoramidite compound as ligand, wherein the phosphoramidite compound is selected from among compounds of the formulae I and II 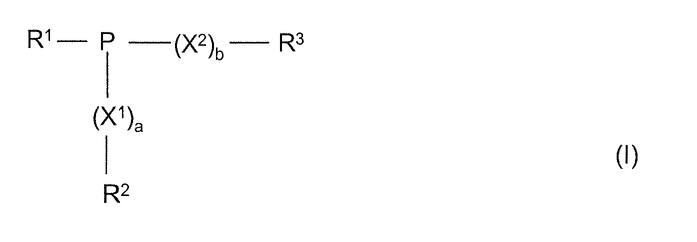 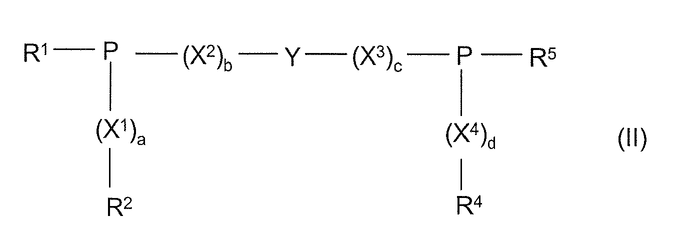 where
R¹ and R⁵ are each, independently of one another, pyrrole groups bound via the nitrogen atom to the phosphorus atom,
R², R³ and R⁴ are each, independently of one another, alkyl, cycloalkyl, heterocycloalkyl, aryl or hetaryl,
or R¹ together with R² and/or R⁴ together with R⁵ forms a divalent group comprising at least one pyrrole group bound via the pyrrolic nitrogen atom to the phosphorus atom,
Y is a divalent bridged group having from 2 to 20 bridge atoms between the flanking bonds,
X¹, X², X³ and X⁴ are selected independently from among 0, S, SiR^{α}R^{β} and NR^{γ}, where R^{α}, R^{β} and R^{γ} are each, independently of one another, hydrogen, alkyl, cycloalkyl, heterocycloalkyl, aryl or hetaryl, and
a, b, c and d are each, independently of one another, 0 or 1, wherein the fluid is brought into contact with a base and the base is selected from among bases soluble in the catalytically active fluid and selected from among trialkylamines, dialkylarylamines, alkyldiarylamines, triarylamines and nitrogen-containing heterocycles, bases immobilized on a solid phase and combinations thereof.

2. The process according to claim 1, wherein
a) the ethylenically unsaturated compound(s) and carbon monoxide and hydrogen are fed into the reaction zone(s) and are reacted in the presence of the catalytically active fluid,
b) an output is taken from the reaction zone and is subjected to a fractionation to give a fraction comprising essentially the hydroformylation product and a fraction comprising the catalytically active fluid in which the by-products of the hydroformylation which have boiling points higher than that of the hydroformylation product are present and the metal complex is dissolved, and
c) the catalytically active fluid is, if appropriate after separating off at least part of the by-products having boiling points higher than that of the hydroformylation product, recirculated to the reaction zone.

3. The process according to any of the preceding claims, wherein the base is selected from among basic nitrogen compounds.

4. The process according to any of the preceding claims, wherein at least one base which is soluble in the catalytic fluid is used and a molar ratio of base to phosphoramidite compound of from 0.01:1 to 5:1, preferably from 0.1:1 to 1.5:1, is maintained in the reaction zone.

5. The process according to any of the preceding claims, wherein a combination of at least one base soluble in the catalytic fluid and at least one base immobilized on a solid phase is used and the immobilized bases are capable of at least partly liberating the soluble bases from the acid-base adducts obtained by reaction of the soluble bases with acids.

6. The process according to any of claims 2 to 5, wherein the fractionation of the output from the reaction in step b) comprises a thermal separation step and at least one high-boiling soluble base which is present in the catalytically active fluid after the fractionation is used.

7. The process according to any of claims 2 to 6, wherein at least one base immobilized on a solid phase is used and the catalytically active fluid obtained in step b) is brought into contact with the immobilized base before it is recirculated to the reaction zone.

8. The process according to any of the preceding claims, wherein the phosphoramidite compound is selected from among compounds of the formula II.1 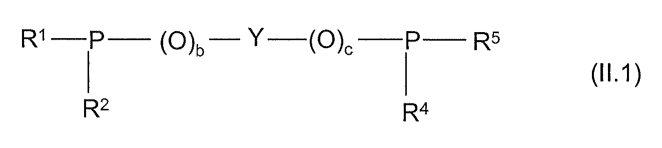 where
R¹, R², R⁴, R⁵, Y, b and c are as defined in claim 1.

9. The process according to any of claims 1 to 8, wherein R¹, R², R⁴ and R⁵ are selected independently from among groups of the formulae III.a to III.k 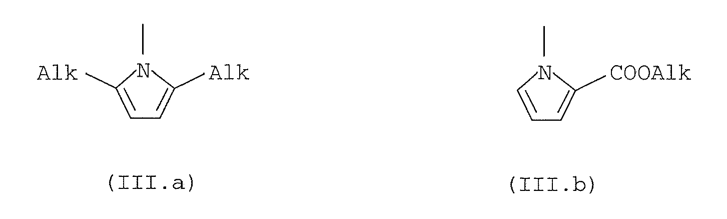 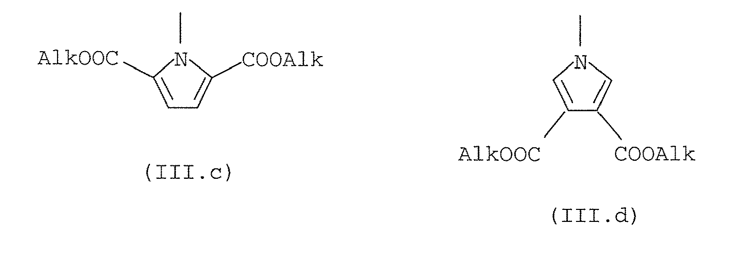 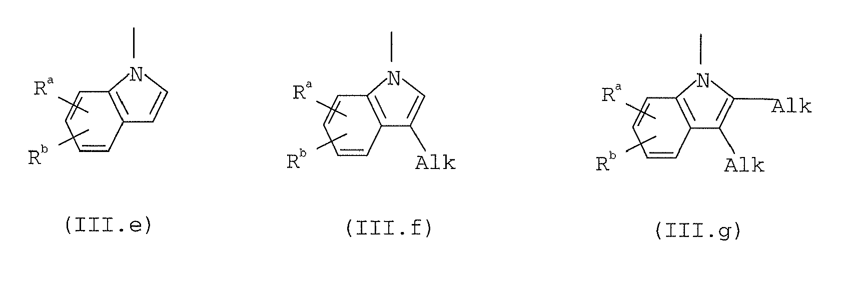 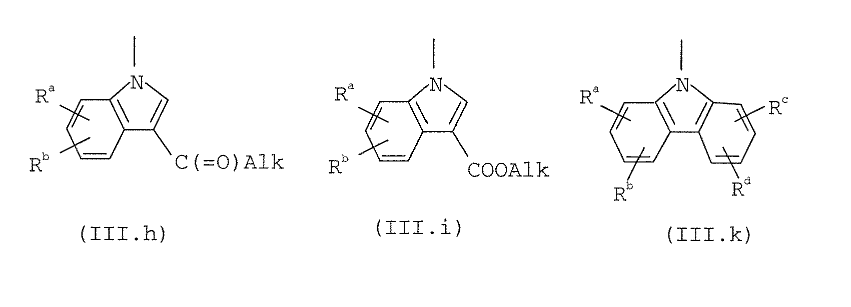 where
Alk is a C₁-C₁₂-alkyl group and
R^{a} , R^{b}, R^{c} and R^{d} are each, independently of one another, hydrogen, C₁-C₄-alkyl, C₁-C₄-alkoxy, acyl, halogen, C₁-C₄-alkoxycarbonyl or carboxyl.

10. The process according to any of the preceding claims, wherein the bridging group Y is selected from among groups of the formulae IV.a to IV.u 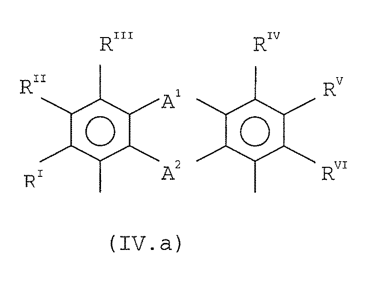 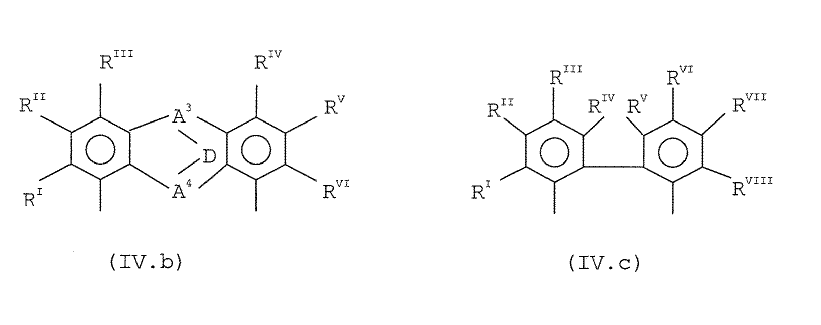 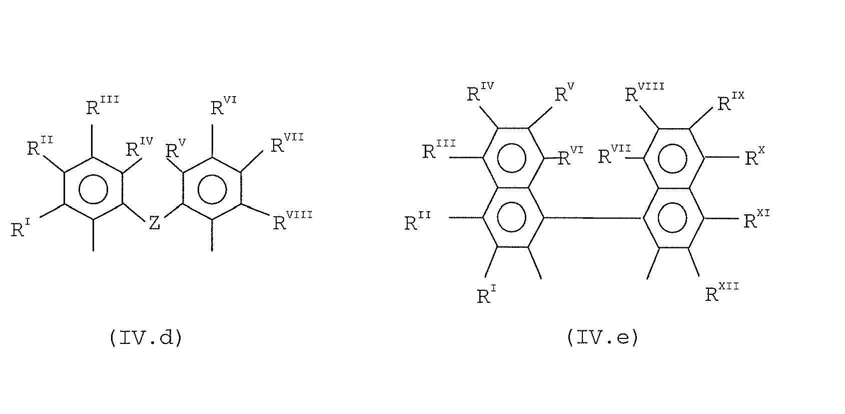 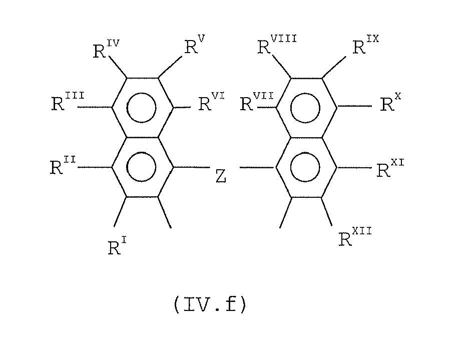 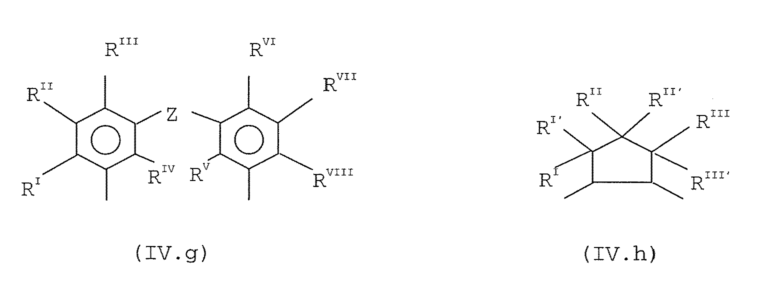 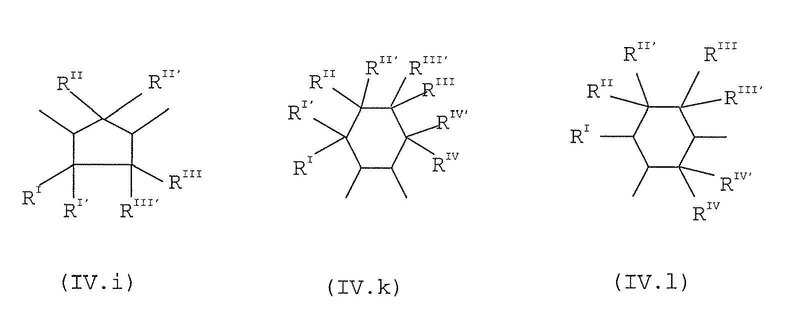 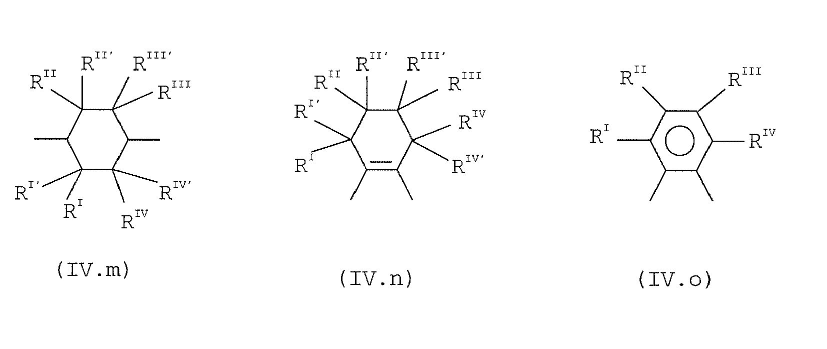 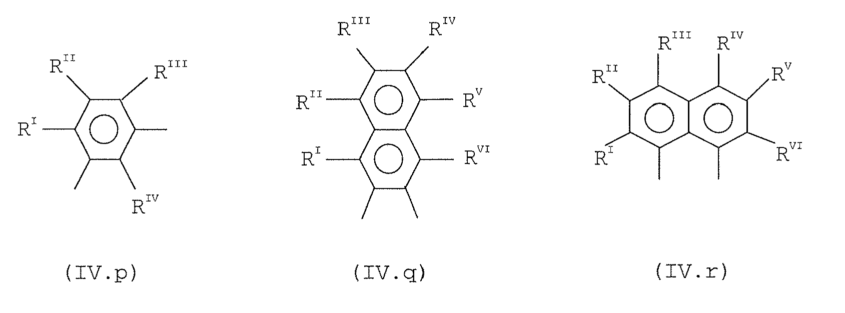 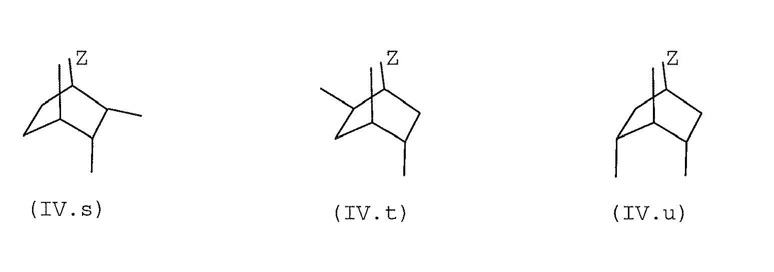 where
R^{I} , R^{I'}, R^{II}, R^{II}, R^{III}, R^{III'}, R^{IV}, R^{IV'}, R^{V}, R^{VI}, R^{VII}, R^{VIII}, R^{IX}, R^{X}, R^{XI} and R^{XII} are each, independently of one another, hydrogen, alkyl, cycloalkyl, heterocycloalkyl, aryl, hetaryl, hydroxy, thiol, polyalkylene oxide, polyalkylenimine, alkoxy, halogen, SO₃H, sulfonate, NE¹E², alkylene-NE¹E², nitro, alkoxycarbonyl, carboxyl, acyl or cyano, where E¹ and E² are identical or different radicals selected from among hydrogen, alkyl, cycloalkyl and aryl,
Z is O, S, NR^{δ} or SiR^{δ}R^{ε}, where
R^{δ} and R^{ε} are each, independently of one another, hydrogen, alkyl, cycloalkyl, heterocycloalkyl, aryl or hetaryl,
or Z is a C₁-C₄-alkylene bridge which may have a double bond and/or bear an alkyl, cycloalkyl, heterocycloalkyl, aryl or hetaryl substituent,
or Z is a C₂-C₄-alkylene bridge which is interrupted by 0, S or NR^{δ} or SiR^{δ}R^{ε},
where, in the groups of the formulae IV.a and IV.b, two adjacent radicals R^{I} to R^{IV} together with the carbon atoms of the benzene ring to which they are bound may also form a fused ring system having 1, 2 or 3 further rings,
where, in the groups of the formulae IV.h to IV.n, two geminal radicals R^{I}, R^{I'}; R^{II}, R^{II'}; R^{III}, R^{III'} and/or R^{IV}, R^{IV"} may also represent oxo or a ketal thereof,
A¹ and A² are each, independently of one another, O, S, SiR^{φ}R^{γ}, NR^{η} or CR^{τ}R^{κ}, where R^{φ}, R^{γ}, R^{η}, R^{η} and R^{κ} are each, independently of one another, hydrogen, alkyl, cycloalkyl, heterocycloalkyl, aryl or hetaryl,
A³ and A⁴ are each, independently of one another, SiR^{φ}, N or CR^{τ},
D is a divalent bridging group of the formula 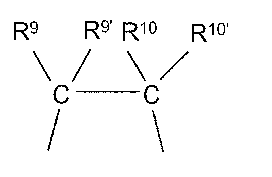 where
R⁹, R^{9'}, R¹⁰ and R^{10'} are each, independently of one another, hydrogen, alkyl, cycloalkyl, aryl, halogen, trifluoromethyl, carboxyl, carboxylate or cyano,
where R^{9'} together with R^{10'} can also represent the second bond of a double bond between the two carbon atoms to which R^{9'} and R^{10'} are bound, and/or R⁹ and R¹⁰ together with the carbon atoms to which they are bound may also form a 4- to 8-membered carbocycle or heterocycle which may additionally be fused with one, two or three cycloalkyl, heterocycloalkyl, aryl or hetaryl groups, where the heterocycle and, if present, the fused-on groups may each bear, independently of one another, one, two, three or four substituents selected from among alkyl, cycloalkyl, heterocycloalkyl, aryl, hetaryl, COOR^{f}, COO⁻M⁺, SO₃R^{f}, SO⁻₃M⁺, NE⁴E⁵, alkylene-NE⁴E⁵, NE⁴E⁵E⁶⁺X⁻, alkylene-NE⁴E⁵E⁶⁺X⁻, OR^{f}, SR^{f}, (CHR^{e}CH₂O)_{y}R^{f}, (CH₂N (E⁴))_{y}R^{f}, (CH₂CH₂N(E⁴))_{y}R^{f}, halogen, trifluoromethyl, nitro, acyl and cyano, where
R^{f}, E⁴, E⁵ and E⁶ are identical or different radicals selected from among hydrogen, alkyl, cycloalkyl and aryl,
R^{e} is hydrogen, methyl or ethyl,
M⁺ is a cation,
X⁻ is an anion and
y is an integer from 1 to 240.

## Revendications

1. Procédé pour l'hydroformylation de composés qui comportent au moins une double liaison à insaturation éthylénique, par mise en réaction avec du monoxyde de carbone et de l'hydrogène dans au moins une zone de réaction, en présence d'un fluide à activité catalytique, qui contient un composé complexe métallique dissous d'un métal du groupe VIII du système périodique des éléments, avec au moins un composé phosphoramidite en tant que ligand, dans lequel le composé phosphoramidite est choisi parmi les composés de formules générales I ou II 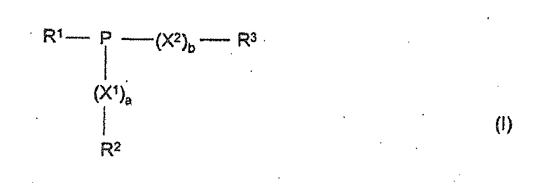 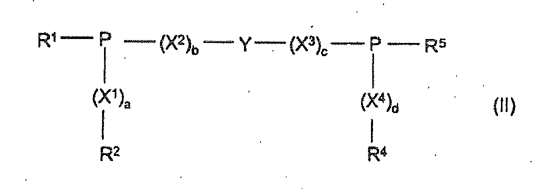 dans lesquelles
R¹ et R⁵ représentent, indépendamment l'un de l'autre, des groupes pyrrole liés à l'atome de phosphore par l'atome d'azote,
R², R³ et R⁴ représentent, chacun indépendamment, un groupe alkyle, cycloalkyle, hétérocycloalkyle, aryle ou hétéroaryle,
ou R¹ conjointement avec R² et / ou R⁴ conjointement avec R⁵ forment un groupe à deux liaisons, qui contient au moins un groupe pyrrole lié à l'atome de phosphore par l'atome d'azote en fonction pyrrole,
Y représente un groupe pontant divalent ayant de 2 à 20 atomes formant le pont entre les liaisons adjacentes,
X¹, X², X³ et X⁴ sont choisis, indépendamment les uns des autres, parmi O, S, SiR^{α}R^{β} et NR^{γ}, R^{α}, R^{β} et R^{γ} représentant, chacun indépendamment, un atome d'hydrogène, un groupe alkyle, cycloalkyle, hétérocycloalkyle, aryle ou hétéroaryle, et
a, b, c et d représentent, indépendamment les uns des autres, 0 ou 1,
dans lequel on met le fluide en contact avec une base et la base est choisie parmi des bases solubles dans le fluide à activité catalytique, choisies parmi des trialkylamines, des dialkylarylamines, des alkyldiarylamines, des triarylamines et des hétérocycles azotés, des bases immobilisées sur une phase solide et des associations de celles-ci.

2. Procédé selon la revendication 1, dans lequel
a) on introduit dans la/les zone(s) de réaction le(s) composé(s) à insaturation éthylénique ainsi que du monoxyde de carbone et de l'hydrogène et on les fait réagir en présence du fluide à activité catalytique,
b) on prélève une décharge de la zone de réaction et on la soumet à un fractionnement, pour obtenir une fraction contenant essentiellement le produit d'hydroformylation et le fluide à activité catalytique, qui contient les sous-produits de l'hydroformylation ayant un point d'ébullition plus élevé que le produit d'hydroformylation, et le composé complexe métallique dissous dans ce fluide, et
c) on renvoie dans la zone de réaction le fluide à activité catalytique, éventuellement après séparation d'au moins une partie des sous-produits d'hydroformylation ayant un point d'ébullition plus élevé que le produit d'hydroformylation.

3. Procédé selon l'une quelconque des revendications précédentes, dans lequel la base est choisie parmi des composés azotés basiques.

4. Procédé selon l'une quelconque des revendications précédentes, dans lequel on utilise au moins une base soluble dans le fluide catalytique et on maintient dans la zone de réaction un rapport molaire de base à composé phosphoramidite de 0,01:1 à 5:1, de préférence de 0,1:1 à 1,5:1.

5. Procédé selon l'une quelconque des revendications précédentes, dans lequel on utilise une association d'au moins une base soluble dans le fluide catalytique et d'au moins une base immobilisée sur une phase solide, les bases immobilisées étant capables de libérer au moins en partie les bases solubles à partir des adduits acide-base obtenus par réaction des bases solubles avec des acides.

6. Procédé selon l'une quelconque des revendications 2 à 5, dans lequel la séparation de la décharge de réaction dans l'étape b) comprend une étape de séparation thermique et où on utilise au moins une base soluble à haut point d'ébullition, qui est contenue dans le liquide à activité catalytique après la séparation.

7. Procédé selon l'une quelconque des revendications 2 à 6, dans lequel on utilise au moins une base immobilisée sur une phase solide et on met en contact avec la phase immobilisée le fluide à activité catalytique, obtenu dans l'étape b), avant le renvoi dans la zone de réaction.

8. Procédé selon l'une quelconque des revendications précédentes, dans lequel le composé phosphoramidite est choisi parmi des composés de formule générale II.1 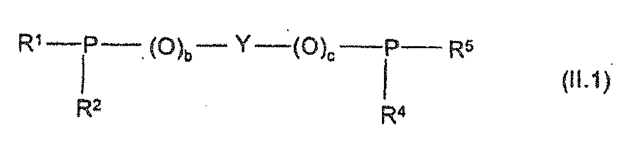 dans laquelle
R¹, R², R⁴, R⁵, Y, b et c ont les significations indiquées dans la revendication 1.

9. Procédé selon l'une quelconque des revendications 1 à 8, dans lequel R¹, R², R⁴ et R⁵ sont choisis, indépendamment les uns des autres, parmi des groupes de formules III.a à III.k 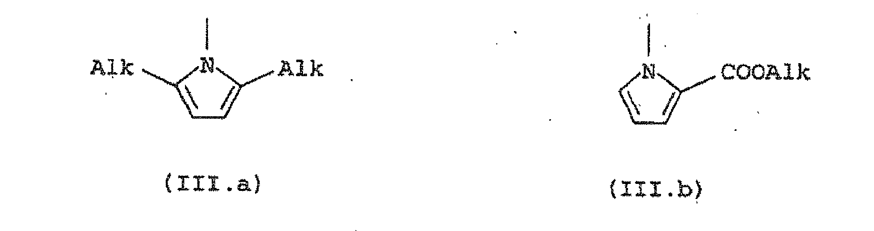 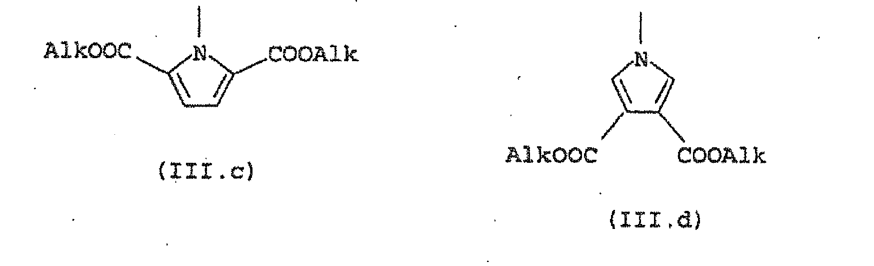 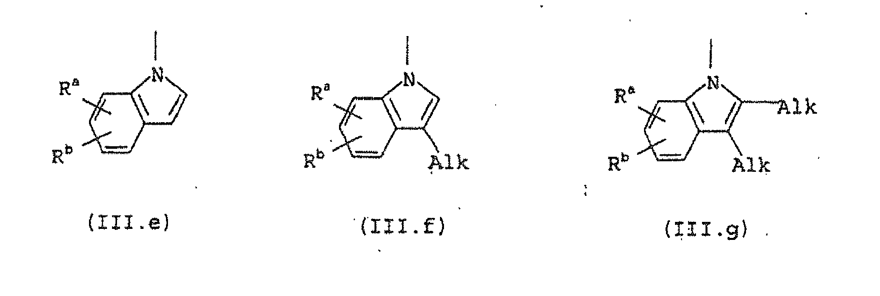 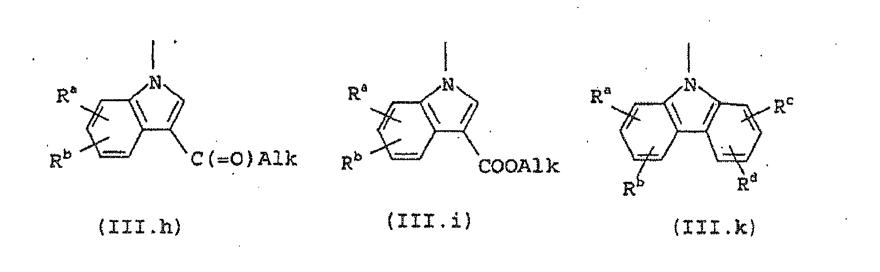 dans lesquelles
Alk est un groupe alkyle en C₁-C₁₂ et
R^{a}, R^{b}, R^{c} et R^{d} représentent, indépendamment les uns des autres, un atome d'hydrogène ou d'halogène ou un groupe alkyle en C₁-C₄, alcoxy en C₁-C₄, acyle, alcoxy(C₁-C₄)carbonyle ou carboxy.

10. Procédé selon l'une quelconque des revendications précédentes, dans lequel le groupe pontant Y est choisi parmi des groupes de formules IV.a à IV.u 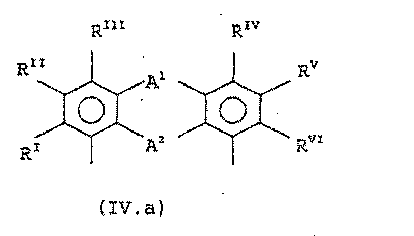 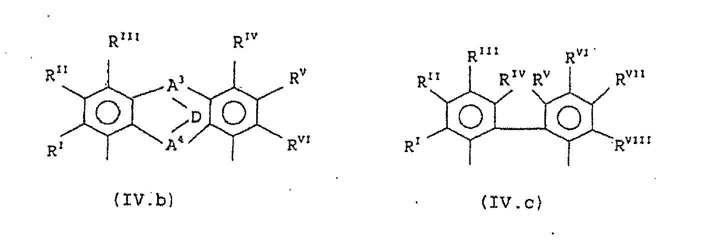 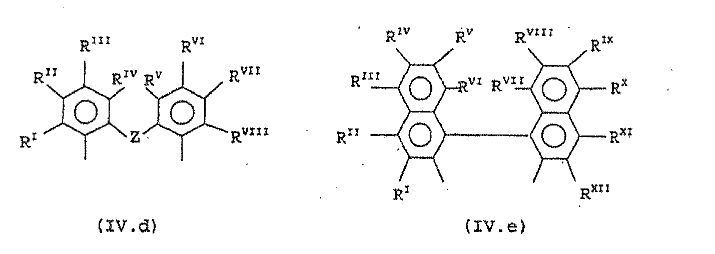 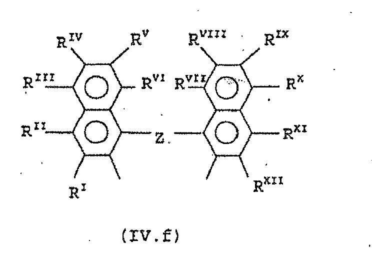 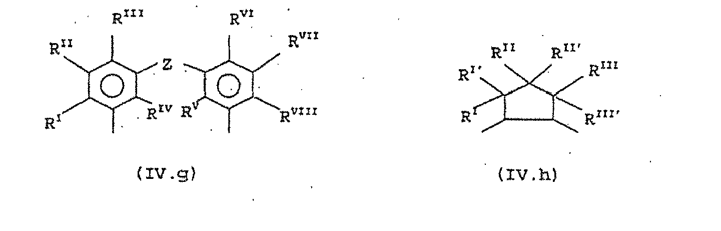 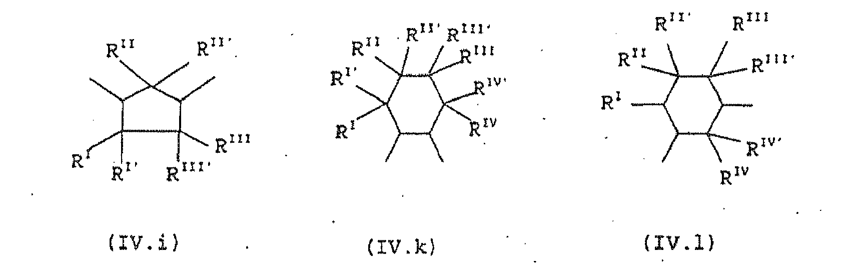 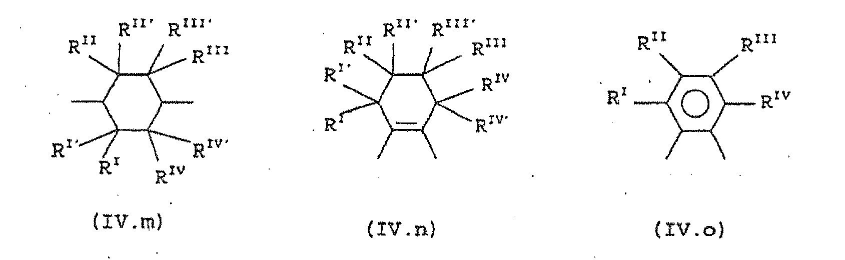 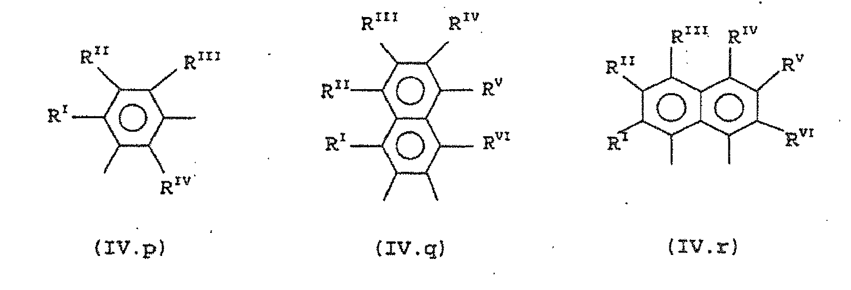 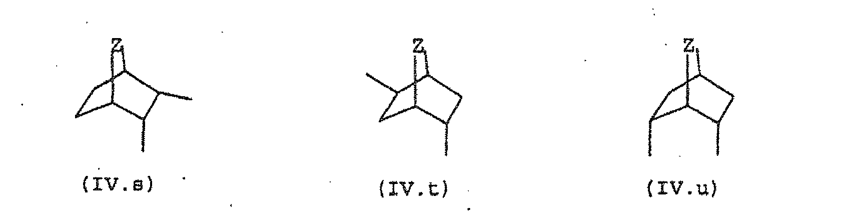 dans lesquelles
R^{I'}, R^{I'}, R^{II}, R^{II'}, R^{III}, R^{III'}, R^{IV}, R^{IV'}, R^{V}, R^{VI}, R^{VII}, R^{VIII}, R^{IX}, R^{X}, R et R^{XII} représentent, indépendamment les uns des autres, un atome d'hydrogène, un groupe alkyle, cycloalkyle, hétérocycloalkyle, aryle, hétéroaryle, hydroxy, thiol, polyoxyalkylène, polyalkylène-imine, alcoxy, un atome d'halogène, un groupe SO₃H, sulfonate, NE¹E², alkylène-NE¹E², nitro, alcoxycarbonyle, carboxy, acyle ou cyano, E¹ et E² représentant chacun des radicaux identiques ou différents, choisis parmi un atome d'hydrogène, des radicaux alkyle, cycloalkyle et aryle,
Z représente 0, S, NR^{δ} ou SiR^{δ}R^{ε},
R^{δ} et R^{ε} représentant, indépendamment l'un de l'autre, un atome d'hydrogène, un groupe alkyle, cycloalkyle, hétérocycloalkyle, aryle ou hétéroaryle,
ou Z représente un pont alkylène en C₁-C₄, qui peut comporter une double liaison et/ou un substituant alkyle, cycloalkyle, hétérocycloalkyle, aryle ou hétéroaryle,
ou Z représente un pont alkylène en C₂-C₄, qui est interrompu par 0, S ou NR^{δ} ou SiR^{δ}R^{ε},
deux radicaux R^{I} à R^{VI} adjacents dans les groupes de formule IV.a et IV.b pouvant également former conjointement avec les atomes de carbone du noyau benzénique, auxquels ils sont fixés, un système cyclique condensé à 1, 2 ou 3 autres cycles,
deux radicaux R^{I}, R^{I'}; R^{II}, R^{II'}; R^{III}, R^{III'} et / ou R^{IV}, R^{IV'} géminés dans les groupes de formule IV.h à IV.n pouvant également représenter un groupe oxo ou cétal de ceux-ci,
A¹ et A² représentent, indépendamment l'un de l'autre, O, S, SiR^{φ}R^{γ}, NR^{η}, ou CR^{τ}R^{κ}, R^{φ}, R^{γ}, R^{η}, R^{τ} et R^{κ} représentant, indépendamment les uns des autres, un atome d'hydrogène, un groupe alkyle, cycloalkyle, hétérocycloalkyle, aryle ou hétéroaryle,
A³ et A⁴ représentent, indépendamment l'un de l'autre, SiR^{φ}, N ou CR^{τ},
D représente un groupe pontant à deux liaisons, de formule générale 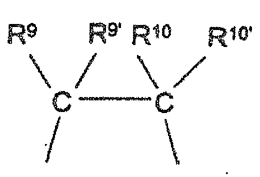
dans laquelle
R⁹, R^{9'}, R¹⁰ et R^{10'} représentent, indépendamment les uns des autres, un atome d'hydrogène, un groupe alkyle, cycloalkyle, aryle, un atome d'halogène, un groupe trifluorométhyle, carboxy, carboxylate ou cyano,
R^{9'} pouvant également représenter conjointement avec R^{10'} le segment de liaison d'une double liaison entre les deux atomes de carbone auxquels sont liés R^{9'} et R^{10'}, et/ou R⁹ et R¹⁰ peuvent également représenter conjointement avec les atomes de carbone auxquels ils sont liés un cycle carbocyclique ou hétérocyclique à 4-8 chaînons, qui éventuellement est en outre soudé une, deux ou trois fois à un groupe cycloalkyle, hétérocycloalkyle, aryle ou hétéroaryle, l'hétérocycle et, s'ils sont présents, les groupes soudés pouvant porter, chacun indépendamment, un, deux, trois ou quatre substituants, qui sont choisis parmi des groupes alkyle, cycloalkyle, hétérocycloalkyle, aryle, hétéroaryle, COOR^{f}, COO⁻M⁺, SO₃R^{f}, SO⁻₃M⁺, NE⁴E⁵, alkylène-NE⁴E⁵, NE⁴E⁵E⁶⁺X⁻, alkylène-NE⁴E⁵E⁶⁺X⁻, OR^{f}, SR^{f}, (CHR^{e}CH₂O)_{y}R^{f}, (CH₂N(E⁴))_{y}R^{f}, (CH₂CH₂N(E⁴))_{y}R^{f}, halogéno, trifluorométhyle, nitro, acyle ou cyano,
R^{f}, E⁴, E⁵ et E⁶ représentant chacun des radicaux identiques ou différents, choisis parmi un atome d'hydrogène, des groupes alkyle, cycloalkyle ou aryle,
R^{e} représentant un atome d'hydrogène, un groupe méthyle ou éthyle,
M⁺ représentant un cation,
X représentant un anion, et
y représentant un nombre entier valant de 1 à 240.
